(19) Europäisches Patentamt
European Patent Office
Office européen des brevets

(11) EP 1 354 951 B1

(12) EUROPEAN PATENT SPECIFICATION

(45) Date of publication and mention of the grant of the patent:
**04.06.2008 Bulletin 2008/23**

(21) Application number: **01272818.4**

(22) Date of filing: **17.12.2001**

(51) Int Cl.:
*C12N 15/12* (2006.01) *C12Q 1/02* (2006.01)
*C12Q 1/68* (2006.01) *A61K 45/00* (2006.01)
*A61P 25/00* (2006.01) *G01N 33/15* (2006.01)
*G01N 33/50* (2006.01) *C12R 1/91* (2006.01)

(86) International application number:
**PCT/JP2001/011063**

(87) International publication number:
**WO 2002/053736 (11.07.2002 Gazette 2002/28)**

(54) **METHOD OF EXAMINING ABILITY TO CONTROL NERVE CELL PLASTICITY**

VERFAHREN ZUR UNTERSUCHUNG DER FÄHIGKEIT ZUR KONTROLLE DER NERVENZELLPLASTIZITÄT

METHODE D'EXAMEN DE LA CAPACITE A CONTROLER LA PLASTICITE DES CELLULES NERVEUSES

(84) Designated Contracting States:
**AT BE CH CY DE DK ES FI FR GB GR IE IT LI LU MC NL PT SE TR**

(30) Priority: **27.12.2000 JP 2000398548**
**19.03.2001 JP 2001077740**

(43) Date of publication of application:
**22.10.2003 Bulletin 2003/43**

(73) Proprietor: **Sumitomo Chemical Company, Limited**
**Tokyo 104-8260 (JP)**

(72) Inventor: **OHE, Norihisa**
**Nara-shi, Nara 631-0035 (JP)**

(74) Representative: **Vossius & Partner**
**Siebertstrasse 4**
**81675 München (DE)**

(56) References cited:
**WO-A-02/12565** **WO-A-98/27206**

- **YAMAKI AKIKO ET AL: "The mammalian single-minded (SIM) gene: Mouse cDNA structure and diencephalic expression indicate a candidate gene for Down syndrome" GENOMICS, vol. 35, no. 1, 1996, pages 136-143, XP002303068 ISSN: 0888-7543**
- **SADEK CHRISTINE M ET AL: "Isolation and characterization of AINT: A novel ARNT interacting protein expressed during murine embryonic development" MECHANISMS OF DEVELOPMENT, vol. 97, no. 1-2, October 2000 (2000-10), pages 13-26, XP002303069 ISSN: 0925-4773**
- **MA Y. ET AL.: 'Functional interactions between drosophila bHLH/PAS, SOX and POU transcription factors regulate CNS midline expression of the slit gene' J. NEUROSCI. vol. 20, no. 12, June 2000, pages 4596 - 4605, XP002909393**
- **MICHAUD J.L. ET AL.: 'ARNT2 acts as the dimerization partner of SIM1 for the development of the hypothalamus' MECH. DEV. vol. 90, no. 2, February 2000, pages 253 - 261, XP002909394**
- **MICHAUD J.L. ET AL.: 'Development of neuroendocrine lineages requires the bHLH-PAS transcription factor SIM1' GENES & DEV. vol. 12, 1998, pages 3264 - 3275, XP002909395**
- **CREWS S.T. ET AL.: 'Control of cell lineage-specific development and transcription by bHLH-PAS proteins' GENES & DEV. vol. 12, 1998, pages 607 - 620, XP002127029**
- **TAGUCHI E. ET AL.: 'Zep:A novel zinc finger protein containing a large proline-rich domain' J. BIOCHEM. vol. 124, 1998, pages 1220 - 1228, XP002909396**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **TEAR G.: 'Molecular cues that guide the development of neural connectivity' ESSAYS BIOCHEM. vol. 33, 1998, pages 1 - 13, XP002909397**
- **GERLAI R. ET AL.: 'Regulation of learning by EphA receptors: a protein targeting study' J. NEUROSCI. vol. 19, no. 21, 1999, pages 9538 - 9549, XP002909398**
- **SOGAWA K. ET AL.: 'Possible function of Ah receptor nuclear translocator (Arnt), homodimer in transcriptional regulation' PROC. NATL. ACAD. SCI. USA vol. 92, 1995, pages 1936 - 1940, XP002909399**

## Description

TECHNICAL FIELD.

The present invention relates to a method for assaying an ability to control a neurocyte plasticity which is dependent on a transcription regulatory factor.

BACKGROUND ART

A cranial nervous function is based on a neural circuit consisting of various neurocytes. Such a complicated and precise network is formed as a result of a correct induction of a neuroaxon into a target cell to effect a synaptic binding with a correct target cell. This induction/binding process (hereinafter referred to as an axonal guidance) involves, as a matter of course, a neuroaxonal extension control (promotion, suppression, attraction, repelling and the like).

Recent studies suggested that a reduction in the cognitive ability in response to an ordinary aging is due to a neural dysfunction rather than due to a loss of neurocytes or synapses. Such a neural dysfunction is considered to be a disturbed mechanism of the neurocyte plasticity due to a difficulty in maintaining a normal condition of the structural plasticity (remodelling; hereinafter sometimes referred to as a neurocyte plasticity) of two protrusions possessed by a neurocyte (dendrite and neuroaxon) for some reason. Similarly, the disturbed mechanism of the neurocyte plasticity is considered recently to be one of the pathogenic factors in diseases such as a mental retardation and Alzheimer-related cognition insufficiency.

Based on the understandings mentioned above, it is possible to improve diseases such as an aging-related cognition insufficiency, mental retardation and Alzheimer-related cognition insufficiency by avoiding the disturbance of the neurocyte plasticity mechanism via an appropriate control of the neurocyte plasticity.

Accordingly, it has been desired to develop a method for assaying an ability to control the neurocyte plasticity, which is essential in searching for a substance employed for controlling the neurocyte plasticity in mammalian cells.

DISCLOSURE OF THE INVENTION

Now we discovered that a specific protein (i.e., a transcription regulatory factor) exists, which controls the expression of a marker protein gene present on a neurocyte plasticising pathway such as an Eph A receptor which is a tyrosine kinase-type cell membrane receptor believed to control the neurocyte plasticity and a controlling factor referred to as a Rho GDP dissociation inhibitor (or Rho GTPase inhibitor), and established the invention by means of utilizing such a protein.

Thus, the present invention provides:

1. a method for assaying (hereinafter sometimes referred to as an assay method of the present invention) an ability to control the neurocyte plasticity which is dependent on a transcription regulatory factor comprising any of the following amino acid sequences (hereinafter sometimes referred to as present amino acid sequences), comprising the steps:

(1) a first step for bringing a test substance into contact with a mammalian cell expressing the transcription regulatory factor; and,
(2) a second step, after the first step, for measuring the expression level of a marker protein gene present on the transcription regulatory factor-dependent neurocyte plasticising pathway in the mammalian cell or an index value correlating with the level; and,
(3) a step for evaluating the ability possessed by the test substance based on the expression level or the index value correlating with the level measured in the second step,

wherein the group of the amino acid sequences being:

(a) the amino acid sequence represented by any of SEQ ID Nos.1 to 3,
(b) the amino acid sequence of a protein comprising an amino acid sequence exhibiting an amino acid identity of 90% or more to the amino acid sequence represented by any of SEQ ID Nos.1 to 3 and also having a transcription regulation ability,
(c) the amino acid sequence of a protein comprising an amino acid sequence encoded by a DNA which hybridizes under a stringent condition with a DNA consisting of the nucleotide sequence represented by the nucleotide numbers 102 to 2507 in the nucleotide sequence represented by SEQ ID No.4 and also having a transcription regulation ability,

(d) the amino acid sequence of a protein comprising an amino acid sequence encoded by a DNA which hybridizes under a stringent condition with a DNA consisting of the nucleotide sequence represented by the nucleotide numbers 51 to 2456 in the nucleotide sequence represented by SEQ ID No.5 and also having a transcription regulation ability,
(e) the amino acid sequence a protein comprising an amino acid sequence encoded by a DNA which hybridizes under a stringent condition with a DNA consisting of the nucleotide sequence represented by the nucleotide numbers 35 to 2440 in the nucleotide sequence represented by SEQ ID No.6 and also having a transcription regulation ability;

2. a method for assaying an ability to control the neurocyte plasticity which is dependent on a transcription regulatory factor comprising a present amino acid sequence, comprising the steps :

(1) a first step for bringing a test substance into contact with a transformed mammalian cell obtainable by introducing a gene comprising a nucleotide sequence encoding the present amino acid sequence; and,
(2) a second step, after the first step, for measuring the expression level of a marker protein gene present on the transcription regulatory factor-dependent neurocyte plasticising pathway in the transformed mammalian cell or an index value correlating with the level; and,
(3) a step for evaluating the ability possessed by the test substance based on the expression level or the index value correlating with the level measured in the second step;

3. an assay method according to the above 1 or 2, wherein the marker protein gene is an Eph A receptor gene or an Rho GDP dissociation inhibitor gene;
4. an assay method according to the above 1 or 2, wherein the marker protein genes are an Eph A receptor gene and an Rho GDP dissociation inhibitor gene;
5. an assay method according to the above 1 or 2, wherein the ability possessed by the test substance is evaluated based on the difference obtained by comparing the marker protein gene expression level or an index value correlating with the level in the groups employing two or more different substances independently as test substances;
6. an assay method according to the above 1 or 2, wherein at least one of the two or more different substance is a substance which does not have the ability;
7. a method for searching for a substance having an ability to control the neurocyte plasticity which is dependent on a transcription regulatory factor comprising a present amino acid sequence, wherein a substance having the ability is selected on the basis of the ability evaluated by an assay method according to the above 1 or 2 (hereinafter sometimes referred to as a searching method of the present invention);
8. a neurocyte plasticity regulator comprising as an active ingredient a substance selected by a searching method according to the above 7 or a pharmaceutically acceptable salt thereof and obtained by formulating the active ingredient into a pharmaceutically acceptable carrier (hereinafter sometimes referred to as a neurocyte plasticity regulator of the present invention);
9. a use of a gene comprising the nucleotide sequence encoding a present amino acid sequence for controlling neurocyte plasticity in a mammalian cell;
10. a use of a gene comprising the nucleotide sequence encoding a present amino acid sequence as an exogenous gene for promoting the expression of a marker protein gene present on the neurocyte plasticising pathway which is dependent on a transcription regulatory factor comprising a present amino acid sequence in a mammalian cell by means of providing the exogenous gene in a position enabling the expression in the cell; and the like.

## BEST MODE FOR CARRYING OUT THE INVENTION

The invention is further detailed below.

As used herein, a "transcription regulatory factor comprising any of the following amino acid sequences (i.e., present amino acid sequences)" means a protein having a basic helix-loop-helix (hereinafter referred to as bHLH) motif and a PAS domain (Per-Arnt-Sim homology domain) involved in the neurocyte plasticity, which binds to a DNA by forming a heterodimer whereby acting as a transcription regulatory factor. Hereinafter it may be referred to as a present transcription regulatory factor. Detailed descriptions are made later.

As used herein, a "neurocyte plasticity which is dependent on a transcription regulatory factor comprising any of the following amino acid sequences (i.e., a present amino acid sequences)" means a neurocyte plasticity in which the transcription regulatory factor comprising the present amino acid sequence (i.e., a present transcription regulatory factor) is involved and which is believed to occur as a result of a series of the cascade reactions starting from the present transcription regulatory factor. Such a series of the cascade reactions (i.e., metabolic process or signal transmission) is referred to as a "the (i.e., present amino acid sequence-carrying) transcription regulatory factor-dependent neurocyte plasticising pathway" in the present invention, and a protein capable of being utilized as a measure of the neurocyte plasticity in the invention among the proteins present on the pathway as being subjected to the expression regulation is referred to as a "marker protein present on the (i.e., present amino acid sequence-carrying) transcription regulatory factor-dependent neurocyte plasticising pathway". Those exemplified typically are an Eph A receptor, Rho GDP dissociation inhibitor (or Rho GTPase inhibitor) and the like.

In this context, the Eph A receptor is a tyrosine kinase type cell membrane receptor which is known widely to be related with the development of a brain, and several recent studies indicated the evidences of the involvement of the Eph A in the synapse plasticity, learning and memory. It is also implemented that the Eph A is involved in the potentiation of a memory, and its electrophysiological study results (LTP: long term potentiation of a synapse transmission efficiency) and behavior observation study results suggested that the activation of the Eph A is associated with the change in the cognition leading to the improvement in the memory in an adult brain. Also since the synapse plasticity is critical for the memory and the mnemonic data accumulation (as a basis of the mechanism), the activation of Eph A is considered to be associated with a neurocyte plasticising-promoting effect.

On the other hand, a Rho is an intracellular signal transmitter, which is a protein known widely to be involved in the cell growth control, cell adhesion formation regulation, actin cytoskeleton structure formation and the like. In order to control the activation of the Rho, a control factor (inhibitory protein) referred to as a Rho GDP dissociation inhibitor (or Rho GTPase inhibitor) is present. This forms a complex with a GDP-binding type of the Rho to prevent the Rho from forming a GTP-binding type (activated form of Rho), whereby inhibiting the activation of the Rho (Rho GTPase activity). Thus, the Rho GDP dissociation inhibitor is considered to regulate the neurocyte plasticity by means of controlling the GTPase activity of the Rho.

As used herein, a "transcription regulatory factor (i.e., a present transcription regulatory factor) comprising any of the following amino acid sequences (i.e., a present amino acid sequences)" is a protein consisting of any amino acid sequence included in the amino acid group consisting of (a) the amino acid sequence represented by any of SEQ ID Nos.1 to 3, (b) the amino acid sequence of a protein comprising an amino acid sequence exhibiting an amino acid identity of 90% or more to the amino acid sequence represented by any of SEQ ID Nos.1 to 3 and also having a transcription regulation ability, (c) the amino acid sequence of a protein comprising an amino acid sequence encoded by a DNA which hybridizes under a stringent condition with a DNA consisting of the nucleotide sequence represented by the nucleotide numbers 102 to 2507 in the nucleotide sequence represented by SEQ ID No.4 and also having a transcription regulation ability, (d) the amino acid sequence of a protein comprising an amino acid sequence encoded by a DNA which hybridizes under a stringent condition with a DNA consisting of the nucleotide sequence represented by the nucleotide numbers 51 to 2456 in the nucleotide sequence represented by SEQ ID No.5 and also having a transcription regulation ability and (e) the amino acid sequence of a protein comprising an amino acid sequence encoded by a DNA which hybridizes under a stringent condition with a DNA consisting of the nucleotide sequence represented by the nucleotide numbers 35 to 2440 in the nucleotide sequence represented by SEQ ID No.6 and also having a transcription regulation ability. Such a protein has a molecular weight preferably of about 80,000 to 100,000 as being measured by an SDS-PAGE.

A present transcription regulatory factor includes a protein comprising the amino acid sequence represented by any of SEQ ID Nos.1 to 3(i.e., a present amino acid sequences) (wherein the protein comprising the present amino acid sequence represented by SEQ ID No.1 is a human-derived present transcription regulatory factor, which may sometimes be designated as hNXF; the protein comprising the present amino acid sequence represented by SEQ ID No.2 is a mouse-derived present transcription regulatory factor, which may sometimes be designated as mNXF; the protein comprising the present amino acid sequence represented by SEQ ID No.3 is a rat-derived present transcription regulatory factor, which may sometimes be designated as rNXF), a protein comprising an amino acid sequence exhibiting an amino acid identity of 90% or more to the amino acid sequence represented by any of SEQ ID Nos.1 to 3 and also having a transcription regulation ability, a protein comprising an amino acid sequence encoded by a DNA which hybridizes under a stringent condition with a DNA consisting of the nucleotide sequence represented by the nucleotide numbers 102 to 2507 in the nucleotide sequence represented by SEQ ID No.4 and also having a transcription regulation ability, a protein comprising an amino acid sequence encoded by a DNA which hybridizes under a stringent condition with a DNA consisting of the nucleotide sequence represented by the nucleotide numbers 51 to 2456 in the nucleotide sequence represented by SEQ ID No.5 and also having a transcription regulation ability and a protein comprising an amino acid sequence encoded by a DNA which hybridizes under a stringent condition with a DNA consisting of the nucleotide sequence represented by the nucleotide numbers 35 to 2440 in the nucleotide sequence represented by SEQ ID No.6 and also having a transcription regulation ability.

The difference from the amino acid sequence represented by any of SEQ ID Nos.1 to 3 observed in the amino acid sequence of a present transcription regulatory factor may for example be a variation such as the deletion, substitution, modification and addition of amino acids. Such a variation includes a variation which can artificially be introduced by means of a site-directed mutagenesis method or a mutagenic treatment as well as a polymorphic variation which occurs naturally such as a difference in an amino acid sequence resulting from the difference by the animal line, individual, organ and tissue.

In the invention, the "amino acid identity" means an identity and a homology in the amino acid sequence between two proteins. The "amino acid identity" described above can be determined by comparing two amino acid sequence which are aligned optimally over the entire range of a reference amino acid. A reference protein here may have an addition or deletion (for example, a gap) in the optimal alignment of the two amino acid sequences. Such an amino acid identity can be calculated for example by producing an alignment utilizing a Clustal W algorism [Nucleic Acid Res., 22 (22): 4673-4680 (1994)] using a Vector NTI. The amino acid identity can be investigated also by using a sequence analysis software, typically Vector NTI, GENETYX-MAC or any other analytical tools provide DNA public database. Such a public database can generally be available for example in the following URL: http://www.ddbj.nig.ac.jp.

A preferred amino acid identity in the invention may for example be 90% or higher.

A "DNA which hybridizes under a stringent condition" described above may for example be a DNA capable of maintaining a hybrid, which was formed previously as a DNA-DNA hybrid by a hybridization at 65°C at a high ion concentration [for example using 6 X SSC (900 mM sodium chloride, 90 mM sodium citrate)], even after washing for 30 minutes at 65°C at a low ion concentration [for example using 0.1 X SSC (15 mM sodium chloride, 1.5 mM sodium citrate)]. The transcription regulation ability of a present transcription regulatory factor can be evaluated based for example on an assay using a reporter gene described below.

A gene comprising the nucleotide sequence encoding the amino acid sequence of a present transcription regulatory factor (hereinafter referred to as a present transcription regulatory factor gene) may be obtained for example from a tissue of an animal such as human, mouse, rat and the like in accordance with a genetic engineering method described for example in J. Sambrook, E.F.Frisch, T.Maniatis, Molecular Cloning, 2nd Edition, Cold Spring Harbor Laboratory (1989).

Typically, a total RNA derived from a tissue of an animal such as human, mouse and rat is first prepared. For example, a brain tissue is pulverized in a solution containing a protein denaturant such as guanidine hydrochloride or guanidine thiocyanate, and then the pulverized material is treated with phenol, chloroform and the like, to denature the protein. The denatured protein is removed for example by a centrifugation to obtain a supernatant, from which the total RNA is extracted by a guanidine hydrochloride/phenol method, SDS-phenol method, guanidine thiocyanate/CsCl method and the like. A commercially available kit based on the methods described above may for example be ISOGEN (NIPPON GENE)-. The resultant total RNA is used as a template and an oligo dT primer is annealed to a poly A sequence of the RNA, whereby synthesizing a single-stranded cDNA using a reverse transcriptase. Then, the synthesized single-stranded cDNA is used as a template together with a primer which is an RNA obtained by inserting a nick and a gap into the RNA chain using an E.coli RnaseH, whereby synthesizing a double-stranded cDNA using an E.coli DNA polymerase I. Subsequently, the both ends of the synthesized double-stranded cDNA is made blunt using a T4 DNA polymerase. The double-stranded cDNA having both blunt ends is purified and recovered by means of a standard procedure such as a phenol-chloroform extraction and ethanol precipitation. A commercially available kit based on the methods described above may for example be a cDNA synthesis system plus (Amarsham Pharmacia Biotech) or a TimeSaver cDNA synthesis kit (Amarsham Pharmacia Biotech). Then the resultant double-stranded cDNA is ligated to a vector such as a plasmid pUC118 or phage λgt10 using a ligase to prepare a cDNA library. As such a cDNA library, a commercially available cDNA library (GIBCO-BPL or Clontech) may also be employed.

Alternatively, a genomic DNA may be prepared from a tissue sample of an animal such as human, mouse and rat in accordance with a standard method described for example in J. Sambrook, E.F.Frisch, T.Maniatis, Molecular Cloning, 2nd Edition, Cold Spring Harbor Laboratory (1989), or M. Muramatsu, "Labomanual genetic engineering" (Maruzen, 1988). For example, when the sample is a hair, 2 or 3 hairs are washed with a sterilized water and then with ethanol, cut into 2 to 3 mm pieces, which are combined with 200 μl of a BCL-Buffer [10mM Tris-HCl (pH7.5), 5mM $MgCl_2$, 0.32 sucrose, 1 Triton X-100] followed by a Proteinase K at the final concentration of 100 μl/ml and SDS at the final concentration of 0.5 (w/v). The mixture thus obtained is incubated at 70°C for 1 hour, and then subjected to a phenol/chloroform extraction to obtain a genomic DNA. When the sample is a peripheral blood, the sample is treated using a DNA-Extraction kit (Stratagene) and the like to obtain a genomic DNA. The resultant genomic DNA is ligated to a vector such as a λgt10 using a ligase to obtain a genomic DNA library. As such a genomic DNA library, a commercially available genomic DNA library (Stratagene) may also be employed.

From a cDNA library or genomic DNA library obtained as described above, a present transcription regulatory factor gene can be obtained for example by a polymerase chain reaction (hereinafter abbreviated as PCR) using as a primer an oligonucleotide comprising a partial nucleotide sequence of the nucleotide sequence represented by SEQ ID No.4, 5, 6 or 54 or the nucleotide sequence complementary to said partial nucleotide sequence or by a hybridization method using as a probe a DNA comprising the nucleotide sequence represented by SEQ ID No.4, 5, 6 or 54 or a partial nucleotide sequence of said partial nucleotide sequence.

A primer employed in a PCR may for example be an oligonucleotide having a length of about 10 nucleotides to about 50 nucleotides which is an oligonucleotide comprising a nucleotide sequence selected from a 5' non-translation region of the nucleotide sequence represented by SEQ ID No.4, 5, 6 or 54 and which is an oligonucleotide comprising the nucleotide sequence complementary to a nucleotide sequence selected from a 3' non-translation region of the nucleotide sequence represented by SEQ ID No.4, 5, 6 or 54. Typically, the forward primer may for example be the oligonucleotide consisting of the nucleotide sequence represented by SEQ ID NO.7 and the oligonucleotide consisting of the nucleotide sequence represented by SEQ ID NO.8. The reverse primer may for example be the oligonucleotide consisting of the nucleotide sequence represented by SEQ ID NO.9 and the oligonucleotide consisting of the nucleotide sequence represented by SEQ ID NO.10. An example of the PCR condition involves an incubation in 50 μl of a reaction solution containing 5 μl of a 10-fold diluted buffer for a LA-Taq polymerase (Takara), 5 μl of a 2.5 mM dNTP mixture (each 2.5mM dATP, dGTP, dCTP and dTTP) (the final concentration of each of dATP, dGTP, dCTP and dTTP is 0.25 mM), each 0.25 to 1.25 μl of 20 μM primers (final concentration of 0.1 to 0.5 μM), 0.1 to 0.5 μg of a template cDNA and 1.25 units of a LA-Taq polymerase (Takara) for 1 minutes at 95°C followed by 3 minutes at 68°C in a single cycle, the cycle being repeated 35 times.

A probe employed in a hybridization method may for example be the DNA consisting of the nucleotide sequence represented by the nucleotide numbers 102 to 2507 in the nucleotide sequence represented by SEQ ID No.4, a DNA consisting of the nucleotide sequence represented by the nucleotide numbers 51 to 2456 in the nucleotide sequence represented by SEQ ID No.5, a DNA consisting of the nucleotide sequence represented by the nucleotide numbers 35 to 2440 in the nucleotide sequence represented by SEQ ID No.6, a DNA consisting of the nucleotide sequence represented by the nucleotide numbers 1419 to 6164 in the nucleotide sequence represented by SEQ ID No.54 and the like. An example of the hybridization condition involves an incubation at 65°C in the presence of 6 x SSC (0.9M sodium chloride, 0.09M sodium citrate), 5 x Denhart's solution (0.1 (w/v) ficoll 400, 0.1 (w/v) polyvinyl pyrrolidone), 0.1 (w/v) BSA), 0.5 (w/v) SDS and 100 μg/ml denatured salmon sperm DNA followed by an incubation at room temperature for 15 minutes in the presence of 1 x SSC (0.15M sodium chloride, 0.015M sodium citrate) and 0.5 (w/v) SDS, followed by an incubation at 68°C for 30 minutes in the presence of 0.1 x SSC (0.015M sodium chloride, 0.0015M sodium citrate) and 0.5 (w/v) SDS. Alternatively, an incubation at 65°C in the presence of 5 x SSC, 50mM HEPES, pH7.0, 10 x Denhart's solution and 20 μg/ml denatured salmon sperm DNA followed by an incubation at room temperature for 30 minutes in 2 x SSC, followed by an incubation at 65°C for 40 minutes in 0.1 x SSC, which is repeated twice, may also be exemplified.

A present transcription regulatory factor gene can be prepared also by performing a chemical synthesis of a nucleic acid in accordance with a standard method such as a phosphite triester method (Hunkapiller, M. et al., Nature, 310, 105, 1984) based on the nucleotide sequence represented by SEQ ID NO.4, 5, 6 or 54.

A present transcription regulatory factor gene thus obtained can be cloned into a vector in accordance with a genetic engineering method described in J. Sambrook, E.F.Frisch, T.Maniatis, Molecular Cloning, 2nd Edition, Cold Spring Harbor Laboratory (1989). Typically, the cloning can for example be performed using a TA cloning kit (Invitrogen) or a commercially available plasmid vector such as pBluescriptII (Stratagene).

The nucleotide sequence of a resultant inventive DNA can be identified by a Maxam Gilbert method (described for example in Maxam, A. M. & W. Glibert, Proc. Natl. Acad. Sci. USA, 74, 560, 1997) or a Sanger method (described for example in Sanger, F. & A. R. Coulson, J. Mol. Biol., 94, 441, 1975, Sanger, F. & Nicklen and A.R. Coulson., Proc. Natl. Acad. Sci. USA, 74, 5463, 1997).

A typical example of a present transcription regulatory factor gene may for example be the DNA consisting of the nucleotide sequence represented by the nucleotide numbers 102 to 2507 in the nucleotide sequence represented by SEQ ID No.4, a DNA consisting of the nucleotide sequence represented by the nucleotide numbers 51 to 2456 in the nucleotide sequence represented by SEQ ID No.5, a DNA consisting of the nucleotide sequence represented by the nucleotide numbers 35 to 2440 in the nucleotide sequence represented by SEQ ID No.6, a DNA consisting of the nucleotide sequence represented by the nucleotide numbers 1419 to 6164 in the nucleotide sequence represented by SEQ ID No.54 and the like.

A present transcription regulatory factor gene is, as described above and below, can be utilized also for promoting the expression of a marker protein gene present on a present transcription regulatory factor-dependent neurocyte plasticising pathway in a mammalian cell by means of providing the DNA in the mammalian cell as an exogenous gene in a position enabling the expression in the cell.

A present transcription regulatory factor gene vector can be constructed by integrating a present transcription regulatory factor gene, in accordance with a standard genetic engineering method, into a vector capable of being utilized in a host cell to which said gene is introduced (hereinafter referred to as a basic vector), such as a vector which contains a gene information capable of being replicated in the host cell, which can independently be proliferated, which can be isolated and purified from the host cell and which has a detectable marker.

A basic vector which can be employed for constructing A present transcription regulatory factor gene vector may for example be a plasmid pUC119 (Takara) or phagimid pBluescriptII (Stratagene) when using a coliform as a host cell. When using a budding yeast as a host cell, then plasmids pGBT9, pGAD242, pACT2 (Clontech) may be exemplified. When using a mammalian cell as a host cell, a vector containing an autonomous replication origin derived from a virus such as pRc/RSV, pRc/CMV (Invitrogen), bovine papilloma virus plasmid pBV (Amarsham Pharmacia Biotech) or EB virus plasmid pCEP4 (Invitrogen) and a virus such as a vaccinia virus may be exemplified, while an insect virus such as a baculovirus may be exemplified when using a insect cell as a host cell.

In order to integrate a present transcription regulatory factor gene into a virus such as a baculovirus or vaccinia virus, a transfer vector containing a nucleotide sequence homologous to the genome of a virus to be employed can be used. Such a transfer vector is typically a plasmid available from Pharmingen such as pVL1372, pVL1393 (Smith, G. E., Summers M.E. et al., Mol. Cell Biol., 3, 2156-2165 (1983) and pSFB5 (Funahashi, S. et al., J. Virol., 65, 5584-5588 (1991). When a present transcription regulatory factor gene is inserted into a transfer vector described above and the transfer vector and the genome of a virus are introduced into a host cell simultaneously, a homologous recombination occurs between the transfer vector and the genome of the virus, whereby obtaining a virus into whose genome the present transcription regulatory factor gene is integrated. The genome of a virus may be the genome for example of Baculovirus, Adenovirus, Vacciniavirus and the like.

More specifically, a present transcription regulatory factor gene is integrated for example into a baculovirus by inserting the present transcription regulatory factor gene into a multiple cloning site of a transfer vector such as pVL1393 or pBL1392 followed by introducing the DNA of said transfer vector and a baculovirus genomic DNA (Baculogold; Pharmingen) into an insect cell line Sf21 (available from ATCC) for example by a calcium phosphate method followed by incubating the resulting cell. A virus particle containing the genome of the virus into which the present transcription regulatory factor gene has been inserted is recovered from the culture medium for example by a centrifugation, and then made free from proteins using phenol and the like, whereby obtaining the genome of the virus containing the present transcription regulatory factor gene. Subsequently, the genome of said virus is introduced into a host cell having a virus particle forming ability such as an insect cell line Sf21 for example by a calcium phosphate method and the resultant cell is incubated, whereby proliferating the virus particle containing the present transcription regulatory factor gene.

On the other hand, a relatively small genome such as that of a mouse leukemia retrovirus can directly be integrated with a present transcription regulatory factor gene without using any transfer vector. For example, a virus vector DC(X) (Eli Gilboa et al., BioTechniques, 4, 504-512 (1986)) is integrated with a present transcription regulatory factor gene on its cloning site. The resultant virus vector into which the present transcription regulatory factor gene has been integrated is introduced into a packaging cell such as an Ampli-GPE (J. Virol., 66, 3755 (1992)), whereby obtaining a virus particle containing the genome of the virus into which the present transcription regulatory factor gene has been inserted.

A promoter capable of functioning in a host cell is operably connected to the upstream of a present transcription regulatory factor gene and then integrated into a basic vector such as those described above, whereby constructing an present transcription regulatory factor gene vector capable of allowing the present transcription regulatory factor gene to be expressed in the host cell. The expression "operably connected" means that a promoter and a present transcription regulatory factor gene are bound to each other in a condition which allows the present transcription regulatory factor gene is expressed under the control of the promoter in a host cell into which the present transcription regulatory factor gene is to be introduced. A promoter capable of functioning in a host cell may for example be a DNA exhibiting a promoter activity in a host cell into which it is to be introduced. Those which may be exemplified when the host cell is a coliform cell are E.coli lactose operon promoter (lacP), tryptophan operon promoter (trpP), arginine operon promoter (argP), galactose operon promoter (galP), tac promoter, T7 promoter, T3 promoter, λ phage promoter (λ-pL, λ-pR) and the like, while those which may be exemplified when the host cell is an animal cell or fission yeast are Rous: sarcoma virus (RSV) promoter, cytomegalovirus (CMV) promoter, simian virus (SV40) early or late promoter, mouse mammary tumor virus (MMTV) promoter and the like. Those which may be exemplified when the host cell is a budding yeast are an ADH1 promoter and the like (the ADH1 promoter can be prepared by a standard genetic engineering method for example from an yeast expression vector pAAH5 comprising an ADH1 promoter and terminator [available from Washington Research Foundation, Ammerer et al., Method in Enzymology, 101 part (p.192-201)]; the ADH1 promoter is encompassed in the United State Patent Application 299,733 by Washington Research Foundation, and should be used industrially or commercially in United States only after obtaining the approval from the claimant).

When a basic vector which initially possesses a promoter capable of functioning in a host cell is employed, a present transcription regulatory factor gene may be inserted to the downstream of said promoter so that the vector-possessed promoter and the present transcription regulatory factor gene are operably connected to each other. For example, each of the plasmids such as pRc/RSV and pRc/CMV described above is provided with a cloning site downstream of a promoter capable of functioning in an animal cell, and by inserting a present transcription regulatory factor gene into said cloning site followed by a introduction into an animal cell, the present transcription regulatory factor gene can be expressed. Since any of these plasmids has previously been integrated with a SV40 autonomous replication origin, the introduction of said plasmid into a host cell which has been transformed with an SV40 genome from which an ori is deleted, such as a COS cell, leads to an extremely increased number of the intracellular plasmid copies, resulting in a high expression of the present transcription regulatory factor gene which has been integrated into said plasmid. Also since the plasmid pACT2 for yeast described above possesses an ADH1 promoter, an present transcription regulatory factor gene vector capable of allowing a present transcription regulatory factor gene to be expressed highly in a budding yeast such as CG1945 (Clontech) can be constructed by inserting the present transcription regulatory factor gene into the downstream of the ADH1 promoter of said plasmid or a derivative thereof.

By introducing a constructed present transcription regulatory factor gene vector into a host cell, a transformant can be obtained. A method for introducing a present transcription regulatory factor gene vector into a host cell may be a standard introducing method suitable for the host cell. For example, when E.coli is employed as a host cell, a standard method such as a calcium chloride method or electroporation described for example in J. Sambrook, E.F.Frisch, T.Maniatis, Molecular Cloning, 2nd Edition, Cold Spring Harbor Laboratory (1989) may be employed. When a mammalian cell or insect cell is employed as a host cell, the introduction into a cell described above can be effected in accordance with a general gene introduction method such as a calcium phosphate method, DEAE dextran method, electroporation, lipofection and the like. When an yeast is employed as a host cell, the introduction can be effected for example by means of an Yeast transformation kit (Clontech) based on a lithium method.

When a virus is employed as a vector, the genome of the virus can be introduced into a host cell by a standard gene introduction method described above, or a virus particle containing the genome of the virus into which a present transcription regulatory factor gene has been inserted is infected to a host cell, whereby introducing the genome of said virus into the host cell.

In order to screen for the transformant, a marker gene is introduced into a host cell simultaneously with a present transcription regulatory factor gene vector and the cell is cultured in a manner suitable to the nature of the marker gene. For example, when the marker gene is a gene which impart the host cell with a resistance to a lethally active screening drug, then the cell into which the present transcription regulatory factor gene vector has been introduced is cultured in a medium supplemented with said drug. The combination of such a drug resistance imparting gene and a screening drug may for example be the combination of a neomycin resistance imparting gene with neomycin, the combination of a hygromycin resistance imparting gene with hygromycin, and the combination of blasticidin S resistance imparting gene and blasticidin S. When the marker gene is a gene which compensates the auxotrophic nature of the host cell, then a minimum medium free from the relevant nutrition is used to culture the cell into which the present transcription regulatory factor gene vector has been introduced.

In order to obtain a transformant generated as a result of the introduction of a present transcription regulatory factor gene into a chromosome of a host cell, a present transcription regulatory factor gene vector and a marker gene-carrying vector are made linear by a digestion with restriction enzymes, and then introduced as described above into a host cell, which is cultured usually for several weeks to screen for an intended transformant on the basis of the expression of the introduced marker gene. Alternatively, it is also possible to screen for a transformant generated as a result of the introduction of a present transcription regulatory factor gene into a chromosome of a host cell by introducing a present transcription regulatory factor gene vector comprising as a marker gene a gene providing a resistance to a screening drug describe above into a host cell as described above, subculturing this cell for several weeks in a medium supplemented with the screening drug, and then incubating a selected drug resistance clone surviving as a colony in a pure culture manner. In order to verify that the introduced present transcription regulatory factor gene has surely been integrated into the chromosome of the host cell, a standard genetic engineering method may be employed to prepare the genomic DNA of the cell, from which the presence of the present transcription regulatory factor gene is detected by a PCR using as a primer an oligonucleotide comprising a partial nucleotide sequence of the introduced present transcription regulatory factor gene or by a southern hybridization method using as a probe the introduced present transcription regulatory factor gene. Since such a transformant can be stored frozen and can be made viable upon any need of use, it allows the step for producing the transformant at every time of the experiment to be omitted, and allows the experiment to be conducted using a transformant whose characteristics and the handling condition for which are well established.

By culturing a transformant obtained as described above, a present transcription regulatory factor can be produced.

For example, when a transformant described above is a microorganism, this transformant can be cultured using any culture medium containing carbon sources, nitrogen sources, organic salts and inorganic salts, as appropriate, used in an ordinary culture of an ordinary microorganism. The culture can be conducted in accordance with a usual procedure for an ordinary microorganism, such as a solid culture, liquid culture (rotary shaking culture, reciprocal shaking culture, Jar Fermenter, tank culture and the like). The culture temperature and the pH of the medium may appropriately be selected from the range enabling the growth of the microorganisms, and the culture is conducted usually at a temperature of about 15°C to about 40°C at a pH of about 6 to about 8. The culture time period is usually about 1 day to about 5 days, although it may vary depending on various culture conditions. When an expression vector comprising a promoter of a temperature shift type or an induction type such as an IPTG induction type, the induction time is preferably within 1 day, usually several hours.

When a transformant described above is an animal cell such as an insect cell, then the transformant can be cultured using a culture medium employed in an ordinary culture of an ordinary cell. When such a transformant was prepared using a screening drug, then the culture is conducted preferably in the presence of the relevant drug. In the case of a mammalian cell, the culture is conducted for example in a DMEM medium supplemented with FBS at the final concentration of 10% (v/v) (NISSUI and the like) at 37°C in the presence of 5% $CO_2$ with replacing the culture medium with a fresh medium every several days. When the culture became confluent, a PBS solution supplemented with trypsin for example at a concentration of about 0.25 (w/v) is added to disperse the culture into individual cells, which are subjected to a several-fold dilution and then inoculated to new dishes where they are further cultured. Similarly in the case of an insect cell, an insect cell culture medium such as a Grace's medium containing 10% (v/v) FBS and 2% (w/v) Yeastlate is employed to conduct the culture at a temperature of 25°C to 35°C. In this case, a cell which tends to be peeled off from a dish easily such as a Sf21 cell can be dispersed by pipetting instead of using a trypsin solution, whereby continuing the subculture. In the case of a transformant containing a vector of a virus such as a baculovirus, the culture time period is preferably shorter than the time period allowing a cytoplasm effect to be evident to cause the cell death, for example up to 72 hours after the virus infection.

A present transcription regulatory factor produced by a transformant described above can be recovered appropriately by a combination of ordinary isolation and purification methods, and a fraction containing the present transcription regulatory factor can be obtained by collecting the transformant cells by a centrifugation after completion of the culture, suspending the collected cells in an ordinary buffer solution, pelletizing the cells for example using Polytron, ultrasonic treatment, Dounce homogenizer and the like, and then centrifuging the pelletized cell fluid to recover the supernatant. A further purified present transcription regulatory factor can be recovered by subjecting the supernatant fraction described above to various chromatographic procedures such as ion exchange chromatography, hydrophobic interaction chromatography, gel filtration chromatography, affinity chromatography and the like. When a present transcription regulatory factor or a polypeptide comprising its partial amino acid sequence is produced as a fusion protein with GST, the purification can be accomplished by an affinity chromatography using a glutathione sepharose (Amersham Pharmacia).

A present transcription regulatory factor thus produced can be employed as an immune antigen for producing an antibody which recognizes a present transcription regulatory factor or a polypeptide comprising its partial amino acid sequence, and can also be employed in an assay for screening for a substance which binds to the present transcription regulatory factor.

Using a present transcription regulatory factor produced as described above as an immune antigen, an animal such as mouse, rabbit, chicken and the like is immunized in accordance with an immunological procedure described in Frederick M.Ausubel et al., Short Protocols in Molecular Biology 3nd Edition, John Wiley & Sons, Inc, whereby producing an antibody which recognizes a present transcription regulatory factor or a polypeptide comprising its partial amino acid sequence. More typically and in one example, a present transcription regulatory factor as an antigen is mixed with a complete Freunds adjuvant to form an emulsion. The resultant emulsion is administered subcutaneously to a rabbit. After about 4 weeks, an antigen emulsified in an incomplete Freunds adjuvant is administered. If necessary, a similar administration is further conducted every two weeks. The blood is sampled to obtain a serum fraction, the antibody titre of which against the present transcription regulatory factor is then verified. The resultant serum fraction having the antibody titre which recognizes the present transcription regulatory factor or a polypeptide comprising its partial amino acid sequence is fractionated in accordance for example with an ordinary ammonium sulfate sedimentation method, whereby obtaining an IgG which recognizes the present transcription regulatory factor or a polypeptide comprising its partial amino acid sequence.

Alternatively, a polypeptide comprising a partial amino acid sequence of a present transcription regulatory factor is synthesized chemically and administered as an immune antigen to an animal, whereby producing an antibody which recognizes the present transcription regulatory factor or a polypeptide comprising its partial amino acid sequence. As the amino acid sequence of a polypeptide employed as an immune antigen, an amino acid sequence which has as a low homology as possible with the amino acid sequences of other proteins and which has many differences from the amino acid sequence of a present transcription regulatory factor possessed by an animal species to be immunized is selected for example from the amino acid sequences represented by SEQ ID Nos.1 to 3. A polypeptide having a length of 10 amino acids to 15 amino acids consisting of the selected amino acid sequence is synthesized chemically by a standard method and crosslinked for example with a carrier protein such as Limulus plyhemus hemocyanin using MBS and-the like and then used to immunize an animal such as a rabbit as described above.

As a result, an antibody recognizing a polypeptide comprising a present transcription regulatory factor or a partial amino acid sequence thereof can be produced.

In order to assay an ability to control the neurocyte plasticity which is dependent on a present transcription regulatory factor thus produced (hereinafter sometimes referred to as a present neurocyte plasticity-controlling ability), an assay method (i.e., an assay method of the present invention) comprising the steps: (1) a first step for bringing a test substance into contact with a mammalian cell expressing a present transcription regulatory factor; and, (2) a second step, after the first step, for measuring the expression level of a marker protein gene present on the transcription regulatory factor-dependent neurocyte plasticising pathway in the mammalian cell or an index value correlating with the level; and a step for evaluating the ability possessed by the test substance based on the expression level (measured in the second step) or the index value correlating with the level can be employed. In this steps, a difference is investigated by comparing the marker protein gene expression level or an index value (first measured value, second measured value) correlating with the level in the groups employing two or more different substances independently as test substances. Based on the difference thus obtained (difference between the first measured value and the second measured value), the present neurocyte plasticity-controlling ability possessed by the test substance described above is evaluated, whereby accomplishing the assay of the ability. Based on the present neurocyte plasticity-controlling ability thus evaluated, the substance is identified as a substance having the present neurocyte plasticity-controlling ability.

In the method described above, it is possible to use at least one of the two or more different substance described above as a substance which does not have a present neurocyte plasticity-controlling ability whereby evaluating the present neurocyte plasticity-controlling ability possessed by any of the other test substances, and it is also possible to use the present neurocyte plasticity-controlling ability possessed by at least one of the two or more different substance described above as a standard for evaluating the present neurocyte plasticity-controlling ability of any of the other test substances.

Thus, a present transcription regulatory factor can be utilized in a method for effectively analyzing the expression level of a marker protein gene present on the transcription regulatory factor-dependent neurocyte plasticising pathway or an index value correlating with the level which is essential for assaying a present neurocyte plasticity-controlling ability possessed by a substance.

A mammalian cell employed in an assay method of the present invention may be a cell isolated from a tissue, or a cell forming a population having identical functions and morphology, or a cell present in the body of a mammal. It is also possible to use an extracted system of the cell mentioned above. The origin of the cell may for example be a mammal, typically human, monkey, cattle, rabbit, mouse, rat, hamster and the like.

In a first step of an assay method of the present invention, the concentration of a test substance to be brought into contact with a mammalian cell which expresses a present transcription regulatory factor may usually be about 0.1 gM to about 100 μM, preferably 1 μM to 50 μM. The time period during which the mammalian cell is in contact with the test substance is usually 10 minutes to 2 days, preferably several hours to 1 days.

The environment in which a test substance is brought into contact with a mammalian cell mentioned above is preferably an environment allowing the viable activity of the mammalian cell to be maintained, for example an environment in which an energy source for the mammalian cell is coexisting. Typically, it is convenient to perform a first step in a culture medium.

It is also possible to perform a first step of an assay method of the present invention for example by bringing a test substance into contact with a transformed mammalian cell (hereinafter sometimes referred to as a present transformed mammalian cell) obtainable by introducing a gene comprising a nucleotide sequence encoding the present amino acid sequence into the mammalian cell.

In this step, the concentration of a test substance to be brought into contact with a present transformed mammalian cell may usually be about 0.1 μM o about 100 μM, preferably 1 μM to 50 μM. The time period during which the transformed mammalian cell is in contact with the test substance is usually 10 minutes to 2 days.

A present transformed mammalian cell can be prepared as follows.

A present transcription regulatory factor gene is inserted using an ordinary genetic engineering technology into a vector capable of being used in a mammalian cell into which the present transcription regulatory factor gene is introduced in such a manner it is connected to a promoter in an expressible form to form a plasmid. The promoter employed here may be one which is functional in the mammalian cell into which the present transcription regulatory factor gene is introduced, such as an SV40 virus promoter, cytomegalovirus promoter (CMV promoter), Raus sarcoma virus promoter (RSV promoter), β actin gene promoter and the like. It is also possible to use a commercially available vector containing any of these promoters upstream of the multiple cloning site.

Then, the plasmid is introduced into a mammalian cell. A method for such a introduction into the mammalian cell may for example be a calcium phosphate method, electroinduction, DEAE dextran method, micelle formation and the like. The calcium phosphate method may be a method described in Grimm, S. et al., Proc. Natl. Acad. Sci. USA, 93, 10923-10927, the electroinduciton and DEAE dextran method may for example be those described in Ting, A.T. et al. EMBO J., 15, 6189-6196, and the micelle formation may for example be a method described in Hawkins, C.J. et al., Proc. Natl. Acad. Sci.USA, 93, 13786-13790. When a micelle formation is employed, a commercially available reagent such as Lipofectamine (Gibco) or Fugene (Boehringer) may be utilized.

A mammalian cell which has been introduced with a plasmid described above is cultured in a medium providing a screening condition suitable to a screening marker gene for example by utilizing the screening marker gene which has previously been contained in vector, whereby screening for the transformed mammalian cell. It is also possible to screen further continuously to obtain the transformed mammalian cell which now became a stable transformed mammalian cell into whose chromosome the present transcription regulatory factor gene has been introduced. In order to verify that the introduced present transcription regulatory factor gene has been integrated into a chromosome present in the mammalian cell, the genomic DNA of the cell may be produced in accordance with an ordinary genetic engineering technology and then the presence of the present transcription regulatory factor gene in the genomic DNA may be detected and identified by means of a PCR employing a DNA comprising a partial nucleotide sequence of the present transcription regulatory factor gene as a primer, or by a southern hybridization method employing a DNA comprising a partial nucleotide sequence of the present transcription regulatory factor gene as a probe.

A present transformed mammalian cell may be prepared also from a transformed non-human animal tissue described below by an ordinary procedure.

In an assay method of the present invention, a method for measuring the expression level of a marker protein gene present on a present transcription regulatory factor-dependent neurocyte plasticising pathway or an index value correlating with the level may for example be:

(1) a method for measuring the level of the marker protein described above for example by a radioimmunoassay (RIA) employing an antibody which recognizes specifically the marker protein, an enzyme linked immunosorbent assay (ELISA), western blotting and the like;
(2) a method for measuring the level, in the mammalian cell, of an mRNA of a marker protein as an index value correlating with the marker protein;
(3) a method for measuring the level of a marker protein using a DNA array or DNA chip onto which an oligonucleotide capable of being hybridized with the marker gene to enable the measurement of the level of an mRNA of the marker protein has been immobilized.

The quantification of an mRNA in the procedure (2) described above may employ an ordinary method such as an RT-PCR or northern hybridization (for example see, J. Sambrook, E.F.Frisch, T.Maniatis,Molecular Cloning, 2nd Edition, Cold Spring Harbor Laboratory Press).

Based on the difference obtained by comparing the marker protein gene expression level or an index value correlating with the level in the groups employing two or more different substances independently as test substances which has been measured by an assay method of the present invention as described above, the present neurocyte plasticity-controlling ability of a substance mentioned above is evaluated. Thus, the present neurocyte plasticity-controlling ability possessed by a test substance can be assayed.

In an assay method of the present invention, it is possible to use at least one of the two or more different substance described above as a substance which does not have a present neurocyte plasticity-controlling ability (for example, solvent, test system solution serving as a background) whereby evaluating the present neurocyte plasticity-controlling ability possessed by any of the other test substances, and it is also possible to use the present neurocyte plasticity-controlling ability possessed by at least one of the two or more different substance described above as a standard for evaluating the present neurocyte plasticity-controlling ability of any of the other test substances.

It is also possible to compare the expression level of a marker protein gene relevant to a present transcription regulatory factor or an index value correlating with the level (hereinafter referred to as a measured value 1) with the expression level of the marker protein gene or the index value correlating with the level observed in a mammalian cell without any contact between the present transcription regulatory factor and a test substance (hereinafter referred to as a measured value 2), whereby evaluating the present neurocyte plasticity-controlling ability of the test substance. In such a case, the present neurocyte plasticity-controlling ability may be determined as a % control using the measured values described above in accordance with the following equation.

$$\% \text{ Control} = [(\text{Measured value 1} - \text{Measured value 2})/\text{Measured value 2}] \times 100$$

In order to search for a substance having a present neurocyte plasticity-controlling ability, a substance having the present neurocyte plasticity-controlling ability may be selected based on the present neurocyte plasticity-controlling ability evaluated by an assay method of the present invention(a searching method of the present invention).

For example, a test substance whose absolute value of a % control indicating a present neurocyte plasticity-controlling ability is a statistically significant value, preferably 30% or higher, more preferably 50% or higher, is selected as a substance having the present neurocyte plasticity-controlling ability. When the % control is a positive value, the substance is selected as one having a. promoting ability, while the substance is selected as one having an inhibiting ability when the % control is a negative value.

Such a substance may be any substance such as a low molecular weight compound, protein (including antibody) or peptide, as long as it has a present neurocyte plasticity-controlling ability.

A substance searched for by a searching method of the present invention has a present neurocyte plasticity-controlling ability, and may be used as an active ingredient of a neurocyte plasticity regulator (for example, therapeutic agent against cognitive ability insufficiency and mental retardation).

A neurocyte plasticity regulator containing as an active ingredient a substance selected by a searching method of the present invention or a pharmaceutically acceptable salt thereof (thus, an inventive neurocyte plasticity regulator) can be administered at an effective dose orally or parenterally to a mammalian animal such as human. For example, the inventive neurocyte plasticity regulator when administered orally can be given in an ordinary form such as a tablet, capsule, syrup and suspension. When the inventive neurocyte plasticity regulator given parenterally, it can be administered in an ordinary liquid form such as a solution, emulsion and suspension. A method for administering the inventive neurocyte plasticity regulator in a form described above parenterally may for example be an injection or a rectal administration of a suppository.

Such a suitable dosage form can be produced by incorporating a substance selected by a searching method of the present invention or a pharmaceutically acceptable salt thereof into a pharmaceutically acceptable ordinary carrier, excipient, binder, stabilizer, diluent and the like. When employing an injection formulation, it may be possible to add acceptable buffering agents, solubilizing aids, osmotic agents and the like.

The dosage may vary depending on the age and the sex of the mammalian subject, degree of the disease, type of the arteriosclerotic condition exacerbation factor secretion inhibitor, dosage form and the like, the oral dose is usually about 1 mg to about 2 g as an active ingredient per day in an adult, preferably about 5 mg to about 1 g, while the injection may be given at about 0.1 mg to about 500 mg as an active ingredient in an adult. Such a daily dose may be given all at once or in several portions.

A disease indicated for an inventive neurocyte plasticity regulator may for example be a senile cognition insufficiency, mental retardation and Alzheimer-related cognition insufficiency.

The present invention also provides a utilization of a present transcription regulatory factor gene for promoting the expression of a marker protein gene present on a present transcription regulatory factor-dependent neurocyte plasticising pathway in a mammalian cell by means of providing the present transcription regulatory factor gene in the mammalian cell as an exogenous gene in a position enabling the expression in the cell.

Such a mammalian cell may for example be a cell derived from a mammal such as human, monkey, mouse, rat, hamster and the like. Such a cell may be a cell which constitutes a population having identical functions and morphologies, or a cell present in the body of said mammalian animal.

Accordingly, when the mammalian animal is a human, a range from a human receiving a so called gene therapy to a cell line employed in various experiments is contemplated, while when the mammalian animal is a non-human animal then a range from a non-human animal receiving a so called gene therapy to an animal model or a cell line employed in various experiments is contemplated. In the latter case, a preferred species is rat, mouse and the like.

Moreover, a case in which a mammalian cell is a cell in the body of a mammalian animal which can be diagnosed to suffer from a disease accompanied with a mental retardation or from Alzheimer's disease can be exemplified as a more typical case.

A method for preparing a DNA encoding a present transcription regulatory factor may be prepared in accordance with a method equivalent to that described above.

Using such a DNA thus prepared, a transformant is prepared as described below, whereby obtaining a transformant in which said DNA is provided in a position which enables its expression in the mammalian cell.

In a present invention described above, the phrase "provided in a position enabling the expression" means that a DNA molecule is placed in the position adjacent to a DNA sequence directing the transcription and the translation of its nucleotide sequence (i.g., promoting the production of a present transcription regulatory factor or its RNA molecule).

The expression level of the present transcription regulatory factor gene may be any level which is sufficient to promote the expression of a marker protein gene when compared with a cell into which no present transcription regulatory factor gene has been introduced. In such a case, the present transcription regulatory factor gene may be a DNA encoding the entire or a part of the present transcription regulatory factor.

In a present invention described above, it is also possible to promote a marker protein gene by preparing a transformant in which a present transcription regulatory factor gene is integrated into a genome.

In an expression promoting method described above,"a gene construct employed for introducing a present transcription regulatory factor gene into a mammalian cell (hereinafter sometimes referred to as a present gene construct) and a method for accomplishing a gene import may employ a virus vector having an affinity to the mammalian cell to which said DNA is to be introduced, such as a retrovirus vector, adenovirus vector, adeno-associated virus vector or others. For example, known vectors described in Miller, Human Gene Therapy 15 to 14, 1990; Friedman, Science 244: 1275 to 1281, 1989; Eglitis and Anderson, BioTechniques 6:608 to 614, 1988; Tolstoshev and Anderson, Current opinion in Biotechnology 1;55 to 61, 1990; Sharp, The Lancet 337:1277 to 1278, 1991; Cornetta et al, Nucleic Acid Research and Molecular Biology 36:311 to 322, 1987; Anderson, Science 22-:401 to 409, 1984; Moen, Blood Cells 17:407 to 416, 1991; Miller et al., Biotechniques 7:980 to 990, 1989; Le Gai La Salle et al., Science 259:988 to 990, 1993; and Johnson) Chest 107:77S to 83S, 1995 and the like may be exemplified. The retroviruses described for example in Rosenberg et al, N. Engl. J. Med 323:370, 1990; Anderson et al., United States Patent No. 5,399,346 have extensively developed, and have already been introduced into a clinical stage For example, when said cell is an animal cell, those which may be exemplified are an SV40 virus promoter, cytomegalovirus promoter (CMV promoter), Rous sarcoma virus promoter (RSV promoter, β actin gene promoter, aP2 gene promoter and the like. It is also possible to use a commercially available vector containing any of these promoters upstream of the multiple cloning site.

Said DNA may be placed under the control of a promoter which allows a present transcription regulatory factor gene to be expressed constitutively. Such a DNA may also be placed under the control of a promoter which regulates the expression of a present transcription regulatory factor gene via an environmental stimulation. For example, said DNA may be expressed using a tissue-specific or cell type-specific promoter or a promoter which is activated by a chemical signal or exogenous signal such as a drug or by the introduction of a drug.

It is also possible to employ an non-viral technique. Those which may be exemplified are a lipofection described in Felgner et al., Proc. Natl. Acad. Sci. USA 84:7413, 1987; Ono et al., Neurosci. Lett. 117:259, 1990; Brigham et al., Am. J. Med. Sci. 298:278, 1989; Staubinger et al., Meth. Enz. 101:512, 1983, asialoorosomucoid-polylysine conjugation (Wu et al., J. Biol. Chem. 263:14621, 1988, lipofection described in Wu et al., J. Biol. Chem. 264:16985, 1989 and the like, microinjection-described in Wolff et al., Science 247:1465, 19.90 and the like, calcium phosphate method, DEAE dextran method, electroporation, protoplast fusion method, liposome method and the like.

While in any of the technologies described above a present gene construct is applied (for example by an infusion) to the site where an underexpression of a maker protein gene described above is expected, it may be applied to a tissue near the site where an event such as an underexpression of a maker protein gene described above is expected or to a vessel supplying to the cell assumed to undergo an underexpression of a maker protein gene described above.

In a present gene construct, the expression of a present transcription regulatory factor gene (cDNA) can be directed by an appropriate promoter (for example, a promoter of human cytomegalovirus (CMV), simian virus 40 (SV40) or metallothionein and the like), and may also be regulated by an appropriate mammalian animal regulatory factor. For example, a present transcription regulatory factor gene can be expressed if necessary using an enhancer known to direct predominantly to the expression of the DNA in a neurocyte. Such an enhancer may be any enhancer whose expression is characterized to be specific to a tissue or cell. When a clone of a present transcription regulatory factor gene (genomic DNA) is employed as a gene construct [for example, a clone of a present transcription regulatory factor gene (genomic DNA) isolated by the hybridization with a present transcription regulatory factor gene (cDNA) described above], the regulation can be accomplished also via a cognate regulatory sequence, if necessary together with a regulatory sequence derived from an heterologous source containing any promoter or regulatory element described above.

When an expression promoting method described above is applied as a method for a gene therapy, it can be used by a direct administration of the present transcription regulatory factor gene into a cell. While the gene which may be employed may be any gene which has been produced or isolated by a standard method, a most convenient production can be accomplished by an in vivo transcription employing the present transcription regulatory factor gene under the control of a highly efficient promoter (for example, human cytomegalovirus promoter). The administration of the present transcription regulatory factor gene can be conducted by any of the direct nucleic acid administration methods described above.

An expression promoting method described above can be applied also as a gene therapy method in which a normal gene is implanted into a diseased cell of a patient. In ' this method, the normal present transcription regulatory factor gene is transfected into the cell which is exogenous or endogenous to the patient and which can be cultured. Then, the transfected cell is infused serologically into a target tissue.

Ideally, the production of a present transcription regulatory factor by all technologies for the gene therapy gives an intracellular level of the present transcription regulatory factor which is at least equal to a normal intracellular level of the present transcription regulatory factor in a non-diseased cell.

As an example, a present invention described above in the case where the mammalian animal is a transformed mouse is detailed below.

A method for introducing a present transcription regulatory factor gene in the production of a transformed mouse may for example be a microinjection method, a method employing a retrovirus, a method employing an embryonic stem cell (ES cell) and the like. Among those listed above, the microinjection method is employed most frequently. The microinjection method employs a micromanipulator to infuse a solution containing the relevant DNA into the pronucleus of a fertilized ovum under the observation by a microscope.

First, a present transcription regulatory factor gene is infused into a fertilized ovum. In this step, it is preferably to remove the vector region employed for isolating this DNA as much as possible, to remove an AU-rich region contributing to the instabilization of a mRNA and to make the DNA linear for the purpose of integrating the DNA into a chromosome at a high probability. It is also preferable to insert an intron previously into the DNA, and such an intron may for example be a β-globin intron and the like.

A fertilized ovum is obtained from a mouse of a line suitable for the purpose. An inbred C57BL/6 mouse or C3H mouse, a cross line of the C57BL/6 mouse with another line (such as (C57BL/6 x DBA/2) F1), a non-inbred line ICR mouse may be exemplified. The fertilized ovum is obtained by mating a female mouse whose superovulation is induced by intraperitoneal administration of both of a pregnant mare's serum gonadotropin and chorionic gonadotropin with a male mouse followed by isolating the ovum from this female mouse. The isolated fertilized ovum is placed in a culture drop, which is maintained in a $CO_2$ gas incubator, whereby enabling the storage until the infusion of the relevant DNA.

The infusion of the DNA is conducted under the observation with an inverted microscope fitted with a micromanipulator. A fertilized ovum employed is preferably one in a developmental stage of the time when the male pronucleus becomes larger than the female pronucleus through the time when the both pronuclei are fused with each other. First, the fertilized ovum is fixed, and a DNA solution containing the relevant DNA is infused into the male pronucleus of the fertilized ovum. This DNA solution can be prepared as a complex if necessary. A substance used for forming a complex may for example be a liposome, calcium phosphate, retrovirus and the like. The infusion of the DNA solution is evident from the swelling of the male pronucleus. The amount of the DNA infused may for example be an amount containing about 200 to about 3,000 copies of the relevant DNA.

A fertilized ovum into which a present transcription regulatory factor gene has been infused is then cultured as described above until it becomes a blastocyst, which is then implanted into the uterus of a surrogate mother. Preferably, the ovum is implanted into the oviduct of the surrogate mother immediately after the infusion of the DNA. The surrogate mother is preferably a female mouse in a pseudo-pregnant female mouse after mating with a male mouse whose seminal duct has been ligated. Typically, the relevant female mouse is excised at the back skin and muscle near the kidneys to take the ovaries, oviducts and uterus out, and the ovarian membrane is opened to search for the oviduct opening. Then a surviving fertilized ovum after infusing the relevant DNA is imported from the oviduct opening, and then the ovaries, oviducts and uterus are returned into the abdominal cavity, and then the muscle coats are sutured and the skin is clipped. After about 20 days, a neonate is born.

A part of the somatic tissue of the neonate thus obtained, such as a part of the tail, is cut out as a sample, from which DNAs are extracted and subjected for example to a southern blotting, whereby identifying the relevant DNA. As described above, it can be verified that the relevant DNA has been introduced into a non-human animal. Otherwise, a PCR may also be employed for identification.

While a present transcription regulatory factor gene as an active ingredient of a present transcription regulatory factor gene therapy agent may be prepared as described above, it can be employed in the form of a recombinant vector or recombinant virus containing the relevant DNA. Such a form may for example be a virus vector such as a retrovirus vector, adenovirus vector, adeno-associated virus vector, herpes simplex virus vector, SV40 vector, polyoma virus vector, papilloma virus vector, picornavirus vector and vaccinia virus vector and the like. When an adenovirus vector is employed, an AdEasy Kit produced by QUANTUM is employed to integrate a present transcription regulatory factor gene into a multiple cloning site of a Transfer Vector, and the resultant recombinant vector is made linear, and then transformed into a coliform microorganism together with a pAdEasy vector, and a homologous recombinant DNA is integrated into a human 293A cell, whereby producing a recombinant virus containing the present transcription regulatory factor gene, which is then recovered and used.

It is also possible to use a non-viral vector such as a plasmid DNA comprising a human cytomegalovirus promoter region. Similarly to a case where a present transcription regulatory factor gene is infused directly into a fibrotic tissue site, a use of a plasmid DNA is extremely beneficial in a system where the present transcription regulatory factor gene is delivered locally using a non-viral vector. By employing a method in which a cell once taken out of a body is introduced with an expression vector and then returned to the body, i.e., an ex vivo method, all of the known introduction methods can be utilized. For example, a non-viral vector can be introduced by means of a) direct infusion, b) liposome-mediated introduction, c) cell transfection by calcium phosphate method, electroporation and DEAE-dextran method, d) polybrene-mediated delivery, e) protoplast fusion, f) microinjection, g) introduction using polylysine and the like.

A present transcription regulatory factor gene therapy agent can be given at an effective dose parenterally to a mammalian animal such as a human. For example, a parenteral administration can be accomplished for example by an injection (subcutaneous, intravenosu) as described above. A suitable dosage form described above can be produced by incorporating a present transcription regulatory factor gene (including vector form, virus form, plasmid form of the present transcription regulatory factor gene) into a pharmaceutically acceptable carrier such as an aqueous solvent, non-aqueous solvent, buffering agent, solubilizing aid, osmotic agent, stabilizer and the like. If necessary, auxiliary agents such as a preservative, suspending agent, emulsifier and the like may also be added.

While the dose may vary depending on the age, sex, body weight of a mammalian animal to be treated, the type of a present fat accumulation inhibitor, and the dosage form, it is usually an amount of an active ingredient which gives an intracellular level of a present transcription regulatory factor which is equal to a level allowing the present transcription regulatory factor to act effectively in the cell of the patient. The daily dose described above may be given all at once or in portions.

## EXAMPLES

The present invention is further described in the following Examples, which are not intended to restrict the invention.

EXAMPLE 1 (preparation of pGEM-mNXF as vector containing present transcription regulatory factor gene)

Polynucleotides consisting of the nucleotide sequences represented by SEQ ID NO.7 (aagcacggag gaggaagccg ccggtgcgtc gggac) and 8 (ggagagcggc tccacgtctt gatgacaata tgcca) were synthesized using a DNA synthesizer (Applied Biosystems Model 394). As a template, 10 ng of a mouse Brain cDNA library (#10655-017, Gibco BRL) was employed together with the polynucleotides described above as primers whereby effecting a PCR. In this PCR, each 10 pmol of the polynucleotide described above was added to 50 μl of the reaction solution, and an LA-Taq polymerase (Takara) and a buffer attached to the kit containing this enzyme were employed. The PCR reaction solution was incubated using a PCR system 9700 (Applied Biosystems) and subjected to 35 cycles, each cycle consisting of an incubation for 1 minutes at 95°C followed by 3 minutes at 68°C.

Then, the entire volume of the PCR reaction solution was subjected to an agarose gel electrophoresis (agarose L, Nippon Gene). After identifying a single band of an about 2.5 kb DNA, this DNA was recovered. A part of the DNA recovered was used together with a dye terminator sequence kit FS (Applied Biosystems) to prepare a direct sequencing sample, which was subjected to a direct nucleotide sequencing using an autosequencer (Applied Biosystems, Model 3700).

Subsequently, the DNA (about 1 μg) which was recovered as described above was mixed with a pGEM T easy vector (Promega) (10 ng), and combined with a T4 DNA ligase to effect a reaction, whereby obtaining a pGEM-mNXF. The nucleotide sequence of the resultant pGEM-mNXF was determined using an ABI Model 3700 autosequencer by a dye terminator method. The determined nucleotide sequence was compared with the nucleotide sequence obtained by the direct sequencing described above, and it was confirmed that the nucleotide sequence in the translation region exhibited a complete agreement.

EXAMPLE 2 (preparation of pRC/RSV-mNXFsense and pRC/RSV-mNXFantisense)

Subsequently, a plasmid for expressing a full length present transcription regulatory factor in a mammalian cell (hereinafter sometimes referred to as a present expression plasmid) was prepared as described below.

First, the direction of the insertion fragment in relation with the multiple cloning site of the pGEM-mNXF obtained in EXAMPLE 1 was in such a construction that the Sp6 promoter of a commercial pGEM vector was positioned upstream of the initiation codon. Then this pGEM-mNXF (1 μg) was employed as a template together with the oligonucleotide primers represented by SEQ ID Nos.11 and 12 (primer pair: forward primer 5'-gggcgctgcagcccagccaccatgtaccgatccaccaaggg-3', reverse primer 5'-aatctcggcgttgatctggt-3') to effect a PCR using a KODplus polymerase (TOYOBO), whereby a partial DNA fragment. of the present transcription regulatory factor gene into which a Kozac sequence (S'CCAGCCACC-3') immediately before the initiation codon of the present transcription regulatory factor gene and a PstI restriction enzyme site upstream thereof had been introduced.

The PCR conditions employed 35 cycles, each cycle involving an incubation at 95°C for 1 minute followed by 55°C for 30 seconds followed by 72°C for 1 minute.

The amplified DNA fragment thus obtained was cleaved with PstI and BssHII, and subjected to a low melting point agarose gel electrophoresis (NusieveGTG agarose; FMCbio), whereby accomplishing the purification and recovery. The DNA fragment thus purified and recovered was used as an insert fragment as described below. Then, a vector GEM-mNXF which had been cleaved with PstI and BssHII and then BAP-treated was subjected to a low melting point agarose gel electrophoresis (Agarose L, Nippon Gene) to recover a DNA fragment. The recovered DNA fragment (0.1 μg) was ligated with the insert fragment (0.5 μg) described above using a T4 Ligase to obtain a pGEM-mNXF kozac into which a Kozac sequence (5'CCAGCCACC-3') had been introduced immediately before the initiation codon of the present protein gene derived from a mouse. The nucleotide sequence of the insert fragment was verified to be correct using a DNA sequencer (Model 3700; PE biosystems).

Then, this pGEM-mNXF kozac was cleaved simultaneously with 3 enzymes PstI, NotI and SeaI, and then subjected to a low melting point agarose electrophoresis to recover an about 2.5 kbp of an mNXF kozac PstI-NotI DNA fragment. The recovered DNA fragment was imparted with a blunt end using a T4 polymerase and then used as an insert fragment. After cleaving an RSV promoter-carrying pRC/RSV (Invitorgen) was cleaved with HindIII, imparted with a blunt end using a T4 polymerase, and subjected to a BAP treatment, whereby obtaining a vector. This vector (0.1 μg) was ligated with the insert fragment (0.5 μg) described above using a T4 Ligase to obtain (a) a pRC/RSV-mNXFsense which is a plasmid expressing the sense strand of the mNXF kozac under the control of the RSV promoter and (2) a pRC/RVS-mNXFantisense which is a plasmid expressing the antisense strand of the mNXF kozac under the control of the RSV promoter. Whether the prepared plasmid was the desired plasmid or not was checked by investigating the nucleotide sequence of the margin between the vector and the inserted fragment.

EXAMPLE 3 (Promotion of EphA1 by present transcription regulatory factor)

First, about 1 x $10^7$ cells of an SK-N-MC cell (ATCC No.HTB10; purchased from DAINIPPON SEIYAKU) were cultured in a 10% FBS-supplemented DMEM medium (NISSUI SEIYAKU) at 37°C in the presence of 5% $CO_2$ in a petri dish (Falcon) whose diameter was about 10 cm. On the next day, the cultured cells were dispersed by a trypsin treatment, washed twice with an FBS-free DMEM medium, and then dispersed again in an FBS-free DMEM medium at the cell density of 1 x $10^7$. 0.4 ml of this cell dispersion was combined with each 10 μg of the plasmids prepared in EXAMPLE 2 ((a) pRC/RSV-mNXFsense or (b)pRC/RSV-mNXFantisense), and the mixture was transferred into an electroporation cuvette, where a transfection was conducted by an electroporation method employing a Gene pulser (BIORAD) under the conditions involving 200V and 950 μF. After the transfection, the culture medium was replaced with a 10% FBS-supplemented DMEM, and then further cultured in a 10-cm petri dish for about 24 hours. After the culture, the cells from five dishes were employed as a starting material to be subjected to a purification of DNA-free total RNA using an Atlas Pure Total RNA Labeling system (Kl038-1; Clonetech) which is a commercial RNA purifying and radiolabelling kit. The RNA yield was 23 μg when using the plasmid (a) (i.e., pRC/RSV-mNXFsense) and 26 μg when using the plasmid (b) (i.e., pRC/RSV-mNXFantisense). Then, the commercial kit described above and the RNA obtained were employed as a starting material for radiolabeling each RNA with [$\alpha$-$P^{32}$] -dATP (Amersham Pharmacia) using specific primers and reverse transcriptases contained in the commercial kit. Then the radiolabeled RNA (hereinafter referred to as a probe) was purified using the commercial kit described above, and the purified RNA was adjusted at 1.3 x $10^5$ DPM, and then used in the hybridization reaction described below. The hybridization reaction was conducted using a commercial kit of a nylon membrane having various genes blotted thereon (Atlas cDNA Expression array-Neurobiology; 7736-1, Clontech) together with the hybridization buffers attached thereto. The hybridization employed a 18-hour reaction of (a) a nylon membrane corresponding to the probe derived from a pRC-RSV-mNXFsense-introduced cell and (b) a nylon membrane corresponding to the probe derived from a pRC-RSV-mNXFantisense-introduced cell each in an identical incubator under an identical condition. After the reaction, the nylon membrane was washed with 2 x SSC, 1% SDS buffer (68°C, 30 minutes). This procedure was repeated 4 times, and then a further washing was made with 0.1 x SSC, 0.5% SDS buffer (68°C, 30 minutes). Each nylon membrane was wrapped with a plastic film, exposed to an IP plate (FUJI. FILM) for 7 days, and subjected to an image analyzer (BASstation; FUJI FILM) to quantify and compare the intensity of a probe hybridization signal corresponding to each of various genes on the nylon membrane.

As a result, the hybridization signal to the EphA1 gene on (a) the nylon membrane corresponding to the probe derived from a pRC-RSV-mNXFsense-introduced cell was significantly more intense than that on (b) the nylon membrane corresponding to the probe derived from a pRC-RSV-mNXFantisense-introduced cell. Thus, the present transcription regulatory factor was verified to have an ability of promoting the expression of the EphAl.

EXAMPLE 4 (Inhibition of Rho GDP dissociation inhibitor by present transcription regulatory factor)

First, about 1 x $10^7$ cells of an SK-N-MC cell (ATCC No.HTB10; purchased from DAINIPPON SEIYAKU) were cultured in a 10% FBS-supplemented DMEM medium (NISSUI SEIYAKU) at 37°C in the presence of 5% $CO_2$ in a petri dish (Falcon) whose diameter was about 10 cm. On the next day, the cultured cells were dispersed by a trypsin treatment, washed twice with an FBS-free DMEM medium, and then dispersed again in an FBS-free DMEM medium at the cell density of 1 x $10^7$. 0.4 ml of this cell dispersion was combined with each 10 μg of the plasmids prepared in EXAMPLE 2 ((a) pRC/RSV-mNXFsense or (b)pRC/RSV-mNXFantisense), and the mixture was transferred into an electroporation cuvette, where a transfection was conducted by an electroporation method employing a Gene pulser (BIORAD) under the conditions involving 200V and 950 μF. After the transfection, the culture medium was replaced with a 10% FBS-supplemented DMEM, and then further cultured in a 10-cm petri dish for about 24 hours. After the culture, the cells from five dishes were employed as a starting material to be subjected to a purification of DNA-free total RNA using an Atlas Pure Total RNA Labeling system (K1038-1; Clonetech) which is a commercial RNA purifying and radiolabelling kit. The RNA yield was 23 μg when using the plasmid (a) (i.e., pRC/RSV-mNXFsense) and 26 μg when using the plasmid (b) (i.e., pRC/RSV-mNXFantisense). Then, the commercial kit described above and the RNA obtained were employed as a starting material for radiolabeling each RNA with [$\alpha P^{32}$]-dATP (Amersham Pharmacia) using specific primers and reverse transcriptases contained in the commercial kit. Then the radiolabeled RNA (hereinafter referred to as a probe) was purified using the commercial kit described above, and the purified RNA was adjusted at 1.3 x $10^5$ DPM, and then used in the hybridization reaction described below. The hybridization reaction was conducted using a commercial kit of a nylon membrane having various genes blotted thereon (Atlas cDNA Expression array-Neurobiology; 7736-1, Clontech) together with the hybridization buffers attached thereto, the hybridization employed a 18-hour reaction of (a) a nylon membrane corresponding to the probe derived from a pRC-RSV-mNXFsense-introduced cell and (b) a nylon membrane corresponding to the probe derived from a pRC-RSV-mNXFantisense-introduced cell each in an identical incubator under an identical condition. After the reaction, the nylon membrane was washed with 2 x SSC, 1% SDS buffer (68°C, 30 minutes). This procedure was repeated 4 times, and then a further washing was made with 0.1 x SSC, 0.5% SDS buffer (68°C, 30 minutes). Each nylon membrane was wrapped with a plastic film, exposed to an IP plate (FUJI FILM) for 7 days, and subjected to an image analyzer (BASstation; FUJI FILM) to quantify and compare the intensity of a probe hybridization signal corresponding to each of various genes on the nylon membrane.

As a result, the hybridization signal to the Rho GDP dissociation inhibitor gene on (a) the nylon membrane corresponding to the probe derived from a pRC-RSV-mNXFsense-introduced cell was significantly less intense than that on (b) the nylon membrane corresponding to the probe derived from a pRC-RSV-mNXFantisense-introduced cell. Thus, the present transcription regulatory factor was verified to have an ability of inhibiting the expression of the Rho GDP dissociation inhibitor.

EXAMPLE 5 (Assay method of the present invention)

The pRC/RSV-mNXFsense-introduced cells prepared in EXAMPLE 2 are cultured in a 10% FBS-supplemented DMEM medium (NISSUI SEIYAKU) at 37°C in the presence of 5% $CO_2$ in a petri dish (Falcon) whose diameter is about 10 cm. On the next day, the cultured cells are dispersed by a trypsin treatment, washed twice with an FBS-free DMEM medium, and then dispersed again in the DMEM medium.

Subsequently, the dispersed cells are inoculated at $10^6$ cells/well in a 6-well plate to which (a) a culture medium supplemented only with DMSO or (b) a culture medium supplemented with a test substance dissolved at a varying concentration in DMSO has previously been added. The cells in this plate are cultured at 37°C for about 24 hours, and then the culture medium is discarded from the plate, and the cells are washed with PBS (-), and then the total RNA is extracted from each well using Isogen (NIPPON GENE).

The total RNA thus extracted is employed as a starting material to produce a single-stranded cDNA using an oligodT (Amersham Pharmacia) and a Reverse transcriptase (SuperScript II, Gibco etc.). Then this single-stranded cDNA is employed as a template to perform a PCR using a LA-Taq (Takara) together with a combination of the forward primers (SEQ ID Nos.13 to 17) and the reverse primers (SEQ ID Nos.18 to 22) listed below.

<Examples of forward primers>

5'-agtgaacagcaacagatact-3' (SEQ ID Nos.13)
5'-gcttcccgcgtccacgtgga-3' (SEQ ID Nos.14)
5'-gatgtgggcattcagctccg-3' (SEQ ID Nos.15)
5'-cagagcttcaccattcgaga-3' (SEQ ID Nos.16)
5'-gtggccctggtgtctgtccg-3' (SEQ ID Nos.17)

<Examples of reverse primers>

5'-ctggcccgcgcgtggggcaa-3' (SEQ ID Nos.18)
5'-acacatgcttcgccactgcc-3' (SEQ ID Nos.19)
5'-taatggccgctctcacaggt-3' (SEQ ID Nos.20)
5'-agctgagtccctgaggcaga-3' (SEQ ID Nos.21)
5'-tgtgctgtgccctgacactg-3' (SEQ ID Nos.22)

The PCR employs 30 cycles, each cycle being performed at 95°C for 1 minute followed by 68°C for 1 minute. The amount of the mRNA of the EphA 1 present in the total RNA is thus calculated and the amount of the mRNA of the EphA 1 in the total RNA extracted from the cells cultured in the culture medium supplemented only with DMSO is compared with the amount of the mRNA of the EphA 1 in the RNA extracted from the cells cultured in the culture medium supplemented with any of various test substances, whereby assaying and evaluating the ability to control the neurocyte plasticity dependent on the present transcription regulatory factor possessed by the test substance. Based on the results, desired substances are selected.

<Examples of forward primers>

5'-ttgcagcggagaacgaggag-3' (SEQ ID Nos.23)

5'-gatgagcactcggtcaacta-3' (SEQ ID Nos.24)
5'-gcatccaggagatccaggag-3' (SEQ ID Nos.25)
5'-ctggacaaggacgacgagag-3' (SEQ ID Nos.26)
5'-cctgcgaaagtacaaggagg-3' (SEQ ID Nos.27)

<Examples of reverse primers>

5'-cctaccatgtagtcagtctt-3' (SEQ ID Nos.28)
S'-ggtcaggaactcgtactcct-3' (SEQ ID Nos.29)
5'-ttgggtgcctcctccacggg-3' (SEQ ID Nos.30)
5'-gcgggacttgatgctgtagc-3' (SEQ ID Nos.31)
5'-gtcttgtcgtcgtctgtgaa-3' (SEQ ID Nos.32)

The amount of the mRNA of the Rho GDP dissociation inhibitor present in the total RNA is thus calculated and the amount of the mRNA of the Rho GDP dissociation inhibitor in the total RNA extracted from the cells cultured in the culture medium supplemented only with DMSO is compared with the amount of the mRNA of the Rho GDP dissociation inhibitor in the total RNA extracted from the cells cultured in the culture medium supplemented with any of various test substances, whereby assaying and evaluating the ability to control the neurocyte plasticity dependent on the present transcription regulatory factor possessed by the test substance. Based on the results, desired substances are selected.

INDUSTRIAL APPLICABILITY

According to the invention, a method for assaying an ability to control a neurocyte plasticity which is essential for searching for a substance used for controlling the neurocyte plasticity in a mammalian cell can for example be provided.

FREE TEXT IN SEQUENCE LISTING

SEQ ID No.7
Designed oligonucleotide primer for PCR
SEQ ID No.8
Designed oligonucleotide primer for PCR
SEQ ID No.9
Designed oligonucleotide primer for PCR
SEQ ID No.10
Designed oligonucleotide primer for PCR
SEQ ID No.11
Designed oligonucleotide primer for PCR
SEQ ID No.12
Designed oligonucleotide primer for PCR
SEQ ID No.13
Designed oligonucleotide primer for PCR
SEQ ID No.14
Designed oligonucleotide primer for PCR
SEQ ID No.15
Designed oligonucleotide primer for PCR
SEQ ID No.16
Designed oligonucleotide primer for PCR
SEQ ID No.17
Designed oligonucleotide primer for PCR
SEQ ID No.18
Designed oligonucleotide primer for PCR
SEQ ID No.19
Designed oligonucleotide primer for PCR
SEQ ID No.20

Designed oligonucleotide primer for PCR
SEQ ID No.21
Designed oligonucleotide primer for PCR
SEQ ID No.22
Designed oligonucleotide primer for PCR
SEQ ID No.23
Designed oligonucleotide primer for PCR
SEQ ID No.24
Designed oligonucleotide primer for PCR
SEQ ID No.25
Designed oligonucleotide primer for PCR
SEQ ID No.26
Designed oligonucleotide primer for PCR
SEQ ID No.27
Designed oligonucleotide primer for PCR
SEQ ID No.28
Designed oligonucleotide primer for PCR
SEQ ID No.29
Designed oligonucleotide primer for PCR
SEQ ID No.30
Designed oligonucleotide primer for PCR
SEQ ID No.31
Designed oligonucleotide primer for PCR
SEQ ID No.32
Designed oligonucleotide primer for PCR

**Claims**

1. An in vitro method for assaying an ability to control neurocyte plasticity which is dependent on a transcription regulatory factor, comprising the steps:

(i) bringing a test substance into contact with a mammalian cell expressing the transcription regulatory factor;
(ii) measuring the expression level of a marker protein gene present on the transcription regulatory factor-dependent neurocyte plasticizing pathway in the mammalian cell or an index value correlating with the level; and
(iii) evaluating the ability possessed by the test substance based on the expression level or the index value correlating with the level measured in step (ii),

wherein the transcription regulatory factor comprises any of the following amino acid sequences:

(a) the amino acid sequence represented by any of SEQ ID Nos. 1 to 3;
(b) the amino acid sequence of a protein comprising an amino acid sequence exhibiting an amino acid identity of 90% or more to the amino acid sequence represented by any of SEQ ID Nos. 1 to 3 and also having a transcription regulation ability;
(c) the amino acid sequence of a protein comprising an amino acid sequence encoded by a DNA which hybridizes under a stringent condition with a DNA consisting of the nucleotide sequence represented by the nucleotide numbers 102 to 2507 in the nucleotide sequence represented by SEQ ID No. 4 and also having a transcription regulation ability;
(d) the amino acid sequence of a protein comprising an amino acid sequence encoded by a DNA which hybridizes under a stringent condition with a DNA consisting of the nucleotide sequence represented by the nucleotide numbers 51 to 2456 in the nucleotide sequence represented by SEQ ID No. 5 and also having a transcription regulation ability; and
(e) the amino acid sequence of a protein comprising an amino acid sequence encoded by a DNA which hybridizes under a stringent condition with a DNA consisting of the nucleotide sequence represented by the nucleotide numbers 35 to 2440 in the nucleotide sequence represented by SEQ ID No. 6 and also having a transcription regulation ability.

2. An in vitro method for assaying an ability to control neurocyte plasticity which is dependent on a transcription regulatory factor, comprising the steps :

(i) bringing a test substance into contact with a transformed mammalian cell obtainable by introducing into the cell a gene comprising a nucleotide sequence encoding the amino acid sequence of said transcription regulatory factor;
(ii) measuring the expression level of a marker protein gene present on the transcription regulatory factor-dependent neurocyte plasticizing pathway in the transformed mammalian cell or an index value correlating with the level; and
(iii) evaluating the ability possessed by the test substance based on the expression level or the index value correlating with the level measured in step (ii),

wherein the transcription regulatory factor comprises any of the following amino acid sequences:

(a) the amino acid sequence represented by any of SEQ ID Nos. 1 to 3;
(b) the amino acid sequence of a protein comprising an amino acid sequence ' exhibiting an amino acid identity of 90% or more to the amino acid sequence represented by any of SEQ ID Nos. 1 to 3 and also having a transcription regulation ability;
(c) the amino acid sequence of a protein comprising an amino acid sequence encoded by a DNA which hybridizes under a stringent condition with a DNA consisting of the nucleotide sequence represented by the nucleotide numbers 102 to 2507 in the nucleotide sequence represented by SEQ ID No. 4 and also having a transcription regulation ability;
(d) the amino acid sequence of a protein comprising an amino acid sequence encoded by a DNA which hybridizes under a stringent condition with a DNA consisting of the nucleotide sequence represented by the nucleotide numbers 51 to 2456 in the nucleotide sequence represented by SEQ ID No. 5 and also having a transcription regulation ability; and
(e) the amino acid sequence of a protein comprising an amino acid sequence encoded by a DNA which hybridizes under a stringent condition with a DNA consisting of the nucleotide sequence represented by the nucleotide numbers 35 to 2440 in the nucleotide sequence represented by SEQ ID No. 6 and also having a transcription regulation ability.

**3.** The assay method according to claim 1 or 2, wherein the marker protein gene is an Eph A receptor gene or an Rho GDP dissociation inhibitor gene.

**4.** The assay method according to claim 1 or 2, wherein the marker protein genes are an Eph A receptor gene and an Rho GDP dissociation inhibitor gene.

**5.** The assay method according to claim 1 or 2, wherein the ability possessed by the test substance is evaluated based on the difference obtained by comparing the marker protein gene expression level or an index value correlating with the level in the groups employing two or more different substances independently as test substances.

**6.** The assay method according to claim 5, wherein at least one of the two or more different substances is a substance which does not have a neurocyte plasticity-controlling ability, thereby evaluating the neurocyte plasticity-controlling ability possessed by any of the other test substances.

**7.** A method for searching for a substance having an ability to control the neurocyte plasticity which is dependent on a transcription regulatory factor, wherein a substance having said ability is selected on the basis of the ability evaluated by an assay method according to claim 1 or 2, wherein the transcription regulatory factor comprises any of the following amino acid sequences:

(a) the amino acid sequence represented by any of SEQ ID Nos. 1 to 3;
(b) the amino acid sequence of a protein comprising an amino acid sequence exhibiting an amino acid identity of 90% or more to the amino acid sequence represented by any of SEQ ID Nos. 1 to 3 and also having a transcription regulation ability;
(c) the amino acid sequence of a protein comprising an amino acid sequence encoded by a DNA which hybridizes under a stringent condition with a DNA consisting of the nucleotide sequence represented by the nucleotide numbers 102 to 2507 in the nucleotide sequence represented by SEQ ID No. 4 and also having a transcription regulation ability;
(d) the amino acid sequence of a protein comprising an amino acid sequence encoded by a DNA which hybridizes under a stringent condition with a DNA consisting of the nucleotide sequence represented by the nucleotide numbers 51 to 2456 in the nucleotide sequence represented by SEQ ID No. 5 and also having a transcription regulation ability; and
(e) the amino acid sequence of protein comprising an amino acid sequence encoded by a DNA which hybridizes under a stringent condition with a DNA consisting of the nucleotide sequence represented by the nucleotide numbers 35 to 2440 in the nucleotide sequence represented by SEQ ID No. 6 and also having a transcription regulation ability.

**8.** Use of a gene for the preparation of a pharmaceutical composition for controlling neurocyte plasticity in a mammalian cell in gene therapy, wherein the gene comprises the nucleotide sequence encoding any of the following amino acid sequences:

(a) the amino acid sequence represented by any of SEQ ID Nos. 1 to 3;
(b) the amino acid sequence of a protein comprising an amino acid sequence exhibiting an amino acid identity of 90% or more to the amino acid sequence represented by any of SEQ ID Nos. 1 to 3 and also having a transcription regulation ability;
(c) the amino acid sequence of a protein comprising an amino acid sequence encoded by a DNA which hybridizes under a stringent condition with a DNA consisting of the nucleotide sequence represented by the nucleotide numbers 102 to 2507 in the nucleotide sequence represented by SEQ ID No. 4 and also having a transcription regulation ability;
(d) the amino acid sequence of a protein comprising an amino acid sequence encoded by a DNA which hybridizes under a stringent condition with a DNA consisting of the nucleotide sequence represented by the nucleotide numbers 51 to 2456 in the nucleotide sequence represented by SEQ ID No. 5 and also having a transcription regulation ability; or
(e) the amino acid sequence of a protein comprising an amino acid sequence encoded by a DNA which hybridizes under a stringent condition with a DNA consisting of the nucleotide sequence represented by the nucleotide numbers 35 to 2440 in the nucleotide sequence represented by SEQ ID No. 6 and also having a transcription regulation ability.

**9.** Use of a gene as an exogenous gene for the preparation of a pharmaceutical composition for promoting the expression of a marker protein gene in a cell in gene therapy,
wherein the marker protein gene is present on the transcription regulatory factor-dependent neurocyte plasticizing pathway, and
wherein the gene is an exogenous gene comprising the nucleotide sequence encoding any of the following amino acid sequences:

(a) the amino acid sequence represented by any of SEQ ID Nos. 1 to 3;
(b) the amino acid sequence of a protein comprising an amino acid sequence exhibiting an amino acid identity of 90% or more to the amino acid sequence represented by any of SEQ ID Nos. 1 to 3 and also having a transcription regulation ability;
(c) the amino acid sequence of a protein comprising an amino acid sequence encoded by a DNA which hybridizes under a stringent condition with a DNA consisting of the nucleotide sequence represented by the nucleotide numbers 102 to 2507 in the nucleotide sequence represented by SEQ ID No. 4 and also having a transcription regulation ability;
(d) the amino acid sequence of a protein comprising an amino acid sequence encoded by a DNA which hybridizes under a stringent condition with a DNA consisting of the nucleotide sequence represented by the nucleotide numbers 51 to 2456 in the nucleotide sequence represented by SEQ ID No. 5 and also having a transcription regulation ability; or
(e) the amino acid sequence of a protein comprising an amino acid sequence encoded by a DNA which hybridizes under a stringent condition with a DNA consisting of the nucleotide sequence represented by the nucleotide numbers 35 to 2440 in the nucleotide sequence represented by SEQ ID No. 6 and also having a transcription regulation ability.

**Patentansprüche**

**1.** In vitro-Verfahren zum Testen einer Fähigkeit, Neurocytenplastizität zu steuern, die von einem Transkriptionsregulationsfaktor abhängig ist, umfassend die Schritte:

(i) Inkontaktbringen einer Testsubstanz mit einer Säugerzelle, die den Transkriptionsregulationsfaktor exprimiert;
(ii) Messen des Expressionsspiegels eines Markerproteingens, das in dem Transkriptionsregulationsfaktor-abhängigen Neurocyten-Plastifizierungsweg in der Säugerzelle vorhanden ist, oder eines Indexwertes, der mit dem Spiegel korreliert; und
(iii) Bewerten der von der Testsubstanz besessenen Fähigkeit basierend auf dem Expressionsspiegel oder dem Indexwert, der mit dem in Schritt (ii) gemessenen Spiegel korreliert,

wobei der Transkriptionsregulationsfaktor eine der folgenden Aminosäuresequenzen umfasst:

(a) die Aminosäuresequenz dargestellt in einer der SEQ ID Nos. 1 bis 3;
(b) die Aminosäuresequenz eines Proteins, umfassend eine Aminosäuresequenz, welche eine Aminosäureidentität von 90% oder mehr zu der Aminosäuresequenz dargestellt in einer der SEQ ID Nos. 1 bis 3 zeigt, und die auch eine Fähigkeit zur Transkriptionsregulierung hat;
(c) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 102 bis 2507 in der Nucleotidsequenz dargestellt in SEQ ID No. 4 besteht, und die auch eine Fähigkeit zur Transkriptionsregulierung hat;
(d) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 51 bis 2456 in der Nucleotidsequenz dargestellt in SEQ ID No. 5 besteht, und die auch eine Fähigkeit zur Transkriptionsregulierung hat; und
(e) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 35 bis 2440 in der Nucleotidsequenz dargestellt in SEQ ID No. 6 besteht, und die auch eine Fähigkeit zur Transkriptionsregulierung hat.

**2.** In vitro-Verfahren zum Testen einer Fähigkeit, Neurocytenplastizität zu steuern, die abhängig ist von einem Transkriptionsregulationsfaktor, umfassend die Schritte:

(i) Inkontaktbringen einer Testsubstanz mit einer transformierten Säugerzelle, die erhältlich ist durch das Einführen eines Gens in die Zelle, das eine Nucleotidsequenz umfasst, die die Aminosäuresequenz des Transkriptionsregulationsfaktors codiert;
(ii) Messen des Expressionsspiegels eines Markerproteingens, das in dem Transkriptionsregulationsfaktor-abhängigen Neurocyten-Plastifizierungsweg in der transformierten Säugerzelle vorhanden ist, oder eines Indexwertes, der mit dem Spiegel korreliert; und
(iii) Bewerten der von der Testsubstanz besessenen Fähigkeit basierend auf dem Expressionsspiegel oder dem Indexwert, der mit dem in Schritt (ii) gemessenen Spiegel korreliert,

wobei der Transkriptionsregulationsfaktor eine der folgenden Aminosäuresequenzen umfasst:

(a) die Aminosäuresequenz dargestellt in einer der SEQ ID Nos. 1 bis 3;
(b) die Aminosäuresequenz eines Proteins, umfassend eine Aminosäuresequenz, die eine Aminosäureidentität von 90% oder mehr zu der Aminosäuresequenz dargestellt in einer der SEQ ID Nos. 1 bis 3 zeigt, und die auch eine Fähigkeit zur Transkriptionsregulierung hat;
(c) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 102 bis 2507 in der Nucleotidsequenz dargestellt in SEQ ID No. 4 besteht, und die auch eine Fähigkeit zur Transkriptionsregulierung hat;
(d) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 51 bis 2456 in der Nucleotidsequenz dargestellt in SEQ ID No. 5 besteht, und die auch eine Fähigkeit zur Transkriptionsregulierung hat; und
(e) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 35 bis 2440 in der Nucleotidsequenz dargestellt in SEQ ID No. 6 besteht, und die auch eine Fähigkeit zur Transkriptionsregulierung hat.

**3.** Testverfahren gemäß Anspruch 1 oder 2, wobei das Markerproteingen ein Eph A-Rezeptorgen oder ein Rho GDP-Dissoziationsinhibitorgen ist.

**4.** Testverfahren gemäß Anspruch 1 oder 2, wobei die Markerproteingene ein Eph A-Rezeptorgen und ein Rho GDP-Dissoziationsinhibitorgen sind.

**5.** Testverfahren gemäß Anspruch 1 oder 2, wobei die von der Testsubstanz besessene Fähigkeit basierend auf dem Unterschied bewertet wird, der erhalten wird durch das Vergleichen des Expressionsspiegels des Markerproteingens oder eines Indexwertes, der mit dem Spiegel korreliert, in den Gruppen, in denen zwei oder mehr unterschiedliche Stoffe unabhängig als Testsubstanzen verwendet werden.

**6.** Testverfahren gemäß Anspruch 5, wobei mindestens einer der zwei oder mehr unterschiedlichen Stoffe ein Stoff ist, der keine Fähigkeit zur Steuerung einer Neurocytenplastizität hat, wodurch die von einem der anderen Testsubstanzen besessene Fähigkeit zur Steuerung der Neurocytenplastizität bewertet wird.

**7.** Verfahren zur Suche nach einem Stoff, der eine Fähigkeit hat, die Neurocytenplastizität zu steuern, die von einem Transkriptionsregulationsfaktor abhängig ist, wobei ein Stoff, der die Fähigkeit hat, auf der Grundlage der Fähigkeit ausgewählt wird, die durch ein Testverfahren gemäß Anspruch 1 oder 2 bewertet wird, wobei der Transkriptionsregulationsfaktor eine der folgenden Aminosäuresequenzen umfasst:

(a) die Aminosäuresequenz dargestellt in einer der SEQ ID Nos. 1 bis 3;
(b) die Aminosäuresequenz eines Proteins, umfassend eine Aminosäuresequenz, die eine Aminosäureidentität von 90% oder mehr zu der Aminosäuresequenz dargestellt in einer der SEQ ID Nos. 1 bis 3 zeigt, und die auch eine Fähigkeit zur Transkriptionsregulierung hat;
(c) die Aminosäuresequenz eines Proteins, umfassend eine Aminosäuresequenz, die durch eine DNA codiert ist, welche unter einer stringenten Bedingung mit einer DNA hybridisiert, die aus der Nucleotidsequenz dargestellt in den Nucleotidnummern 102 bis 2507 in der Nucleotidsequenz dargestellt in SEQ ID No. 4 besteht, und die auch eine Fähigkeit zur Transkriptionsregulierung hat;
(d) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 51 bis 2456 in der Nucleotidsequenz dargestellt in SEQ ID No. 5 besteht, und die auch eine Fähigkeit zur Transkriptionsregulierung hat; und
(e) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 35 bis 2440 in der Nucleotidsequenz dargestellt in SEQ ID No. 6 besteht, und die auch eine Fähigkeit zur Transkriptionsregulierung hat.

**8.** Verwendung eines Gens für die Herstellung eines Arzneimittels zur Steuerung der Neurocytenplastizität in einer Säugerzelle in einer Gentherapie, wobei das Gen die Nucleotidsequenz umfasst, die eine der folgenden Aminosäuresequenzen codiert:

(a) die Aminosäuresequenz dargestellt in einer der SEQ ID Nos. 1 bis 3;
(b) die Aminosäuresequenz eines Proteins, umfassend eine Aminosäuresequenz, die eine Aminosäureidentität von 90% oder mehr zu der Aminosäuresequenz dargestellt in einer der SEQ ID Nos. 1 bis 3 zeigt, und die auch eine Fähigkeit zur Transkriptionsregulierung hat;
(c) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 102 bis 2507 in der Nucleotidsequenz dargestellt in SEQ m No. 4 besteht, und die auch eine Fähigkeit zur Transkriptionsregulierung hat;
(d) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 51 bis 2456 in der Nucleotidsequenz dargestellt in SEQ ID No. 5 besteht, und die auch eine Fähigkeit zur Transkriptionsregulierung hat; oder
(e) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 35 bis 2440 in der Nucleotidsequenz dargestellt in SEQ ID No. 6 besteht, und die auch eine Fähigkeit zur Transkriptionsregulierung hat.

**9.** Verwendung eines Gens als ein exogenes Gen für die Herstellung eines Arzneimittels zur Förderung der Expression eines Markerproteingens in einer Zelle in einer Gentherapie,
wobei das Markerproteingen in dem Transkriptionsregulationsfaktor-abhängigen Neurocyten-Plastifizierungsweg vorhanden ist, und
wobei das Gen ein exogenes Gen ist, das die Nucleotidsequenz umfasst, die eine der folgenden Aminosäuresequenzen codiert:

(a) die Aminosäuresequenz dargestellt in einer der SEQ ID Nos. 1 bis 3;
(b) die Aminosäuresequenz eines Proteins, umfassend eine Aminosäuresequenz, die eine Aminosäureidentität von 90% oder mehr zu der Aminosäuresequenz dargestellt in einer der SEQ ID Nos. 1 bis 3 zeigt, und die auch eine Fähigkeit zur Transkriptionsreglulierung hat;
(c) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in Nucleotidnummern 102 bis 2507 in der Nucleotidsequenz dargestellt in SEQ ID No. 4 besteht, und die auch eine Fähigkeit zur Transkriptionsregulierung hat;
(d) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in Nucleotidnummern 51 bis 2456 in der Nucleotidsequenz dargestellt in SEQ ID No. 5 besteht, und die auch eine Fähigkeit zur Transkriptionsregulierung hat; oder
(e) die Aminosäuresequenz eines Proteins, umfassend eine Aminosäuresequenz, die durch eine DNA codiert wird, welche unter einer stringenten Bedingung mit einer DNA hybridisiert, die aus der Nucleotidsequenz dargestellt in den Nucleotidnummern 35 bis 2440 in der Nucleotidsequenz dargestellt in SEQ ID No. 6 besteht, und die auch eine Fähigkeit zur Transkriptionsregulierung hat.

## Revendications

1. Procédé *in vitro* pour déterminer une capacité à contrôler la plasticité des cellules nerveuses qui dépend d'un facteur de régulation de la transcription, comprenant les étapes consistant à :

   (i) mettre en contact une substance de test avec une cellule de mammifère exprimant le facteur de régulation de la transcription ;
   (ii) mesurer le niveau d'expression d'un gène de protéine marqueur présent sur la voie de plasticité de cellule nerveuse dépendante du facteur de régulation de la transcription dans la cellule de mammifère ou une valeur index corrélée à ce niveau ; et
   (iii) évaluer la capacité de la substance de test en se fondant sur le niveau d'expression ou sur la valeur index corrélée au niveau mesuré au cours de l'étape (ii),

   dans lequel le facteur de régulation de la transcription contient l'une quelconque des séquences d'acides aminés suivantes :

   (a) la séquence d'acides aminés représentée par l'une quelconque de SEQ ID N° 1 à 3 ;
   (b) la séquence d'acides aminés d'une protéine comprenant une séquence d'acides aminés présentant une identité d'acides aminés de 90 % ou plus avec la séquence d'acides aminés représentée par l'une quelconque de SEQ ID N° 1 à 3 et ayant aussi une capacité de régulation de la transcription ;
   (c) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 102 à 2 507 dans la séquence de nucléotides représentée par SEQ m N° 4 et ayant aussi une capacité de régulation de la transcription ;
   (d) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 51 à 2 456 dans la séquence de nucléotides représentée par SEQ ID N° 5 et ayant aussi une capacité de régulation de la transcription ; et
   (e) la séquence d'acides aminés d'une protéine comprenant une séquence d'acides aminés codée par un ADN qui s'hybride dans une condition stringente à un ADN consistant en la séquence de nucléotides représentée par les numéros de nucléotides 35 à 2 440 dans la séquence de nucléotides représentée par SEQ ID N° 6 et ayant aussi une capacité de régulation de la transcription.

2. Procédé *in vitro* pour déterminer une capacité à contrôler la plasticité des cellules nerveuses qui dépend d'un facteur de régulation de la transcription, comprenant les étapes consistant à :

   (i) mettre en contact une substance de test avec une cellule de mammifère transformée pouvant être obtenue en introduisant dans la cellule un gène comprenant une séquence de nucléotides codant la séquence d'acides aminés dudit facteur de régulation de la transcription ;
   (ii) mesurer le niveau d'expression d'un gène de protéine marqueur présent sur la voie de plasticité de cellule nerveuse dépendante du facteur de régulation de la transcription dans la cellule de mammifère transformée ou une valeur index corrélée à ce niveau ; et
   (iii) évaluer la capacité de la substance de test en se fondant sur le niveau d'expression ou la valeur index corrélée au niveau mesuré à l'étape (ii),

   dans lequel le facteur de régulation de la transcription comprend l'une quelconque des séquences d'acides aminés suivantes :

   (a) la séquence d'acides aminés représentée par l'une quelconque de SEQ ID N° 1 à 3;
   (b) la séquence d'acides aminés d'une protéine comprenant une séquence d'acides aminés présentant une identité d'acides aminés de 90 % ou plus avec la séquence d'acides aminés représentée par l'une quelconque de SEQ ID N° 1 à 3 et ayant aussi une capacité de régulation de la transcription ;
   (c) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 102 à 2 507 dans la séquence de nucléotides représentée par SEQ ID N° 4 et ayant aussi une capacité de régulation de la transcription ;
   (d) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 51 à 2 456 dans la séquence de nucléotides représentée par SEQ ID N° 5 et ayant aussi une capacité de régulation de la transcription ; et
(e) la séquence d'acides aminés d'une protéine comprenant une séquence d'acides aminés codée par un ADN qui s'hybride dans une condition stringente à un ADN consistant en la séquence de nucléotides représentée par les numéros de nucléotides 35 à 2 440 dans la séquence de nucléotides représentée par SEQ ID N° 6 et ayant aussi une capacité de régulation de la transcription.

**3.** Procédé de test selon la revendication 1 ou 2, dans lequel le gène de la protéine marqueur est un gène du récepteur Eph A ou un gène de l'inhibiteur de la dissociation Rho GDP.

**4.** Procédé de test selon la revendication 1 ou 2, dans lequel les gènes de la protéine marqueur sont un gène du récepteur Eph A et un gène de l'inhibiteur de la dissociation Rho GDP.

**5.** Procédé de test selon la revendication 1 ou 2, dans lequel la capacité de la substance de test est évaluée en se fondant sur la différence obtenue en comparant le niveau d'expression du gène de protéine marqueur ou une valeur index corrélée à ce niveau dans les groupes utilisant une ou plusieurs substances différentes indépendamment comme substances de test.

**6.** Procédé de test selon la revendication 5, dans lequel au moins l'une des deux substances différentes ou plus est une substance qui n'a pas de capacité de contrôle de la plasticité des cellules nerveuses, évaluant ainsi la capacité de contrôle de la plasticité des cellules nerveuses possédée par l'une quelconque des autres substances de test.

**7.** Procédé pour rechercher une substance ayant une capacité à contrôler la plasticité des cellules nerveuses qui est dépendante d'un facteur de régulation de la transcription, une substance ayant ladite capacité étant choisie en se fondant sur la capacité évaluée par un procédé de test selon la revendication 1 ou 2, le facteur de régulation de la transcription comprenant l'une quelconque des séquences d'acides aminés suivantes :

(a) la séquence d'acides aminés représentée par l'une quelconque de SEQ ID N° 1 à 3 ;
(b) la séquence d'acides aminés d'une protéine comprenant une séquence d'acides aminés présentant une identité d'acides aminés de 90 % ou plus avec la séquence d'acides aminés représentée par l'une quelconque de SEQ ID N° 1 à 3 et ayant aussi une capacité de régulation de la transcription ;
(c) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 102 à 2 507 dans la séquence de nucléotides représentée par SEQ ID N° 4 et ayant aussi une capacité de régulation de la transcription ;
(d) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 51 à 2 456 dans la séquence de nucléotides représentée par SEQ ID N° 5 et ayant aussi une capacité de régulation de la transcription ; et
(e) la séquence d'acides aminés d'une protéine comprenant une séquence d'acides aminés codée par un ADN qui s'hybride dans une condition stringente à un ADN consistant en la séquence de nucléotides représentée par les numéros de nucléotides 35 à 2 440 dans la séquence de nucléotides représentée par SEQ ID N° 6 et ayant aussi une capacité de régulation de la transcription.

**8.** Utilisation d'un gène pour la préparation d'une composition pharmaceutique pour contrôler la plasticité des cellules nerveuses dans une cellule de mammifère dans la thérapie génique, le gène comprenant la séquence de nucléotides codant l'une quelconque des séquences d'acides aminés suivantes :

(a) la séquence d'acides aminés représentée par l'une quelconque de SEQ ID N° 1 à 3;
(b) la séquence d'acides aminés d'une protéine comprenant une séquence d'acides aminés présentant une identité d'acides aminés de 90 % ou plus avec la séquence d'acides aminés représentée par l'une quelconque de SEQ ID N° 1 à 3 et ayant aussi une capacité de régulation de la transcription ;
(c) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 102 à 2 507 dans la séquence de nucléotides représentée par SEQ ID N° 4 et ayant aussi une capacité de régulation de la transcription ;
(d) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 51 à 2 456 dans la séquence de nucléotides représentée par SEQ ID N° 5 et ayant aussi une capacité de régulation de la transcription ; ou
(e) la séquence d'acides aminés d'une protéine comprenant une séquence d'acides aminés codée par un ADN qui s'hybride dans une condition stringente à un ADN consistant en la séquence de nucléotides représentée par les numéros de nucléotides 35 à 2 440 dans la séquence de nucléotides représentée par SEQ ID N° 6 et ayant aussi une capacité de régulation de la transcription.

**9.** Utilisation d'un gène comme gène exogène pour la préparation d'une composition pharmaceutique pour favoriser l'expression d'un gène de protéine marqueur dans une cellule dans la thérapie génique, dans laquelle le gène de protéine marqueur est présent sur la voie de plasticité des cellules nerveuses dépendante du facteur de régulation de la transcription, et dans laquelle le gène est un gène exogène comprenant la séquence de nucléotides codant l'une quelconque des séquences d'acides aminés suivantes :

(a) la séquence d'acides aminés représentée par l'une quelconque de SEQ ID N° 1 à 3 ;
(b) la séquence d'acides aminés d'une protéine comprenant une séquence d'acides aminés présentant une identité d'acides aminés de 90 % ou plus avec la séquence d'acides aminés représentée par l'une quelconque de SEQ ID N° 1 à 3 et ayant aussi une capacité de régulation de la transcription ;
(c) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 102 à 2 507 dans la séquence de nucléotides représentée par SEQ ID N° 4 et ayant aussi une capacité de régulation de la transcription ;
(d) la séquence d'acides aminés d'une protéine comprenant une séquence d'acides aminés codée par un ADN qui s'hybride dans une condition stringente avec un ADN consistant en la séquence de nucléotides représentée par les numéros de nucléotides 51 à 2 456 dans la séquence de nucléotides représentée par SEQ ID N° 5 et ayant aussi une capacité de régulation de la transcription ; ou
(e) la séquence d'acides aminés d'une protéine comprenant une séquence d'acides aminés codée par un ADN qui s'hybride dans une condition stringente à un ADN consistant en la séquence de nucléotides représentée par les numéros de nucléotides 35 à 2 440 dans la séquence de nucléotides représentée par SEQ ID N° 6 et ayant aussi une capacité de régulation de la transcription.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 299733 A
- US 5399346 A, Anderson
- JP 2000398548 A
- JP 2001077740 A

### Non-patent literature cited in the description


- *Nucleic Acid Res.,* 1994, vol. 22 (22), 4673-4680
- **J. SAMBROOK ; E.F.FRISCH ; T.MANIATIS.** Molecular Cloning. Cold Spring Harbor Laboratory, 1989
- **MARUZEN.** *Labomanual genetic engineering,* 1988
- **HUNKAPILLER, M. et al.** *Nature,* 1984, vol. 310, 105
- **A. M. & W. GLIBERT.** *Proc. Natl. Acad. Sci. USA,* 1997, vol. 74, 560
- **SANGER, F. ; A. R. COULSON.** *J. Mol. Biol.,* 1975, vol. 94, 441
- **SANGER, F. ; NICKLEN ; A.R. COULSON.** *Proc. Natl. Acad. Sci. USA,* 1997, vol. 74, 5463
- **SMITH, G. E. ; SUMMERS M.E. et al.** *Mol. Cell Biol.,* 1983, vol. 3, 2156-2165
- **FUNAHASHI, S. et al.** *J. Virol.,* 1991, vol. 65, 5584-5588
- **ELI GILBOA et al.** *BioTechniques,* 1986, vol. 4, 504-512
- *J. Virol.,* vol. 66, 3755
- **AMMERER et al.** *Method in Enzymology,* vol. 101, 192-201
- **FREDERICK M.AUSUBEL et al.** Short Protocols in Molecular Biology. John Wiley & Sons, Inc
- **GRIMM, S. et al.** *Proc. Natl. Acad. Sci. USA,* vol. 93, 10923-10927
- **TING, A.T. et al.** *EMBO J.,* vol. 15, 6189-6196
- **HAWKINS, C.J. et al.** *Proc. Natl. Acad. Sci.USA,* vol. 93, 13786-13790
- **J. SAMBROOK ; E.F.FRISCH ; T.MANIATIS.** Molecular Cloning. Cold Spring Harbor Laboratory Press
- **MILLER.** *Human Gene Therapy,* 1990, 14-15
- **FRIEDMAN.** *Science,* 1989, vol. 244, 1275-1281
- **EGLITIS ; ANDERSON.** *BioTechniques,* 1988, vol. 6, 608-614
- **TOLSTOSHEV ; ANDERSON.** *Current opinion in Biotechnology,* 1990, vol. 1, 55-61
- **SHARP.** *The Lancet,* 1991, vol. 337, 1277-1278
- **CORNETTA et al.** *Nucleic Acid Research and Molecular Biology,* 1987, vol. 36, 311-322
- **ANDERSON.** *Science,* 1984, vol. 22, 401-409
- **MOEN.** *Blood Cells,* 1991, vol. 17, 407-416
- **MILLER et al.** *Biotechniques,* 1989, vol. 7, 980-990
- **LE GAI LA SALLE et al.** *Science,* 1993, vol. 259, 988-990
- **JOHNSON.** *Chest,* 1995, vol. 107, 77S-83S
- **ROSENBERG et al.** *N. Engl. J. Med,* 1990, vol. 323, 370
- **FELGNER et al.** *Proc. Natl. Acad. Sci. USA,* 1987, vol. 84, 7413
- **ONO et al.** *Neurosci. Lett.,* 1990, vol. 117, 259
- **BRIGHAM et al.** *Am. J. Med. Sci.,* 1989, vol. 298, 278
- **STAUBINGER et al.** *Meth. Enz.,* 1983, vol. 101, 512
- **WU et al.** *J. Biol. Chem.,* 1988, vol. 263, 14621
- **WU et al.** *J. Biol. Chem.,* 1989, vol. 264, 16985
- **WOLFF et al.** *Science,* 1990, vol. 247, 1465

Sequence Listing

<110> Sumitomo Chemical Company Limited

<120> A method for examining the ability of regulating plasticity of ner ve cells

<130> 560322

<150> JP 2000/398548
<151> 2000-12-27

<150> JP 2001/077740
<151> 2001-03-19

<160> 34

<210> 1
<211> 802
<212> PRT
<213> Homo sapiens

<400> 1

Met Tyr Arg Ser Thr Lys Gly Ala Ser Lys Ala Arg Arg Asp Gln Ile
1 5 10 15
Asn Ala Glu Ile Arg Asn Leu Lys Glu Leu Leu Pro Leu Ala Glu Ala
20 25 30

Asp Lys Val Arg Leu Ser Tyr Leu His Ile Met Ser Leu Ala Cys Ile
35 40 45
Tyr Thr Arg Lys Gly Val Phe Phe Ala Gly Gly Thr Pro Leu Ala Gly
50 55 60
Pro Thr Gly Leu Leu Ser Ala Gln Glu Leu Glu Asp Ile Val Ala Ala
65 70 75 80
Leu Pro Gly Phe Leu Leu Val Phe Thr Ala Glu Gly Lys Leu Leu Tyr
85 90 95
Leu Ser Glu Ser Val Ser Glu His Leu Gly His Ser Met Val Asp Leu
100 105 110
Val Ala Gln Gly Asp Ser Ile Tyr Asp Ile Ile Asp Pro Ala Asp His
115 120 125
Leu Thr Val Arg Gln Gln Leu Thr Leu Pro Ser Ala Leu Asp Thr Asp
130 135 140
Arg Leu Phe Arg Cys Arg Phe Asn Thr Ser Lys Ser Leu Arg Arg Gln
145 150 155 160
Ser Ala Gly Asn Lys Leu Val Leu Ile Arg Gly Arg Phe His Ala His
165 170 175
Pro Pro Gly Ala Tyr Trp Ala Gly Asn Pro Val Phe Thr Ala Phe Cys
180 185 190
Ala Pro Leu Glu Pro Arg Pro Arg Pro Gly Pro Gly Pro Gly Pro Gly
195 200 205
Pro Ala Ser Leu Phe Leu Ala Met Phe Gln Ser Arg His Ala Lys Asp
210 215 220
Leu Ala Leu Leu Asp Ile Ser Glu Ser Val Leu Ile Tyr Leu Gly Phe
225 230 235 240
Glu Arg Ser Glu Leu Leu Cys Lys Ser Trp Tyr Gly Leu Leu His Pro

245 250 255

Glu Asp Leu Ala His Ala Ser Ala Gln His Tyr Arg Leu Leu Ala Glu

260 265 270

Ser Gly Asp Ile Gln Ala Glu Met Val Val Arg Leu Gln Ala Lys Thr

275 280 285

Gly Gly Trp Ala Trp Ile Tyr Cys Leu Leu Tyr Ser Glu Gly Pro Glu

290 295 300

Gly Pro Ile Thr Ala Asn Asn Tyr Pro Ile Ser Asp Met Glu Ala Trp

305 310 315 320

Ser Leu Arg Gln Gln Leu Asn Ser Glu Asp Thr Gln Ala Ala Tyr Val

325 330 335

Leu Gly Thr Pro Thr Met Leu Pro Ser Phe Pro Glu Asn Ile Leu Ser

340 345 350

Gln Glu Glu Cys Ser Ser Thr Asn Pro Leu Phe Thr Ala Ala Leu Gly

355 360 365

Ala Pro Arg Ser Thr Ser Phe Pro Ser Ala Pro Glu Leu Ser Val Val

370 375 380

Ser Ala Ser Glu Glu Leu Pro Arg Pro Ser Lys Glu Leu Asp Phe Ser

385 390 395 400

Tyr Leu Thr Phe Pro Ser Gly Pro Glu Pro Ser Leu Gln Ala Glu Leu

405 410 415

Ser Lys Asp Leu Val Cys Thr Pro Pro Tyr Thr Pro His Gln Pro Gly

420 425 430

Gly Cys Ala Phe Leu Phe Ser Leu His Glu Pro Phe Gln Thr His Leu

435 440 445

Pro Thr Pro Ser Ser Thr Leu Gln Glu Gln Leu Thr Pro Ser Thr Ala

450 455 460

Thr Phe Ser Asp Gln Leu Thr Pro Ser Ser Ala Thr Phe Pro Asp Pro
465 470 475 480

Leu Thr Ser Pro Leu Gln Gly Gln Leu Thr Glu Thr Ser Val Arg Ser
485 490 495

Tyr Glu Asp Gln Leu Thr Pro Cys Thr Ser Thr Phe Pro Asp Gln Leu
500 505 510

Leu Pro Ser Thr Ala Thr Phe Pro Glu Pro Leu Gly Ser Pro Ala His
515 520 525

Glu Gln Leu Thr Pro Pro Ser Thr Ala Phe Gln Ala His Leu Asp Ser
530 535 540

Pro Ser Gln Thr Phe Pro Glu Gln Leu Ser Pro Asn Pro Thr Lys Thr
545 550 555 560

Tyr Phe Ala Gln Glu Gly Cys Ser Phe Leu Tyr Glu Lys Leu Pro Pro
565 570 575

Ser Pro Ser Ser Pro Gly Asn Gly Asp Cys Thr Leu Leu Ala Leu Ala
580 585 590

Gln Leu Arg Gly Pro Leu Ser Val Asp Val Pro Leu Val Pro Glu Gly
595 600 605

Leu Leu Thr Pro Glu Ala Ser Pro Val Lys Gln Ser Phe Phe His Tyr
610 615 620

Ser Glu Lys Glu Gln Asn Glu Ile Asp Arg Leu Ile Gln Gln Ile Ser
625 630 635 640

Gln Leu Ala Gln Gly Met Asp Arg Pro Phe Ser Ala Glu Ala Gly Thr
645 650 655

Gly Gly Leu Glu Pro Leu Gly Gly Leu Glu Pro Leu Asp Ser Asn Leu
660 665 670

Ser Leu Ser Gly Ala Gly Pro Pro Val Leu Ser Leu Asp Leu Lys Pro

675 680 685

Trp Lys Cys Gln Glu Leu Asp Phe Leu Ala Asp Pro Asp Asn Met Phe

690 695 700

Leu Glu Glu Thr Pro Val Glu Asp Ile Phe Met Asp Leu Ser Thr Pro

705 710 715 720

Asp Pro Ser Glu Glu Trp Gly Ser Gly Asp Pro Glu Ala Glu Gly Pro

725 730 735

Gly Gly Ala Pro Ser Pro Cys Asn Asn Leu Ser Pro Glu Asp His Ser

740 745 750

Phe Leu Glu Asp Leu Ala Thr Tyr Glu Thr Ala Phe Glu Thr Gly Val

755 760 765

Ser Ala Phe Pro Tyr Asp Gly Phe Thr Asp Glu Leu His Gln Leu Gln

770 775 780

Ser Gln Val Gln Asp Ser Phe His Glu Asp Gly Ser Gly Gly Glu Pro

785 790 795 800

Thr Phe

802

<210> 2
<211> 802
<212> PRT
<213> Mus musculus

<400> 2

Met Tyr Arg Ser Thr Lys Gly Ala Ser Lys Ala Arg Arg Asp Gln Ile

1 5 10 15

Asn Ala Glu Ile Arg Asn Leu Lys Glu Leu Leu Pro Leu Ala Glu Ala

20 25 30

Asp Lys Val Arg Leu Ser Tyr Leu His Ile Met Ser Leu Ala Cys Ile

35 40 45

Tyr Thr Arg Lys Gly Val Phe Phe Ala Gly Gly Thr Pro Leu Ala Gly

50 55 60

Pro Thr Gly Leu Leu Ser Ala Gln Glu Leu Glu Asp Ile Val Ala Ala

65 70 75 80

Leu Pro Gly Phe Leu Leu Val Phe Thr Ala Glu Gly Lys Leu Leu Tyr

85 90 95

Leu Ser Glu Ser Val Ser Glu His Leu Gly His Ser Met Val Asp Leu

100 105 110

Val Ala Gln Gly Asp Ser Ile Tyr Asp Ile Ile Asp Pro Ala Asp His

115 120 125

Leu Thr Val Arg Gln Gln Leu Thr Met Pro Ser Ala Leu Asp Ala Asp

130 135 140

Arg Leu Phe Arg Cys Arg Phe Asn Thr Ser Lys Ser Leu Arg Arg Gln

145 150 155 160

Ser Ser Gly Asn Lys Leu Val Leu Ile Arg Gly Arg Phe His Ala His

165 170 175

Pro Pro Gly Ala Tyr Trp Ala Gly Asn Pro Val Phe Thr Ala Phe Cys

180 185 190

Ala Pro Leu Glu Pro Arg Pro Arg Pro Gly Pro Gly Pro Gly Pro Gly

195 200 205

Pro Gly Pro Ala Ser Leu Phe Leu Ala Met Phe Gln Ser Arg His Ala

210 215 220

Lys Asp Leu Ala Leu Leu Asp Val Ser Glu Ser Val Leu Ile Tyr Leu

225 230 235 240

Gly Phe Glu Arg Ser Glu Leu Leu Cys Lys Ser Trp Tyr Gly Leu Leu
245 250 255
His Pro Glu Asp Leu Ala Gln Ala Ser Ser Gln His Tyr Arg Leu Leu
260 265 270
Ala Glu Ser Gly Asp Ile Gln Ala Glu Met Val Val Arg Leu Gln Ala
275 280 285
Lys His Gly Gly Trp Thr Trp Ile Tyr Cys Met Leu Tyr Ser Glu Gly
290 295 300
Pro Glu Gly Pro Phe Thr Ala Asn Asn Tyr Pro Ile Ser Asp Thr Glu
305 310 315 320
Ala Trp Ser Leu Arg Gln Gln Leu Asn Ser Glu Asp Thr Gln Ala Ala
325 330 335
Tyr Val Leu Gly Thr Pro Ala Val Leu Pro Ser Phe Ser Glu Asn Val
340 345 350
Phe Ser Gln Glu Gln Cys Ser Asn Pro Leu Phe Thr Pro Ser Leu Gly
355 360 365
Thr Pro Arg Ser Ala Ser Phe Pro Arg Ala Pro Glu Leu Gly Val Ile
370 375 380
Ser Thr Pro Glu Glu Leu Pro Gln Pro Ser Lys Glu Leu Asp Phe Ser
385 390 395 400
Tyr Leu Pro Phe Pro Ala Arg Pro Glu Pro Ser Leu Gln Ala Asp Leu
405 410 415
Ser Lys Asp Leu Val Cys Thr Pro Pro Tyr Thr Pro His Gln Pro Gly
420 425 430
Gly Cys Ala Phe Leu Phe Ser Leu His Glu Pro Phe Gln Thr His Leu
435 440 445
Pro Pro Pro Ser Ser Ser Leu Gln Glu Gln Leu Thr Pro Ser Thr Val

450 455 460

Thr Phe Ser Glu Gln Leu Thr Pro Ser Ser Ala Thr Phe Pro Asp Pro

465 470 475 480

Leu Thr Ser Ser Leu Gln Gly Gln Leu Thr Glu Ser Ser Ala Arg Ser

485 490 495

Phe Glu Asp Gln Leu Thr Pro Cys Thr Ser Ser Phe Pro Asp Gln Leu

500 505 510

Leu Pro Ser Thr Ala Thr Phe Pro Glu Pro Leu Gly Ser Pro Ala His

515 520 525

Glu Gln Leu Thr Pro Pro Ser Thr Ala Phe Gln Ala His Leu Asn Ser

530 535 540

Pro Ser Gln Thr Phe Pro Glu Gln Leu Ser Pro Asn Pro Thr Lys Thr

545 550 555 560

Tyr Phe Ala Gln Glu Gly Cys Ser Phe Leu Tyr Glu Lys Leu Pro Pro

565 570 575

Ser Pro Ser Ser Pro Gly Asn Gly Asp Cys Thr Leu Leu Ala Leu Ala

580 585 590

Gln Leu Arg Gly Pro Leu Ser Val Asp Val Pro Leu Val Pro Glu Gly

595 600 605

Leu Leu Thr Pro Glu Ala Ser Pro Val Lys Gln Ser Phe Phe His Tyr

610 615 620

Thr Glu Lys Glu Gln Asn Glu Ile Asp Arg Leu Ile Gln Gln Ile Ser

625 630 635 640

Gln Leu Ala Gln Gly Val Asp Arg Pro Phe Ser Ala Glu Ala Gly Thr

645 650 655

Gly Gly Leu Glu Pro Leu Gly Gly Leu Glu Pro Leu Asn Pro Asn Leu

660 665 670

Ser Leu Ser Gly Ala Gly Pro Pro Val Leu Ser Leu Asp Leu Lys Pro
675 680 685

Trp Lys Cys Gln Glu Leu Asp Phe Leu Val Asp Pro Asp Asn Leu Phe
690 695 700

Leu Glu Glu Thr Pro Val Glu Asp Ile Phe Met Asp Leu Ser Thr Pro
705 710 715 720

Asp Pro Asn Gly Glu Trp Gly Ser Gly Asp Pro Glu Ala Glu Val Pro
725 730 735

Gly Gly Thr Leu Ser Pro Cys Asn Asn Leu Ser Pro Glu Asp His Ser
740 745 750

Phe Leu Glu Asp Leu Ala Thr Tyr Glu Thr Ala Phe Glu Thr Gly Val
755 760 765

Ser Thr Phe Pro Tyr Glu Gly Phe Ala Asp Glu Leu His Gln Leu Gln
770 775 780

Ser Gln Val Gln Asp Ser Phe His Glu Asp Gly Ser Gly Gly Glu Pro
785 790 795 800

Thr Phe
802

<210> 3
<211> 802
<212> PRT
<213> Rattus norvegicus

<400> 3

Met Tyr Arg Ser Thr Lys Gly Ala Ser Lys Ala Arg Arg Asp Gln Ile
1 5 10 15

Asn Ala Glu Ile Arg Asn Leu Lys Glu Leu Leu Pro Leu Ala Glu Ala
20 25 30
Asp Lys Val Arg Leu Ser Tyr Leu His Ile Met Ser Leu Ala Cys Ile
35 40 45
Tyr Thr Arg Lys Gly Val Phe Phe Ala Gly Gly Thr Pro Leu Ala Gly
50 55 60
Pro Thr Gly Leu Leu Ser Ala Gln Glu Leu Glu Asp Ile Val Ala Ala
65 70 75 80
Leu Pro Gly Phe Leu Leu Val Phe Thr Ala Glu Gly Lys Leu Leu Tyr
85 90 95
Leu Ser Glu Ser Val Ser Glu His Leu Gly His Ser Met Val Asp Leu
100 105 110
Val Ala Gln Gly Asp Ser Ile Tyr Asp Ile Ile Asp Pro Ala Asp His
115 120 125
Leu Thr Val Arg Gln Gln Leu Thr Met Pro Ser Ala Leu Asp Ala Asp
130 135 140
Arg Leu Phe Arg Cys Arg Phe Asn Thr Ser Lys Ser Leu Arg Arg Gln
145 150 155 160
Ser Ala Gly Asn Lys Leu Val Leu Ile Arg Gly Arg Phe His Ala His
165 170 175
Pro Pro Gly Ala Tyr Trp Ala Gly Asn Pro Val Phe Thr Ala Phe Cys
180 185 190
Ala Pro Leu Glu Pro Arg Pro Arg Pro Gly Pro Gly Pro Gly Pro Gly
195 200 205
Pro Gly Pro Ala Ser Leu Phe Leu Ala Met Phe Gln Ser Arg His Ala
210 215 220
Lys Asp Leu Ala Leu Leu Asp Ile Ser Glu Ser Val Leu Ile Tyr Leu

225 230 235 240

Gly Phe Glu Arg Ser Glu Leu Leu Cys Lys Ser Trp Tyr Gly Leu Leu

245 250 255

His Pro Glu Asp Leu Ala His Ala Ser Ser Gln His Tyr Arg Leu Leu

260 265 270

Ala Glu Asn Gly Asp Ile Gln Ala Glu Met Val Val Arg Leu Gln Ala

275 280 285

Lys His Gly Gly Trp Thr Trp Ile Tyr Cys Met Leu Tyr Ser Asp Gly

290 295 300

Pro Glu Gly Pro Ile Thr Ala Asn Asn Tyr Pro Ile Ser Asp Thr Glu

305 310 315 320

Ala Trp Ser Leu Arg Gln Gln Leu Asn Ser Glu Asn Thr Gln Ala Ala

325 330 335

Tyr Val Leu Gly Thr Pro Ala Val Leu Pro Ser Phe Ser Glu Asn Val

340 345 350

Phe Ser Gln Glu His Cys Ser Asn Pro Leu Phe Thr Pro Ala Leu Gly

355 360 365

Thr Pro Arg Ser Ala Ser Phe Pro Arg Ala Pro Glu Leu Gly Val Ile

370 375 380

Ser Thr Ser Glu Glu Leu Ala Gln Pro Ser Lys Glu Leu Asp Phe Ser

385 390 395 400

Tyr Leu Pro Phe Pro Ala Arg Pro Glu Pro Ser Leu Gln Ala Asp Leu

405 410 415

Ser Lys Asp Leu Val Cys Thr Pro Pro Tyr Thr Pro His Gln Pro Gly

420 425 430

Gly Cys Ala Phe Leu Phe Ser Leu His Glu Pro Phe Gln Thr His Leu

435 440 445

Pro Pro Pro Ser Ser Ser Leu Gln Glu Gln Leu Thr Pro Ser Thr Val
450 455 460

Thr Phe Ser Glu Gln Leu Thr Pro Ser Ser Ala Thr Phe Pro Asp Pro
465 470 475 480

Leu Thr Ser Ser Leu Gln Gly Gln Leu Thr Glu Ser Ser Ala Arg Ser
485 490 495

Phe Glu Glu Gln Leu Thr Pro Cys Thr Ser Thr Phe Pro Asp Gln Leu
500 505 510

Leu Pro Ser Thr Ala Thr Phe Pro Glu Pro Leu Gly Ser Pro Thr His
515 520 525

Glu Gln Leu Thr Pro Pro Ser Thr Ala Phe Gln Ala His Leu Asn Ser
530 535 540

Pro Ser Gln Thr Phe Pro Glu Gln Leu Ser Pro Asn Pro Thr Lys Thr
545 550 555 560

Tyr Phe Ala Gln Glu Gly Cys Ser Phe Leu Tyr Glu Lys Leu Pro Pro
565 570 575

Ser Pro Ser Ser Pro Gly Asn Gly Asp Cys Thr Leu Leu Ala Leu Ala
580 585 590

Gln Leu Arg Gly Pro Leu Ser Val Asp Val Pro Leu Val Pro Glu Gly
595 600 605

Leu Leu Thr Pro Glu Ala Ser Pro Val Lys Gln Ser Phe Phe His Tyr
610 615 620

Thr Glu Lys Glu Gln Asn Glu Ile Asp Arg Leu Ile Gln Gln Ile Ser
625 630 635 640

Gln Leu Ala Gln Gly Met Asp Arg Pro Phe Ser Ala Glu Ala Gly Thr
645 650 655

Gly Gly Leu Glu Pro Leu Gly Gly Leu Glu Pro Leu Asn Pro Asn Leu

660 665 670

Ser Leu Ser Gly Ala Gly Pro Pro Val Leu Ser Leu Asp Leu Lys Pro

675 680 685

Trp Lys Cys Gln Glu Leu Asp Phe Leu Val Asp Pro Asp Asn Leu Phe

690 695 700

Leu Glu Glu Thr Pro Val Glu Asp Ile Phe Met Asp Leu Ser Thr Pro

705 710 715 720

Asp Pro Asn Gly Glu Trp Gly Ser Gly Asp Pro Glu Ala Glu Val Pro

725 730 735

Gly Gly Thr Leu Ser Pro Cys Asn Asn Leu Ser Pro Glu Asp His Ser

740 745 750

Phe Leu Glu Asp Leu Ala Thr Tyr Glu Thr Ala Phe Glu Thr Gly Val

755 760 765

Ser Thr Phe Pro Tyr Glu Gly Phe Ala Asp Glu Leu His Gln Leu Gln

770 775 780

Ser Gln Val Gln Asp Ser Phe His Glu Asp Gly Ser Gly Gly Glu Pro

785 790 795 800

Thr Phe

802

<210> 4
<211> 3252
<212> DNA
<213> Homo sapiens

<220>
<221> CDS
<222> (102)... (2510)

<400> 4

```
tgagcgagag acggggaagc acggaggagg aagccgccgg tgcgtcggga cgggagcgca     60

ggtgctcggg cacccgagct ggagctccgc agccgccggt c atg tac cgc tcc acc    116
                                              Met Tyr Arg Ser Thr
                                              1               5

aag ggc gcc tcc aag gcg cgc cgg gac cag atc aac gcc gag atc cgg      164
Lys Gly Ala Ser Lys Ala Arg Arg Asp Gln Ile Asn Ala Glu Ile Arg
                10                  15                  20

aac ctc aag gag ctg ctg ccg ctg gcc gaa gcg gac aag gtc cgg ctg      212
Asn Leu Lys Glu Leu Leu Pro Leu Ala Glu Ala Asp Lys Val Arg Leu
            25                  30                  35

tcc tac ctg cac atc atg agc ctc gcc tgc atc tac act cgc aag ggc      260
Ser Tyr Leu His Ile Met Ser Leu Ala Cys Ile Tyr Thr Arg Lys Gly
        40                  45                  50

gtc ttc ttc gct ggt ggc act cct ctg gcg ggc ccc acg ggg ctt ctc      308
Val Phe Phe Ala Gly Gly Thr Pro Leu Ala Gly Pro Thr Gly Leu Leu
    55                  60                  65

tca gct caa gag ctt gag gac atc gta gcg gca cta ccc ggc ttt ctg      356
Ser Ala Gln Glu Leu Glu Asp Ile Val Ala Ala Leu Pro Gly Phe Leu
70                  75                  80                  85

ctt gtg ttc aca gcc gag ggg aaa ttg ctc tac ctg tct gag agt gtg      404
Leu Val Phe Thr Ala Glu Gly Lys Leu Leu Tyr Leu Ser Glu Ser Val
                90                  95                  100

agc gag cat ctg ggc cac tcc atg gtg gac ctg gtt gcc cag ggt gac      452
Ser Glu His Leu Gly His Ser Met Val Asp Leu Val Ala Gln Gly Asp
```

105 110 115

agc atc tac gac atc att gac cca gct gac cac ctc act gtg cgc cag 500
Ser Ile Tyr Asp Ile Ile Asp Pro Ala Asp His Leu Thr Val Arg Gln
120 125 130

caa ctc acc ctg ccc tct gcc ctg gac act gat cgc ctc ttc cgc tgc 548
Gln Leu Thr Leu Pro Ser Ala Leu Asp Thr Asp Arg Leu Phe Arg Cys
135 140 145

cgc ttc aac acc tcc aag tcc ctc agg cgc cag agt gca ggc aac aaa 596
Arg Phe Asn Thr Ser Lys Ser Leu Arg Arg Gln Ser Ala Gly Asn Lys
150 155 160 165

ctc gtg ctt att cga ggc cga ttc cat gct cac cca cct gga gcc tac 644
Leu Val Leu Ile Arg Gly Arg Phe His Ala His Pro Pro Gly Ala Tyr
170 175 180

tgg gca gga aat ccc gtg ttc aca gct ttc tgt gcc cct ctg gag ccg 692
Trp Ala Gly Asn Pro Val Phe Thr Ala Phe Cys Ala Pro Leu Glu Pro
185 190 195

aga ccc cgc cca ggt cct ggc cct ggc cct ggc cct gcc tcg ctc ttc 740
Arg Pro Arg Pro Gly Pro Gly Pro Gly Pro Gly Pro Ala Ser Leu Phe
200 205 210

ctg gcc atg ttc cag agc cgc cat gct aaa gac ctg gct cta ctg gac 788
Leu Ala Met Phe Gln Ser Arg His Ala Lys Asp Leu Ala Leu Leu Asp
215 220 225

atc tcc gag agt gtc cta atc tac ctg ggc ttt gag cgc agt gaa ctg 836
Ile Ser Glu Ser Val Leu Ile Tyr Leu Gly Phe Glu Arg Ser Glu Leu
230 235 240 245

ctt tgt aaa tca tgg tat gga ctg ctg cac ccc gag gac ctg gcc cac 884
Leu Cys Lys Ser Trp Tyr Gly Leu Leu His Pro Glu Asp Leu Ala His

250 255 260

gct tct gct caa cac tac cgc ctg ttg gct gag agt gga gat att cag 932

Ala Ser Ala Gln His Tyr Arg Leu Leu Ala Glu Ser Gly Asp Ile Gln

265 270 275

gca gag atg gtg gtg agg cta cag gcc aag act gga ggc tgg gca tgg 980

Ala Glu Met Val Val Arg Leu Gln Ala Lys Thr Gly Gly Trp Ala Trp

280 285 290

att tac tgc ctg tta tac tca gaa ggt cca gag gga ccc att act gcc 1028

Ile Tyr Cys Leu Leu Tyr Ser Glu Gly Pro Glu Gly Pro Ile Thr Ala

295 300 305

aat aac tac cca atc agt gac atg gaa gcc tgg agc ctc cgc cag cag 1076

Asn Asn Tyr Pro Ile Ser Asp Met Glu Ala Trp Ser Leu Arg Gln Gln

310 315 320 325

ttg aac tct gaa gac acc cag gca gct tat gtc ctg ggc act ccg acc 1124

Leu Asn Ser Glu Asp Thr Gln Ala Ala Tyr Val Leu Gly Thr Pro Thr

330 335 340

atg ctg ccc tca ttc cct gaa aac att ctt tcc cag gaa gag tgc tcc 1172

Met Leu Pro Ser Phe Pro Glu Asn Ile Leu Ser Gln Glu Glu Cys Ser

345 350 355

agc act aac cca ctc ttc acc gca gca ctg ggg gct ccc aga agc acc 1220

Ser Thr Asn Pro Leu Phe Thr Ala Ala Leu Gly Ala Pro Arg Ser Thr

360 365 370

agc ttc ccc agt gct cct gaa ctg agt gtt gtc tct gca tca gaa gag 1268

Ser Phe Pro Ser Ala Pro Glu Leu Ser Val Val Ser Ala Ser Glu Glu

375 380 385

ctt ccc cga ccc tcc aaa gaa ctg gac ttc agt tac ctg aca ttc cct 1316

Leu Pro Arg Pro Ser Lys Glu Leu Asp Phe Ser Tyr Leu Thr Phe Pro

390 395 400 405

tct ggg cct gag cct tct ctc caa gca gaa cta agc aag gat ctt gtg 1364

Ser Gly Pro Glu Pro Ser Leu Gln Ala Glu Leu Ser Lys Asp Leu Val

410 415 420

tgc act cca cct tac acg ccc cat cag cca gga ggc tgt gcc ttc ctc 1412

Cys Thr Pro Pro Tyr Thr Pro His Gln Pro Gly Gly Cys Ala Phe Leu

425 430 435

ttc agc ctc cat gag ccc ttc cag acc cat ttg ccc acc cca tcc agc 1460

Phe Ser Leu His Glu Pro Phe Gln Thr His Leu Pro Thr Pro Ser Ser

440 445 450

act ctt caa gaa cag ctg act cca agc act gcg acc ttc tct gat cag 1508

Thr Leu Gln Glu Gln Leu Thr Pro Ser Thr Ala Thr Phe Ser Asp Gln

455 460 465

ttg acg ccc agc agt gca acc ttc cca gat cca cta act agc cca ctg 1556

Leu Thr Pro Ser Ser Ala Thr Phe Pro Asp Pro Leu Thr Ser Pro Leu

470 475 480 485

caa ggc cag ttg act gaa acc tcg gtc aga agc tat gaa gac cag ttg 1604

Gln Gly Gln Leu Thr Glu Thr Ser Val Arg Ser Tyr Glu Asp Gln Leu

490 495 500

act ccc tgc acc tcc acc ttc cca gac cag ctg ctt ccc agc aca gcc 1652

Thr Pro Cys Thr Ser Thr Phe Pro Asp Gln Leu Leu Pro Ser Thr Ala

505 510 515

acc ttc cca gag cct ctg ggc agc cct gcc cat gaa cag ctg act cct 1700

Thr Phe Pro Glu Pro Leu Gly Ser Pro Ala His Glu Gln Leu Thr Pro

520 525 530

ccc agc aca gca ttc caa gca cac ctg gac agc ccc agc caa acc ttc 1748

Pro Ser Thr Ala Phe Gln Ala His Leu Asp Ser Pro Ser Gln Thr Phe

535 540 545

cca gag caa ctg agc ccc aac cct acc aag act tac ttt gcc cag gag 1796

Pro Glu Gln Leu Ser Pro Asn Pro Thr Lys Thr Tyr Phe Ala Gln Glu

550 555 560 565

gga tgc agt ttt ctc tat gag aag ttg ccc cca agt cct agc agc cct 1844

Gly Cys Ser Phe Leu Tyr Glu Lys Leu Pro Pro Ser Pro Ser Ser Pro

570 575 580

ggt aat ggg gac tgc acg ctc ttg gcc cta gcc cag ctc cgg ggc ccc 1892

Gly Asn Gly Asp Cys Thr Leu Leu Ala Leu Ala Gln Leu Arg Gly Pro

585 590 595

ctc tct gtg gat gtc ccc ctg gtg ccc gaa ggc ctg ctc aca cct gag 1940

Leu Ser Val Asp Val Pro Leu Val Pro Glu Gly Leu Leu Thr Pro Glu

600 605 610

gcc tct cca gtc aag cag agt ttc ttc cac tac tct gaa aag gag cag 1988

Ala Ser Pro Val Lys Gln Ser Phe Phe His Tyr Ser Glu Lys Glu Gln

615 620 625

aat gag ata gac cgt ctc atc cag cag att agc caa ttg gct cag ggc 2036

Asn Glu Ile Asp Arg Leu Ile Gln Gln Ile Ser Gln Leu Ala Gln Gly

630 635 640 645

atg gac aga ccc ttc tca gct gag gct ggc act ggc gga cta gag cca 2084

Met Asp Arg Pro Phe Ser Ala Glu Ala Gly Thr Gly Gly Leu Glu Pro

650 655 660

ctt gga gga ctg gag ccc ctg gac tcc aac ctg tcc ctg tca ggg gca 2132

Leu Gly Gly Leu Glu Pro Leu Asp Ser Asn Leu Ser Leu Ser Gly Ala

665 670 675

ggc ccc cct gtg ctc agc ctg gac ctg aaa ccc tgg aaa tgc cag gag 2180

Gly Pro Pro Val Leu Ser Leu Asp Leu Lys Pro Trp Lys Cys Gln Glu

680 685 690

ctg gac ttc ctg gct gac cct gat aac atg ttc ctg gaa gag acg ccc 2228
Leu Asp Phe Leu Ala Asp Pro Asp Asn Met Phe Leu Glu Glu Thr Pro
695 700 705

gtg gaa gac atc ttc atg gat ctc tct acc cca gat ccc agt gag gaa 2276
Val Glu Asp Ile Phe Met Asp Leu Ser Thr Pro Asp Pro Ser Glu Glu
710 715 720 725

tgg ggc tca ggg gat cct gag gca gag ggc cca gga ggg gcc cca tcg 2324
Trp Gly Ser Gly Asp Pro Glu Ala Glu Gly Pro Gly Gly Ala Pro Ser
730 735 740

cct tgc aac aac ctg tcc cca gaa gac cac agc ttc ctg gag gac ctg 2372
Pro Cys Asn Asn Leu Ser Pro Glu Asp His Ser Phe Leu Glu Asp Leu
745 750 755

gcc aca tat gaa acc gcc ttt gag aca ggt gtc tca gca ttc ccc tat 2420
Ala Thr Tyr Glu Thr Ala Phe Glu Thr Gly Val Ser Ala Phe Pro Tyr
760 765 770

gat ggg ttt act gat gag ttg cat caa ctc cag agc caa gtt caa gac 2468
Asp Gly Phe Thr Asp Glu Leu His Gln Leu Gln Ser Gln Val Gln Asp
775 780 785

agc ttc cat gaa gat gga agt gga ggg gaa cca acg ttt tga ataagtctg 2519
Ser Phe His Glu Asp Gly Ser Gly Gly Glu Pro Thr Phe
790 795 800

tgacttaacg tcgtcaagta tggcatattg tcatcaagac gtggagccgc tctccacccc 2579
cccgggactg ttggggggat tctgagggcc agagggggat atatatgatt ccccaggccc 2639
tgcaggattt tggggggggg gaggtgggag ggcaagggag gggagcttct ttttaaaatc 2699
aagagacttc gagcgatccc agtttccatt tcaatctgta ttcactcgta gtgagtttcc 2759
ttgaatggga tttcaagcgg agaatggggg agtctcactt ccccgccgcc ttgccccatt 2819

ggcctgggcc agttctccac tcctaggggc caagccaccc ctagccttgg tgggggaaag 2879

gcagggccca cccgggccag cccgtgccct gaggggctct tgacacccac gtagaattct 2939

ctacacacca gtaacgggat ttcaattccg atggactctg ccgccctggc ggcccttcct 2999

gtgacttttg cgccccgcgc ctggggtggg gggtgcgaaa aaacgctacg ttcctttccg 3059

atggaggaag gcagacctgc cgtcacacgt gtgcttgcac gagtgcgtgt acctggtgcg 3119

ggactcaccc ggccgccaga ctgcctgggc ctgcccaaat ggccacctcg gtggtgctgc 3179

ggtgactttg tagccaactt tataataaag tccagtttgc ctttttggta aaaaaaaaaa 3239

aaaaaaaaaa aaa

<210> 5
<211> 3087
<212> DNA
<213> Mus musculus

<220>
<221> CDS
<222> (51)... (2459)

<400> 5

```
aggatcgcag gtgctcggga gccggagctg gagctccaca gccggcagtc atg tac       56
                                                       Met Tyr
                                                        1

cga tcc acc aag ggc gcc tcc aag gcg cgc cgc gac cag atc aac gcc     104
Arg Ser Thr Lys Gly Ala Ser Lys Ala Arg Arg Asp Gln Ile Asn Ala
    5                   10                  15

gag att cgg aac ctc aag gag ctg ctg ccg ttg gct gaa gcg gac aag     152
Glu Ile Arg Asn Leu Lys Glu Leu Leu Pro Leu Ala Glu Ala Asp Lys
```

20 25 30

gtc cgg ctg tcc tac ctg cac atc atg agt ctt gcc tgc atc tac act 200

Val Arg Leu Ser Tyr Leu His Ile Met Ser Leu Ala Cys Ile Tyr Thr

35 40 45 50

cgc aag ggt gtc ttc ttt gct gga ggc act cct ttg gct ggc ccc acc 248

Arg Lys Gly Val Phe Phe Ala Gly Gly Thr Pro Leu Ala Gly Pro Thr

55 60 65

ggg ctt ctc tct gct caa gag ctt gaa gac att gtg gca gca cta cct 296

Gly Leu Leu Ser Ala Gln Glu Leu Glu Asp Ile Val Ala Ala Leu Pro

70 75 80

gga ttt ctc ctt gta ttc aca gct gag ggg aag ttg cta tac ctg tcg 344

Gly Phe Leu Leu Val Phe Thr Ala Glu Gly Lys Leu Leu Tyr Leu Ser

85 90 95

gag agt gtg agc gag cat ctg ggc cac tct atg gtg gac ctg gtt gcc 392

Glu Ser Val Ser Glu His Leu Gly His Ser Met Val Asp Leu Val Ala

100 105 110

cag ggc gac agt atc tac gat atc att gac cct gct gac cat ctc act 440

Gln Gly Asp Ser Ile Tyr Asp Ile Ile Asp Pro Ala Asp His Leu Thr

115 120 125 130

gtg cgc cag cag ctc acc atg ccc tct gct ctg gat gct gat cgc ctt 488

Val Arg Gln Gln Leu Thr Met Pro Ser Ala Leu Asp Ala Asp Arg Leu

135 140 145

ttc cgt tgt cga ttc aac acc tcc aag tcc ctc cgg cgc cag agt tca 536

Phe Arg Cys Arg Phe Asn Thr Ser Lys Ser Leu Arg Arg Gln Ser Ser

150 155 160

gga aac aaa ctg gtg ctt att cga ggt cga ttc cat gct cac cca cct 584

Gly Asn Lys Leu Val Leu Ile Arg Gly Arg Phe His Ala His Pro Pro

```
                165                     170                     175
ggg gcc tac tgg gca gga aac cct gtg ttc acc gct ttc tgc gcc cca      632
Gly Ala Tyr Trp Ala Gly Asn Pro Val Phe Thr Ala Phe Cys Ala Pro
            180                     185                     190
ctg gag cca aga ccc cgc cct ggc ccc ggc cct ggc cct ggc cct ggt      680
Leu Glu Pro Arg Pro Arg Pro Gly Pro Gly Pro Gly Pro Gly Pro Gly
        195                     200                     205
cct gct tct ctc ttc ctg gcc atg ttc cag agc cgg cat gct aag gac      728
Pro Ala Ser Leu Phe Leu Ala Met Phe Gln Ser Arg His Ala Lys Asp
    210                     215                     220
cta gcc cta ctg gac gtt tct gaa agt gtc cta atc tac ctg ggc ttt      776
Leu Ala Leu Leu Asp Val Ser Glu Ser Val Leu Ile Tyr Leu Gly Phe
225                     230                     235                     240
gag cgc agc gaa ctg ctc tgt aaa tca tgg tat gga ctg cta cac ccc      824
Glu Arg Ser Glu Leu Leu Cys Lys Ser Trp Tyr Gly Leu Leu His Pro
                245                     250                     255
gag gac ctg gcc caa gct tct tct caa cac tac cgc ctg ttg gct gaa      872
Glu Asp Leu Ala Gln Ala Ser Ser Gln His Tyr Arg Leu Leu Ala Glu
            260                     265                     270
agt gga gat att cag gct gaa atg gtg gtg aga ctt caa gcc aag cat      920
Ser Gly Asp Ile Gln Ala Glu Met Val Val Arg Leu Gln Ala Lys His
        275                     280                     285
gga ggc tgg aca tgg att tac tgc atg cta tac tca gaa ggt cca gaa      968
Gly Gly Trp Thr Trp Ile Tyr Cys Met Leu Tyr Ser Glu Gly Pro Glu
    290                     295                     300
ggc cct ttt act gcc aat aac tac cct atc agt gac acg gaa gcc tgg     1016
Gly Pro Phe Thr Ala Asn Asn Tyr Pro Ile Ser Asp Thr Glu Ala Trp
305
```

```
            310                 315                 320
agc ctc cgc cag cag cta aac tct gaa gac acc cag gca gcc tat gtc    1064
Ser Leu Arg Gln Gln Leu Asn Ser Glu Asp Thr Gln Ala Ala Tyr Val
        325                 330                 335
cta gga acc cca gct gtg cta ccc tca ttc tct gag aat gtc ttc tcc    1112
Leu Gly Thr Pro Ala Val Leu Pro Ser Phe Ser Glu Asn Val Phe Ser
    340                 345                 350
cag gag caa tgc tct aat cca ctc ttt aca cca tcc ctg ggg act cct    1160
Gln Glu Gln Cys Ser Asn Pro Leu Phe Thr Pro Ser Leu Gly Thr Pro
355                 360                 365                 370
aga agt gcc agc ttc ccc agg gct cct gaa cta ggt gtg atc tca aca    1208
Arg Ser Ala Ser Phe Pro Arg Ala Pro Glu Leu Gly Val Ile Ser Thr
                375                 380                 385
cca gaa gag ctt ccc caa ccc tcc aaa gag ctg gac ttc agt tac ctg    1256
Pro Glu Glu Leu Pro Gln Pro Ser Lys Glu Leu Asp Phe Ser Tyr Leu
            390                 395                 400
cca ttc cct gct agg cct gag cct tcc ctc caa gca gac ctg agc aag    1304
Pro Phe Pro Ala Arg Pro Glu Pro Ser Leu Gln Ala Asp Leu Ser Lys
        405                 410                 415
gat ttg gtg tgt act cca cct tac aca ccc cac cag cca gga ggc tgt    1352
Asp Leu Val Cys Thr Pro Pro Tyr Thr Pro His Gln Pro Gly Gly Cys
    420                 425                 430
gcc ttc ctc ttc agc ctc cat gaa ccc ttc cag act cac ttg ccc cct    1400
Ala Phe Leu Phe Ser Leu His Glu Pro Phe Gln Thr His Leu Pro Pro
435                 440                 445                 450
ccg tcc agc tct ctc caa gaa cag ctg aca cca agt aca gtg act ttc    1448
Pro Ser Ser Ser Leu Gln Glu Gln Leu Thr Pro Ser Thr Val Thr Phe
```

455 460 465

tct gaa cag ttg aca ccc agc agt gct acc ttc cca gac cca cta acc 1496
Ser Glu Gln Leu Thr Pro Ser Ser Ala Thr Phe Pro Asp Pro Leu Thr

470 475 480

agt tca cta caa gga cag ttg aca gaa agc tca gcc aga agc ttt gaa 1544
Ser Ser Leu Gln Gly Gln Leu Thr Glu Ser Ser Ala Arg Ser Phe Glu

485 490 495

gac cag ttg act cca tgc acc tct tcc ttc cct gac cag cta ctt ccc 1592
Asp Gln Leu Thr Pro Cys Thr Ser Ser Phe Pro Asp Gln Leu Leu Pro

500 505 510

agc act gcc aca ttc cca gag cct ctg ggc agc ccc gcc cat gag cag 1640
Ser Thr Ala Thr Phe Pro Glu Pro Leu Gly Ser Pro Ala His Glu Gln

515 520 525 530

ctg act cct ccc agc aca gca ttc cag gct cat ctg aac agc ccc agc 1688
Leu Thr Pro Pro Ser Thr Ala Phe Gln Ala His Leu Asn Ser Pro Ser

535 540 545

caa acc ttc cca gag caa ctg agc ccc aat cct acc aag act tac ttc 1736
Gln Thr Phe Pro Glu Gln Leu Ser Pro Asn Pro Thr Lys Thr Tyr Phe

550 555 560

gcc cag gag gga tgc agt ttt ctc tat gag aag ttg ccc cca agt cct 1784
Ala Gln Glu Gly Cys Ser Phe Leu Tyr Glu Lys Leu Pro Pro Ser Pro

565 570 575

agc agc cct ggt aat ggg gac tgt aca ctc ctg gcc cta gct cag ctc 1832
Ser Ser Pro Gly Asn Gly Asp Cys Thr Leu Leu Ala Leu Ala Gln Leu

580 585 590

cgg ggc ccc ctc tct gtg gat gtc ccc ctg gtg ccc gaa ggc ctg ctc 1880
Arg Gly Pro Leu Ser Val Asp Val Pro Leu Val Pro Glu Gly Leu Leu

595 600 605 610

aca cct gag gcc tct cca gtc aag caa agt ttc ttc cac tac aca gag 1928

Thr Pro Glu Ala Ser Pro Val Lys Gln Ser Phe Phe His Tyr Thr Glu

615 620 625

aaa gag caa aat gag ata gat cgt ctc att cag cag atc agc cag ttg 1976

Lys Glu Gln Asn Glu Ile Asp Arg Leu Ile Gln Gln Ile Ser Gln Leu

630 635 640

gct cag ggc gtg gac agg ccc ttc tca gct gag gct ggc act ggg ggg 2024

Ala Gln Gly Val Asp Arg Pro Phe Ser Ala Glu Ala Gly Thr Gly Gly

645 650 655

ctg gag cca ctt gga ggg ctg gag ccc ctg aac cct aac ctg tcc ctg 2072

Leu Glu Pro Leu Gly Gly Leu Glu Pro Leu Asn Pro Asn Leu Ser Leu

660 665 670

tca ggg gct gga ccc cct gtg ctt agc ctg gat ctt aaa ccc tgg aaa 2120

Ser Gly Ala Gly Pro Pro Val Leu Ser Leu Asp Leu Lys Pro Trp Lys

675 680 685 690

tgc cag gag ctg gac ttc ctg gtt gac cct gat aat tta ttc ctg gaa 2168

Cys Gln Glu Leu Asp Phe Leu Val Asp Pro Asp Asn Leu Phe Leu Glu

695 700 705

gag acg cca gtg gaa gac atc ttc atg gat ctt tct act cca gac ccc 2216

Glu Thr Pro Val Glu Asp Ile Phe Met Asp Leu Ser Thr Pro Asp Pro

710 715 720

aat ggg gaa tgg ggt tca ggg gat cct gag gca gag gtc cca gga ggg 2264

Asn Gly Glu Trp Gly Ser Gly Asp Pro Glu Ala Glu Val Pro Gly Gly

725 730 735

acc ctg tca cct tgc aac aac ctg tcc cca gaa gat cac agc ttc ctg 2312

Thr Leu Ser Pro Cys Asn Asn Leu Ser Pro Glu Asp His Ser Phe Leu

```
        740                 745                 750

    gag gac ttg gcc acc tat gaa acc gcc ttt gag aca ggt gtc tca aca     2360
    Glu Asp Leu Ala Thr Tyr Glu Thr Ala Phe Glu Thr Gly Val Ser Thr
    755                 760                 765                 770

    ttc ccc tac gaa ggg ttt gct gat gag ttg cat caa ctc cag agc caa     2408
    Phe Pro Tyr Glu Gly Phe Ala Asp Glu Leu His Gln Leu Gln Ser Gln
                    775                 780                 785

    gtt caa gac agc ttc cat gaa gat gga agt gga ggg gaa cca acg ttt     2456
    Val Gln Asp Ser Phe His Glu Asp Gly Ser Gly Gly Glu Pro Thr Phe
                790                 795                 800

    tga ataagtctgt gacttaacgt cttcaagtat ggcatattgt catcaagacg tggagc   2515
    cgctctccac ccccccggga ctgttggggg gattctgggg gccagagggg gatatatctg   2575
    attctccagg ccctgaagga tttagggggg aggtgggagg gtaagggagg ggagcaactt   2635
    tttaaaatca agagacttcg agcgatccca gtttccattt caatctgtat tcactcgtag   2695
    tgagtttcct tgaatggatt tcaagcggag aatgggggag tctcacttcc tcaccgcgct   2755
    gccccatggg cctgggccag ttctccactc ctaggggcaa agccacccct gggctttggt   2815
    gggggaaagg catggcccac ctggggctag cctgtgcccc gaggggctct tgacacccac   2875
    gtagaattct ctacaaacca gtaacgggat ttcaattccg acggactctg ccgccctggc   2935
    ggctcttcct gtgacttttg cgccccgcgc ctggggtggg gggcgcgaag agacgctaca   2995
    ttcctttccg atggaggaag gcagatctgc cgtcacacgt gtgcttgcac gagtgcgtgt   3055
    acctggtgcg ggactcaccc ggccgccaga cc                                 3087
```

<210> 6
<211> 2460
<212> DNA
<213> Rattus norvegicus

<220>
<221> CDS
<222> (35)... (2443)

<400> 6

```
gggagccgga gctggagctc cacggccggc agtc atg tac cga tcc acc aag ggc     55
                                      Met Tyr Arg Ser Thr Lys Gly
                                       1               5
gcc tcc aag gcg cgc cgc gac cag atc aac gcc gag att cgg aac ctc      103
Ala Ser Lys Ala Arg Arg Asp Gln Ile Asn Ala Glu Ile Arg Asn Leu
        10                  15                  20
aag gaa ctg ctg ccg ttg gct gaa gcg gac aag gtc cgg ctg tcc tac      151
Lys Glu Leu Leu Pro Leu Ala Glu Ala Asp Lys Val Arg Leu Ser Tyr
    25                  30                  35
ctg cac atc atg agt ctt gcc tgc atc tac act cgc aag ggt gtc ttc      199
Leu His Ile Met Ser Leu Ala Cys Ile Tyr Thr Arg Lys Gly Val Phe
40                  45                  50                  55
ttt gct gga ggc act cct ttg gct ggc ccc acg ggg ctt ctc tct gct      247
Phe Ala Gly Gly Thr Pro Leu Ala Gly Pro Thr Gly Leu Leu Ser Ala
                60                  65                  70
caa gag ctt gaa gac ata gtg gca gca cta cct gga ttt cta ctt gtg      295
Gln Glu Leu Glu Asp Ile Val Ala Ala Leu Pro Gly Phe Leu Leu Val
            75                  80                  85
ttc aca gct gag ggg aag ttg cta tac ctg tcg gag agt gtg agc gag      343
Phe Thr Ala Glu Gly Lys Leu Leu Tyr Leu Ser Glu Ser Val Ser Glu
        90                  95                  100
cat ctg ggc cat tct atg gtg gat ctg gtt gcc cag ggt gac agt att      391
```

His Leu Gly His Ser Met Val Asp Leu Val Ala Gln Gly Asp Ser Ile
105 110 115

tac gac atc att gac cct gct gac cat ctc act gtg cgc cag cag ctc 439
Tyr Asp Ile Ile Asp Pro Ala Asp His Leu Thr Val Arg Gln Gln Leu
120 125 130 135

acc atg ccc tct gct ctg gat gct gat cgc ctt ttc cgt tgt cga ttt 487
Thr Met Pro Ser Ala Leu Asp Ala Asp Arg Leu Phe Arg Cys Arg Phe
140 145 150

aac aca tcc aag tcc ctc cgg cgc cag agt gca ggc aac aaa ctg gtg 535
Asn Thr Ser Lys Ser Leu Arg Arg Gln Ser Ala Gly Asn Lys Leu Val
155 160 165

ctt att cga ggt cga ttc cat gct cac cca cct ggg gcc tac tgg gca 583
Leu Ile Arg Gly Arg Phe His Ala His Pro Pro Gly Ala Tyr Trp Ala
170 175 180

gga aac ccc gtg ttc aca gct ttc tgt gcc cca ctg gag cca aga ccc 631
Gly Asn Pro Val Phe Thr Ala Phe Cys Ala Pro Leu Glu Pro Arg Pro
185 190 195

cgt ccc ggc cct ggc cct ggc cct ggc cct ggt cct gcc tct ctc ttc 679
Arg Pro Gly Pro Gly Pro Gly Pro Gly Pro Gly Pro Ala Ser Leu Phe
200 205 210 215

ctg gcc atg ttc cag agc cgg cat gct aag gac cta gcc cta ctg gac 727
Leu Ala Met Phe Gln Ser Arg His Ala Lys Asp Leu Ala Leu Leu Asp
220 225 230

att tct gaa agt gtc cta atc tac ctg ggc ttt gag cgc agc gaa ctg 775
Ile Ser Glu Ser Val Leu Ile Tyr Leu Gly Phe Glu Arg Ser Glu Leu
235 240 245

ctc tgt aaa tca tgg tat gga ctg cta cac ccc gag gac ctg gcc cac 823

Leu Cys Lys Ser Trp Tyr Gly Leu Leu His Pro Glu Asp Leu Ala His
250 255 260

gct tct tct caa cac tac cgc ctg ttg gct gaa aat gga gat att cag 871
Ala Ser Ser Gln His Tyr Arg Leu Leu Ala Glu Asn Gly Asp Ile Gln
265 270 275

gct gaa atg gtg gtg aga ctt caa gcc aag cat gga ggc tgg aca tgg 919
Ala Glu Met Val Val Arg Leu Gln Ala Lys His Gly Gly Trp Thr Trp
280 285 290 295

att tac tgc atg cta tac tcg gat ggt cca gaa ggc cct att act gcc 967
Ile Tyr Cys Met Leu Tyr Ser Asp Gly Pro Glu Gly Pro Ile Thr Ala
300 305 310

aat aac tac cct atc agt gac acg gaa gcc tgg agt ctt cgc cag cag 1015
Asn Asn Tyr Pro Ile Ser Asp Thr Glu Ala Trp Ser Leu Arg Gln Gln
315 320 325

cta aac tct gaa aac acc cag gca gcc tat gtc cta gga acc cca gct 1063
Leu Asn Ser Glu Asn Thr Gln Ala Ala Tyr Val Leu Gly Thr Pro Ala
330 335 340

gtg cta ccc tca ttc tct gag aat gtc ttc tcc cag gag cac tgc tct 1111
Val Leu Pro Ser Phe Ser Glu Asn Val Phe Ser Gln Glu His Cys Ser
345 350 355

aat cca ctc ttt aca cca gcc ctg ggg act cct aga agt gcc agc ttc 1159
Asn Pro Leu Phe Thr Pro Ala Leu Gly Thr Pro Arg Ser Ala Ser Phe
360 365 370 375

ccc agg gcc cct gaa cta ggt gtg atc tca aca tca gaa gag ctt gcc 1207
Pro Arg Ala Pro Glu Leu Gly Val Ile Ser Thr Ser Glu Glu Leu Ala
380 385 390

caa ccc tcc aaa gaa ctg gac ttc agt tac ctg cca ttc cct gca agg 1255

Gln Pro Ser Lys Glu Leu Asp Phe Ser Tyr Leu Pro Phe Pro Ala Arg
395 400 405

cct gag cct tcc ctc caa gca gac ttg agc aag gat ttg gtg tgt act 1303
Pro Glu Pro Ser Leu Gln Ala Asp Leu Ser Lys Asp Leu Val Cys Thr
410 415 420

cca cct tac aca ccc cac cag cca gga ggc tgc gcc ttc ctc ttc agc 1351
Pro Pro Tyr Thr Pro His Gln Pro Gly Gly Cys Ala Phe Leu Phe Ser
425 430 435

ctc cat gaa ccc ttc cag act cac ttg ccc cct cca tcc agc tct ctc 1399
Leu His Glu Pro Phe Gln Thr His Leu Pro Pro Pro Ser Ser Ser Leu
440 445 450 455

caa gaa cag ctg acg cca agc acg gtg act ttc tct gaa cag ttg aca 1447
Gln Glu Gln Leu Thr Pro Ser Thr Val Thr Phe Ser Glu Gln Leu Thr
460 465 470

cca agc agt gca acc ttc cca gat cca cta acc agt tca cta caa gga 1495
Pro Ser Ser Ala Thr Phe Pro Asp Pro Leu Thr Ser Ser Leu Gln Gly
475 480 485

cag ttg act gaa agc tca gcc aga agc ttt gaa gaa caa ttg act ccg 1543
Gln Leu Thr Glu Ser Ser Ala Arg Ser Phe Glu Glu Gln Leu Thr Pro
490 495 500

tgc acc tct acc ttc cct gac cag ctg ctt ccc agc act gcc acg ttc 1591
Cys Thr Ser Thr Phe Pro Asp Gln Leu Leu Pro Ser Thr Ala Thr Phe
505 510 515

cca gaa cct ctg ggt agc ccc acc cat gag cag ctg act cct ccc agc 1639
Pro Glu Pro Leu Gly Ser Pro Thr His Glu Gln Leu Thr Pro Pro Ser
520 525 530 535

aca gca ttc caa gca cat ctg aac agt cct agc caa acc ttc cca gag 1687

Thr Ala Phe Gln Ala His Leu Asn Ser Pro Ser Gln Thr Phe Pro Glu
540 545 550

caa ctg agc cct aat cct acc aag act tac ttc gcc cag gag gga tgc 1735
Gln Leu Ser Pro Asn Pro Thr Lys Thr Tyr Phe Ala Gln Glu Gly Cys
555 560 565

agt ttt ctc tat gag aag ttg ccc cca agt cct agc agc cct ggt aat 1783
Ser Phe Leu Tyr Glu Lys Leu Pro Pro Ser Pro Ser Ser Pro Gly Asn
570 575 580

ggg gac tgt aca ctc ttg gcc cta gct caa ctc cgg ggt ccc ctc tct 1831
Gly Asp Cys Thr Leu Leu Ala Leu Ala Gln Leu Arg Gly Pro Leu Ser
585 590 595

gtg gac gtc ccc ctg gtg cct gaa ggc ctg ctc aca cct gag gcc tct 1879
Val Asp Val Pro Leu Val Pro Glu Gly Leu Leu Thr Pro Glu Ala Ser
600 605 610 615

cca gtc aag caa agt ttc ttc cac tat aca gag aaa gag cag aat gag 1927
Pro Val Lys Gln Ser Phe Phe His Tyr Thr Glu Lys Glu Gln Asn Glu
620 625 630

ata gat cgt ctc atc cag cag atc agc cag ttg gct cag ggc atg gac 1975
Ile Asp Arg Leu Ile Gln Gln Ile Ser Gln Leu Ala Gln Gly Met Asp
635 640 645

agg ccc ttc tca gct gag gct ggc act ggg ggg ctg gag cca ctt gga 2023
Arg Pro Phe Ser Ala Glu Ala Gly Thr Gly Gly Leu Glu Pro Leu Gly
650 655 660

ggg ctg gag ccc ctg aac ccc aac ctg tcc ctg tca ggg gct gga ccc 2071
Gly Leu Glu Pro Leu Asn Pro Asn Leu Ser Leu Ser Gly Ala Gly Pro
665 670 675

cct gtg ctt agc ctg gat ctt aaa ccc tgg aaa tgc cag gag ctg gac 2119

Pro Val Leu Ser Leu Asp Leu Lys Pro Trp Lys Cys Gln Glu Leu Asp
680 685 690 695

ttc ttg gtt gac cct gat aat tta ttc ctg gaa gag acg cca gtg gaa 2167
Phe Leu Val Asp Pro Asp Asn Leu Phe Leu Glu Glu Thr Pro Val Glu
700 705 710

gac atc ttc atg gat ctt tct act cca gac ccc aat ggg gaa tgg ggt 2215
Asp Ile Phe Met Asp Leu Ser Thr Pro Asp Pro Asn Gly Glu Trp Gly
715 720 725

tca ggg gat cct gag gca gag gtc cca gga ggg acc ctg tca cct tgc 2263
Ser Gly Asp Pro Glu Ala Glu Val Pro Gly Gly Thr Leu Ser Pro Cys
730 735 740

aac aac ctg tcc cca gaa gat cac agc ttc ctg gag gac ttg gcc acc 2311
Asn Asn Leu Ser Pro Glu Asp His Ser Phe Leu Glu Asp Leu Ala Thr
745 750 755

tat gaa acc gcc ttt gag aca ggt gtc tca aca ttc ccc tat gaa ggg 2359
Tyr Glu Thr Ala Phe Glu Thr Gly Val Ser Thr Phe Pro Tyr Glu Gly
760 765 770 775

ttt gct gat gag ttg cat caa ctc cag agc caa gtt caa gac agc ttc 2407
Phe Ala Asp Glu Leu His Gln Leu Gln Ser Gln Val Gln Asp Ser Phe
780 785 790

cat gaa gat gga agt gga ggg gaa cca acg ttt tga ataagtctgt gactta 2459
His Glu Asp Gly Ser Gly Gly Glu Pro Thr Phe
795 800

<210> 7
<211> 35
<212> DNA
<213> Artificial Sequence

<220>
<223> Designed oligonucleotide primer for PCR

<400> 7
aagcacggag gaggaagccg ccggtgcgtc gggac 35

<210> 8

<211> 35
<212> DNA
<213> Artificial Sequence

<220>
<223> Designed oligonucleotide primer for PCR

<400> 8
acgggagcgc aggtgctcgg gcacccgagc tggag 35

<210> 9
<211> 35
<212> DNA
<213> Artificial Sequence

<220>
<223> Designed oligonucleotide primer for PCR

<400> 9
ggagagcggc tccacgtctt gatgacaata tgcca 35

<210> 10
<211> 35
<212> DNA
<213> Artificial Sequence

<220>
<223> Designed oligonucleotide primer for PCR

<400> 10
ccacgtcttg atgacaatat gccatacttg acgac 35

<210> 11
<211> 41
<212> DNA
<213> Artificial Sequence

<220>
<223> Designed oligonucleotide primer for PCR

<400> 11
gggcgctgca gcccagccac catgtaccga tccaccaagg g 41

<210> 12
<2I1> 20
<212> DNA
<213> Artificial Sequence

<220>
<223> Designed oligonucleotide primer for PCR

<400> 12
aatctcggcg ttgatctggt 20

<210> 13
<211> 20
<212> DNA
<213> Artificial Sequence

<220>
<223> Designed oligonucleotide primer for PCR

<400> 13
agtgaacagc aacagatact 20

<210> 14
<211> 20
<212> DNA
<213> Artificial Sequence

<220>
<223> Designed oligonucleotide primer for PCR

<400> 14
gcttcccgcg tccacgtgga 20

<210> 15
<211> 20
<212> DNA
<213> Artificial Sequence

<220>
<223> Designed oligonucleotide primer for PCR

<400> 15
gatgtgggca ttcagctccg 20

<210> 16
<211> 20
<212> DNA
<213> Artificial Sequence

<220>
<223> Designed oligonucleotide primer for PCR

<400> 16
cagagcttca ccattcgaga 20

<210> 17
<211> 20
<212> DNA
<213> Artificial Sequence

<220>
<223> Designed oligonucleotide primer for PCR

<400> 17
gtggccctgg tgtctgtccg 20

<210> 18
<211> 20
<212> DNA
<213> Artificial Sequence

<220>
<223> Designed oligonucleotide primer for PCR

<400> 18
ctggcccgcg cgtggggcaa 20

<210> 19
<211> 20
<212> DNA
<213> Artificial Sequence

<220>
<223> Designed oligonucleotide primer for PCR

<400> 19
acacatgctt cgccactgcc 20

<210> 20
<211> 20
<212> DNA
<213> Artificial Sequence

<220>
<223> Designed oligonucleotide primer for PCR

<400> 20
taatggccgc tctcacaggt 20

<210> 21
<211> 20
<212> DNA
<213> Artificial Sequence

<220>
<223> Designed oligonucleotide primer for PCR

<400> 21
agctgagtcc ctgaggcaga 20

<210> 22
<211> 20
<212> DNA
<213> Artificial Sequence

<220>
<223> Designed oligonucleotide primer for PCR

<400> 22
tgtgctgtgc cctgacactg 20

<210> 23
<211> 20
<212> DNA
<213> Artificial Sequence

<220>
<223> Designed oligonucleotide primer for PCR

<400> 23
ttgcagcgga gaacgaggag 20

<210> 24
<211> 20
<212> DNA
<213> Artificial Sequence

<220>
<223> Designed oligonucleotide primer for PCR

<400> 24
gatgagcact cggtcaacta 20

<210> 25
<211> 20
<212> DNA
<213> Artificial Sequence

<220>
<223> Designed oligonucleotide primer for PCR

<400> 25
gcatccagga gatccaggag 20

<210> 26
<211> 20
<212> DNA
<213> Artificial Sequence

<220>
<223> Designed oligonucleotide primer for PCR

<400> 26
ctggacaagg acgacgagag 20

<210> 27
<211> 20
<212) DNA
<213> Artificial Sequence

<220>
<223> Designed oligonucleotide primer for PCR

<400> 27
cctgcgaaag tacaaggagg 20

<210> 28
<211> 20
<212> DNA
<213> Artificial Sequence

<220>
<223> Designed oligonucleotide primer for PCR

<400> 28
cctaccatgt agtcagtctt 20

<210> 29
<211> 20
<212> DNA

<213> Artificial Sequence

<220>
<223> Designed oligonucleotide primer for PCR

<400> 29
ggtcaggaac tcgtactcct

<210> 30
<211> 20
<212> DNA
<213> Artificial Sequence

<220>
<223> Designed oligonucleotide primer for PCR

<400> 30
ttgggtgcct cctccacggg 29

<210> 31
<211> 20
<212> DNA
<213> Artificial Sequence

<220>
<223> Designed oligonucleotide primer for PCR

<400> 31
gcgggacttg atgctgtagc 30

<210> 32
<211> 20
<212> DNA
<213> Artificial Sequence

<220>
<223> Designed oligonucleotide primer for PCR

<400> 32
gtcttgtcgt cgtctgtgaa 31

<210> 33
<211> 7408
<212> DNA
<213> Mus musculus

<220>
<221> intron
<222> (1594) .. (2347)

<220>
<221> intron
<222> (2500).. (2673)

<220>
<221> intron
<222> (2777) .. (2886)

<220>
<221> intron
<222> (3161)..(3347)

<220>
<221> intron
<222> (3458).. (3784)

<220>
<221> intron
<222> (3921)..(4050)

<220>
<221> intron
<222> (5481) .. (6138)

<400> 33

aaggaaaaaa aaaaaaagaa aggtgtatgt tgtgcgctca ctcagtgaca agtgcacagg 60

cagaacgagg agccctggag ctaaagatgg agcaagaatt gaagggagat ggggagggga 120

ctctggcaga attaaagggt cttgggtagg tgcagcagcc actgagggca cagcagaccc 180

tggctacttg gcagcctccc cctcttcccg gctgaagcag tggggagagc tttctagagc 240

tgtgcggagg ccggtaggcc ccgcccgccg ctgccgccgc cgcagccgcc ggaggattcc 300

tgtcctaata tggagctggg attcccccgg ccccgccccc gccccccagc ccgccggaga 360

gactggggct cgcaagaggg cgggggaaca gctcgtcttg gggctgaca agcgggaggg 420

```
gatcgtggga gaggttcaaa cacacatcca gatcctcacc aggccctggg tctttgcctc    480
agtttccccg acaggtggct aagatgaact aatagaggaa aaaggaatcc ctgcagatca    540
cagtggagat gtttgtgttc ctatggtgct aaggaaatat gatcaagacc gaattcaagt    600
ggtctcttct ccacaggacc accattccac cctatcctgg agatttagac cctcaggtca    660
gggcatggag gcgaagaagg aattatttat ttgaaactgg ctaagggact ttcagattga    720
atagacccca gaaaagaccc ccagttgtga cctagcccct ccccaaaagc caaggaaagt    780
ccctcatgta atttcgaccc ctgcctggca gatggcggca aattggcaga ggaccaagcc    840
ccttctcatc ctttgcctcc ttagaaaaat ctatgttgat agcagcctcg ctttgccttc    900
tacaaactct cttgttaagg gcttcagacc accctaatcc atgtactgtt cctcctccta    960
atagaatgta atggaaaacc tgggtccctg caccccattc ctggctctgc acagctcttg   1020
ccagccggcc ccctctgcac cctcccttct cccccttccc ccgccccctc cctctcccgt   1080
tcgacgtcac gggatgacgt cggaagtctg ggagggagga ggagcacccc ccctccccag   1140
ccagtggctc cctctgcagc ttgctttagc ccagcctccc gcctcccgct gcccccccg    1200
tctctaaaaa cgagcccccc acgcctgtca ggagctatat aaggcggatc gaggcaggcg   1260
agggggcag cgctgccgag cggagcccag gagtggagcg agagcgagca agagcctgag    1320
cgaaaagacc gggaagcaag gaagaggaag cctccggtgc atcgggaaag gatcgcaggt   1380
gctcgggagc cggagctgga gctccacagc cggcagtc atg tac cga tcc acc aag   1436
                                          Met Tyr Arg Ser Thr Lys
                                          1               5
ggc gcc tcc aag gcg cgc cgc gac cag atc aac gcc gag att cgg aac    1484
Gly Ala Ser Lys Ala Arg Arg Asp Gln Ile Asn Ala Glu Ile Arg Asn
            10                  15                  20
ctc aag gag ctg ctg ccg ttg gct gaa gcg gac aag gtc cgg ctg tcc    1532
Leu Lys Glu Leu Leu Pro Leu Ala Glu Ala Asp Lys Val Arg Leu Ser
        25                  30                  35
tac ctg cac atc atg agt ctt gcc tgc atc tac act cgc aag ggt gtc    1580
Tyr Leu His Ile Met Ser Leu Ala Cys Ile Tyr Thr Arg Lys Gly Val
```

40 45 50

ttc ttt gct gga g gtgagcagct tgggctaccg gagaccagag ctgacgggga cca 1636

Phe Phe Ala Gly

55

gggatggagg agctgaggga atgtgctaaa actgccgctt gtctagcaca gcgtgctgga 1696

agcctgtgga gagaagggac ttgaggggac cctggacttc ctactttttc ttctgagctc 1756

catctagact agcctaaacg atagtcctag cactggattt gtgtgagata gagcgctaaa 1816

acagaatggt ccaggctccc attgcctcag aggcactcca ggaatccggg gagggtacgg 1876

aaggaagcct ggcaagctac agggaaagcc tgcaaaggca aagtatgagg aagagtagct 1936

tgtgctagaa aatgctgaga gggtctttct atgcgctctg gagctgttcg acgtcctgaa 1996

gccatcaccc tttctggcgc tgcccgcggt gctgaaatgg ccatagcccc ttttcgcagg 2056

agctgtccgc ggtgctgaat cccagtcctt tcgggagagc tctgtccaca gtgctgacag 2116

cagagggctg ctgaggttcc ggccaggctt ggaagtccag gggctccctg gctagatagt 2176

tttagcaaca ggtctcctgg ccaagatcca caagcatagg gtcaacaggt gttggcagaa 2236

ataggtctat gggaatttcc tgtgtcttct ccaagactca aaagatgttc tctttatttc 2296

tgtgttgtcc ctgattctta tcctgactca ccacatcttc tcaccctaca g gc act 2352

Gly Thr

60

cct ttg gct ggc ccc acc ggg ctt ctc tct gct caa gag ctt gaa gac 2400

Pro Leu Ala Gly Pro Thr Gly Leu Leu Ser Ala Gln Glu Leu Glu Asp

65 70 75

att gtg gca gca cta cct gga ttt ctc ctt gta ttc aca gct gag ggg 2448

Ile Val Ala Ala Leu Pro Gly Phe Leu Leu Val Phe Thr Ala Glu Gly

80 85 90

aag ttg cta tac ctg tcg gag agt gtg agc gag cat ctg ggc cac tct 2496

Lys Leu Leu Tyr Leu Ser Glu Ser Val Ser Glu His Leu Gly His Ser

95 100 105

atg gtgagtacta aaagtccttg catctcaagt tggggtatat gtgagataaa atgagc 2555
Met

ctctcactac tgaaaacaga gttattagag gcgagtgtgg gggagtcttc cctaagaaaa 2615
atcattggtt gcagataggc tcttgctgcc ttcactaatg atcacttctc ctttctag 2673
gtg gac ctg gtt gcc cag ggc gac agt atc tac gat atc att gac cct 2721
Val Asp Leu Val Ala Gln Gly Asp Ser Ile Tyr Asp Ile Ile Asp Pro
110 115 120 125
gct gac cat ctc act gtg cgc cag cag ctc acc atg ccc tct gct ctg 2769
Ala Asp His Leu Thr Val Arg Gln Gln Leu Thr Met Pro Ser Ala Leu
130 135 140
gat gct g gtaagaacct cctctcggtt cttcagttta ctcctctgct gccctgccct 2826
Asp Ala

aactatctac tctcctccaa tgcccaccct cttagtcagt ttttcctttt gctcacctag 2886

at cgc ctt ttc cgt tgt cga ttc aac acc tcc aag tcc ctc cgg cgc 2933
Asp Arg Leu Phe Arg Cys Arg Phe Asn Thr Ser Lys Ser Leu Arg Arg
145 150 155
cag agt tca gga aac aaa ctg gtg ctt att cga ggt cga ttc cat gct 2981
Gln Ser Ser Gly Asn Lys Leu Val Leu Ile Arg Gly Arg Phe His Ala
160 165 170 175
cac cca cct ggg gcc tac tgg gca gga aac cct gtg ttc acc gct ttc 3029
His Pro Pro Gly Ala Tyr Trp Ala Gly Asn Pro Val Phe Thr Ala Phe
180 185 190
tgc gcc cca ctg gag cca aga ccc cgc cct ggc ccc ggc cct ggc cct 3077
Cys Ala Pro Leu Glu Pro Arg Pro Arg Pro Gly Pro Gly Pro Gly Pro

```
            195                 200                 205
ggc cct ggt cct gct tct ctc ttc ctg gcc atg ttc cag agc cgg cat      3125
Gly Pro Gly Pro Ala Ser Leu Phe Leu Ala Met Phe Gln Ser Arg His
        210                 215                 220
gct aag gac cta gcc cta ctg gac gtt tct gaa ag gtaagcccaa agtgttc    3177
Ala Lys Asp Leu Ala Leu Leu Asp Val Ser Glu Ser
    225                 230                 235
aaactccagt aagaagggag gccagaaaga agggaacttt agattcgtga tcttagattc    3237
agggcaggga ggatggggct taagtgggca gagagcatgg gagggagtga agtgcatgca    3297
ttttgagtaa ggtaaacaga aagctgacct catcatttcc accttcccag t gtc cta     3354
                                                          Val Leu

atc tac ctg ggc ttt gag cgc agc gaa ctg ctc tgt aaa tca tgg tat      3402
Ile Tyr Leu Gly Phe Glu Arg Ser Glu Leu Leu Cys Lys Ser Trp Tyr
        240                 245                 250
gga ctg cta cac ccc gag gac ctg gcc caa gct tct tct caa cac tac      3450
Gly Leu Leu His Pro Glu Asp Leu Ala Gln Ala Ser Ser Gln His Tyr
    255                 260                 265
cgc ctg t gtgagtgtcc tgagaggccg tgcataacac aggaagctgg gagaaagcat     3507
Arg Leu
270
gggagacagg ccagggactg gctgtggtcc aaactgatgt taaggagttt cggaggctac    3567
agagtgagct tgaggatgag aagtcaaggc aagaatagga cagagttaga aaacactgtg    3627
tgataaggtc aagtggggag cctagaggta caggttaggg tagttagaag agaatatgtc    3687
atggctccct caattcagtg tagaggtaag aaaggtgggt gtgtaggtgg tgttgattga    3747
tggaccttct aatccggtat tcctttttc tccccag tg gct gaa agt gga gat       3801
                                           Leu Ala Glu Ser Gly Asp
```

275

att cag gct gaa atg gtg gtg aga ctt caa gcc aag cat gga ggc tgg 3849
Ile Gln Ala Glu Met Val Val Arg Leu Gln Ala Lys His Gly Gly Trp
280 285 290

aca tgg att tac tgc atg cta tac tca gaa ggt cca gaa ggc cct ttt 3897
Thr Trp Ile Tyr Cys Met Leu Tyr Ser Glu Gly Pro Glu Gly Pro Phe
295 300 305

act gcc aat aac tac cct atc ag gtaagctgta agatacaaga tggcggagag g 3951
Thr Ala Asn Asn Tyr Pro Ile Ser
310 315

ggaggggagc tgaggtcagc atagaagaaa tgcaacgaag aaaactactc tggtaatgga 4011

cagcagaccc ttacaagctg ccacctcttc cctttccag t gac acg gaa gcc tgg 4066
Asp Thr Glu Ala Trp
320

agc ctc cgc cag cag cta aac tct gaa gac acc cag gca gcc tat gtc 4114
Ser Leu Arg Gln Gln Leu Asn Ser Glu Asp Thr Gln Ala Ala Tyr Val
325 330 335

cta gga acc cca gct gtg cta ccc tca ttc tct gag aat gtc ttc tcc 4162
Leu Gly Thr Pro Ala Val Leu Pro Ser Phe Ser Glu Asn Val Phe Ser
340 345 350

cag gag caa tgc tct aat cca ctc ttt aca cca tcc ctg ggg act cct 4210
Gln Glu Gln Cys Ser Asn Pro Leu Phe Thr Pro Ser Leu Gly Thr Pro
355 360 365 370

aga agt gcc agc ttc ccc agg gct cct gaa cta ggt gtg atc tca aca 4258
Arg Ser Ala Ser Phe Pro Arg Ala Pro Glu Leu Gly Val Ile Ser Thr
375 380 385

cca gaa gag ctt ccc caa ccc tcc aaa gag ctg gac ttc agt tac ctg 4306

Pro Glu Glu Leu Pro Gln Pro Ser Lys Glu Leu Asp Phe Ser Tyr Leu
390 395 400

cca ttc cct gct agg cct gag cct tcc ctc caa gca gac ctg agc aag 4354
Pro Phe Pro Ala Arg Pro Glu Pro Ser Leu Gln Ala Asp Leu Ser Lys
405 410 415

gat ttg gtg tgt act cca cct tac aca ccc cac cag cca gga ggc tgt 4402
Asp Leu Val Cys Thr Pro Pro Tyr Thr Pro His Gln Pro Gly Gly Cys
420 425 430

gcc ttc ctc ttc agc ctc cat gaa ccc ttc cag act cac ttg ccc cct 4450
Ala Phe Leu Phe Ser Leu His Glu Pro Phe Gln Thr His Leu Pro Pro
435 440 445 450

ccg tcc agc tct ctc caa gaa cag ctg aca cca agt aca gtg act ttc 4498
Pro Ser Ser Ser Leu Gln Glu Gln Leu Thr Pro Ser Thr Val Thr Phe
455 460 465

tct gaa cag ttg aca ccc agc agt gct acc ttc cca gac cca cta acc 4546
Ser Glu Gln Leu Thr Pro Ser Ser Ala Thr Phe Pro Asp Pro Leu Thr
470 475 480

agt tca cta caa gga cag ttg aca gaa agc tca gcc aga agc ttt gaa 4594
Ser Ser Leu Gln Gly Gln Leu Thr Glu Ser Ser Ala Arg Ser Phe Glu
485 490 495

gac cag ttg act cca tgc acc tct tcc ttc cct gac cag cta ctt ccc 4642
Asp Gln Leu Thr Pro Cys Thr Ser Ser Phe Pro Asp Gln Leu Leu Pro
500 505 510

agc act gcc aca ttc cca gag cct ctg ggc agc ccc gcc cat gag cag 4690
Ser Thr Ala Thr Phe Pro Glu Pro Leu Gly Ser Pro Ala His Glu Gln
515 520 525 530

ctg act cct ccc agc aca gca ttc cag gct cat ctg aac agc ccc agc 4738

Leu Thr Pro Pro Ser Thr Ala Phe Gln Ala His Leu Asn Ser Pro Ser
535 540 545

caa acc ttc cca gag caa ctg agc ccc aat cct acc aag act tac ttc 4786
Gln Thr Phe Pro Glu Gln Leu Ser Pro Asn Pro Thr Lys Thr Tyr Phe
550 555 560

gcc cag gag gga tgc agt ttt ctc tat gag aag ttg ccc cca agt cct 4834
Ala Gln Glu Gly Cys Ser Phe Leu Tyr Glu Lys Leu Pro Pro Ser Pro
565 570 575

agc agc cct ggt aat ggg gac tgt aca ctc ctg gcc cta gct cag ctc 4882
Ser Ser Pro Gly Asn Gly Asp Cys Thr Leu Leu Ala Leu Ala Gln Leu
580 585 590

cgg ggc ccc ctc tct gtg gat gtc ccc ctg gtg ccc gaa ggc ctg ctc 4930
Arg Gly Pro Leu Ser Val Asp Val Pro Leu Val Pro Glu Gly Leu Leu
595 600 605 610

aca cct gag gcc tct cca gtc aag caa agt ttc ttc cac tac aca gag 4978
Thr Pro Glu Ala Ser Pro Val Lys Gln Ser Phe Phe His Tyr Thr Glu
615 620 625

aaa gag caa aat gag ata gat cgt ctc att cag cag atc agc cag ttg 5026
Lys Glu Gln Asn Glu Ile Asp Arg Leu Ile Gln Gln Ile Ser Gln Leu
630 635 640

gct cag ggc gtg gac agg ccc ttc tca gct gag gct ggc act ggg ggg 5074
Ala Gln Gly Val Asp Arg Pro Phe Ser Ala Glu Ala Gly Thr Gly Gly
645 650 655

ctg gag cca ctt gga ggg ctg gag ccc ctg aac cct aac ctg tcc ctg 5122
Leu Glu Pro Leu Gly Gly Leu Glu Pro Leu Asn Pro Asn Leu Ser Leu
660 665 670

tca ggg gct gga ccc cct gtg ctt agc ctg gat ctt aaa ccc tgg aaa 5170

Ser Gly Ala Gly Pro Pro Val Leu Ser Leu Asp Leu Lys Pro Trp Lys
675 680 685 690

tgc cag gag ctg gac ttc ctg gtt gac cct gat aat tta ttc ctg gaa 5218
Cys Gln Glu Leu Asp Phe Leu Val Asp Pro Asp Asn Leu Phe Leu Glu
695 700 705

gag acg cca gtg gaa gac atc ttc atg gat ctt tct act cca gac ccc 5266
Glu Thr Pro Val Glu Asp Ile Phe Met Asp Leu Ser Thr Pro Asp Pro
710 715 720

aat ggg gaa tgg ggt tca ggg gat cct gag gca gag gtc cca gga ggg 5314
Asn Gly Glu Trp Gly Ser Gly Asp Pro Glu Ala Glu Val Pro Gly Gly
725 730 735

acc ctg tca cct tgc aac aac ctg tcc cca gaa gat cac agc ttc ctg 5362
Thr Leu Ser Pro Cys Asn Asn Leu Ser Pro Glu Asp His Ser Phe Leu
740 745 750

gag gac ttg gcc acc tat gaa acc gcc ttt gag aca ggt gtc tca aca 5410
Glu Asp Leu Ala Thr Tyr Glu Thr Ala Phe Glu Thr Gly Val Ser Thr
755 760 765 770

ttc ccc tac gaa ggg ttt gct gat gag ttg cat caa ctc cag agc caa 5458
Phe Pro Tyr Glu Gly Phe Ala Asp Glu Leu His Gln Leu Gln Ser Gln
775 780 785

gtt caa gac agc ttc cat gaa g gtaagtctag cctgaatgtc caagagccct gc 5512
Val Gln Asp Ser Phe His Glu
790

ccttctaatc agacattgca tagattgggt gaatcagtcc ccaactctga aactctgttt 5572
tattaagaga acaatattac ctcctactaa gaagagtagt gaggtaggaa taatacaaag 5632
ctttgtgtga aagatgagta gacctggtgg gcgggggagg tgagctagaa aaacgcgata 5692
gacaatccct aggcaaaagc ttgaaagctt ctgagagacc tagaccagac aacaccgtca 5752

ttttatagac aaaaataatc aaggccccag agttaaagaa actttaagtg gcacaaaaat 5812

tgatagaagt tgatgcttcc ccctgaaggg gacccagagc aacaactggt taaaattagg 5872

agacagaaag aacaatgcca agcccctagc tccaatctgg cggccttgtg ctgtttgtcc 5932

aaagctgtgg ccacagtttc cctccatatt tgcatattgc ctcttatctg ctgacaccct 5992

ggggatcagt tcatttggct aacacatttg acgtccatag actatagcaa tattgtacca 6052

ctgcctgagc ccaatgacgc ttttactgaa taagcttgac taacatacgc actttctctc 6112

ttctctctct ctctctttcc ccacag at gga agt gga ggg gaa cca acg ttt 6164

Asp Gly Ser Gly Gly Glu Pro Thr Phe

795 800

tga ataagtctgt gacttaacgt cttcaagtat ggcatattgt catcaagacg tggagc 6223

cgctctccac ccccccggga ctgttggggg gattctgggg gccagagggg gatatatctg 6283

attctccagg ccctgaagga tttagggggg aggtgggagg gtaagggagg ggagcaactt 6343

tttaaaatca agagacttcg agcgatccca gtttccattt caatctgtat tcactcgtag 6403

tgagtttcct tgaatggatt tcaagcggag aatgggggag tctcacttcc tcaccgcgct 6463

gccccatggg cctgggccag ttctccactc ctaggggcaa agccacccct gggctttggt 6523

gggggaaagg catggcccac ctggggctag cctgtgcccc gaggggctct tgacacccac 6583

gtagaattct ctacaaacca gtaacgggat ttcaattccg acggactctg ccgccctggc 6643

ggctcttcct gtgacttttg cgccccgcgc ctggggtggg gggcgcgaag agacgctaca 6703

ttcctttccg atggaggaag gcagatctgc cgtcacacgt gtgcttgcac gagtgcgtgt 6763

acctggtgcg ggactcaccc ggccgccaga ccgcctaggc ttgcccaggt ggccacctcg 6823

tggtgctgcg gtgactttgt agccaacttt ataataaagt ccagtttgcc tttttggtat 6883

ctctggtgtc atgcgctatt gtgaaaaggg aagggagggg aagggagaga ttgaggagcc 6943

cagataggag gctggggcag gagtcacagg ttagacctcc tctcagccct ggtatctcta 7003

agtgagtttg ttcatatctc catttgactc tgcttggtcc acactgtgct agaagactaa 7063

gtacttgtca gaagcagaca ttgcaccaaa gacactggag tcttctctct gccctgggtt 7123

tatggtgtga tggggaggaa agagcctggg gctgagcaag tttgtcactg gtcttggata 7183

tgggtttaaa gtttctggtc atttcctgcc tggtctttca ggatattgat ttcctcatgg 7243

aggcttagat tttaaaaatc agaagctgaa acctgttacg cttgcgtagg gctgttcagt 7303
tagcaaatac ccaatccact gcaataaatt tccacttcat tgggaaagca acccgataac 7363
gggtgttcct ccagttacag gtgagaaaca catcaacccc tcccc 7408

<210> 34
<211> 20775
<212> DNA
<213> Mus musculus

<220>
<221> intron
<222> (9769)..(10522)

<220>
<221> intron
<222> (10675)..(10848)

<220>
<221> intron
<222> (10952).. (11061)

<220>
<221> intron
<222> (11336).. (11522)

<220>
<221> intron
<222> (11633)..(11959)

<220>
<221> intron
<222> (12096)..(12225)

<220>
<221> intron
<222> (13656).. (14313)

<400> 34

```
tctcctgatt tttaaagccc ctctgtcttt cctggccccg cttggcctcc ctgaagatgc    60
cctgccctct gcatacctag ggccaatagg agtgatgagc ccatgtcatg tctgctctgg   120
gattctaatg acccaatccc tacaccagac acacaaggca tggacatctg ctcacctgta   180
ggctccatgt cactgggtac acgcaggtga tattacagac aagtgtaaag cttcggtctg   240
tggtggcctg caggtttgtg tgtacctagg tagaagagga agtgaggagg caccagtcag   300
aagcaactct gagaaacagg agccagaatt taagctgggt aagaacatga aagatggcca   360
aggattgcaa ttgttggccc ctggagaaca cactgggact ggtcttggat gttctgttct   420
gtactggagg gatatgggat gcctgctgac acacaggaag ggtctgaacc cagaccctca   480
gggtcactag gtatgcgtac ctcagtttcc taaggctcat tgacttcttt gttcgtttat   540
tcggagaaca gcacctattc tggccacctc cataaggagg gtttcaggaa gcacccaggg   600
ctatgaaccc atcgagccac ttctgtctga ctgcattcaa aacgatagtt tccttaagac   660
aatggccact ccccgtgcat tctccaacac ttactccgtt ccttccgtgc ctatggcttt   720
gttctgagtg ttttgacaaa ttagttcacc tggctcttgt gtcagagctc taacacaaat   780
tgtattctcc tcttcacaac tctatttaat acactggtaa actgaggcat gagaggctgc   840
agtccttacc tcagcagtgt cacagtctgt aacagaggca agacctccct ccaggcccca   900
ccctcttgcc taccctgcct tggctctctt ccggtctcta tgcgaagatc ctatgtattc   960
```

```
agacccttct tttaattttt taatggcttt tttatttact ctgtgtgcat gcatgtgagt   1020
gagtgtgtgc cgtggtttat gtgtggcagt cagaggacat ctttcggctc tccttccacc   1080
atggaggtcc tggggattga agttaggctg tcaggcttgg cagcaagtgc ctttacctga   1140
ttaaccttgc tgcccacccc taaccccttc ttgctggctc ttccattcgg aataaggcaa   1200
accatgccct ttcagccttc ttttcaccga gaagaattat cttccttctt ttcatcttct   1260
caatttttcc tacaaatata cctggaatgc ttcgatcaga gctgatggca gacaaaggtg   1320
acagctccta cccaggggtc tccaatacaa gccagagaag acagcagctc attaatgaaa   1380
cgaagtgtaa aactgtcacc atcacaactg gcaacagaag cacagggaac cctgggacct   1440
acagctgggg atttgacccg atagagaaga ttttctggag tgatatttga gccaagctat   1500
tgtgaaaaat gaggatcagg tgcaaggaga ggcaaggggc gtgcatgtgt gcacggagct   1560
gcagaaccac aatggaagag ctgcctgtgg ctagaagaga gggacgggga ggaaggaggc   1620
agggtcgggg gttgggggga agatcaccag agtgcagcct gggagaaggc ttagggtggc   1680
tcctcacagt tcttgacatc cttgatgatg gaagctaagc ctggccactt cacctcatag   1740
gacagctcct gcagccatac ctgctgcgta aagaagcctc agctcccttc ccccacagca   1800
ccacctcatt cccatctaat taattgtttg cttttacctt ggctactgct actcaccaca   1860
ccttataaag ccatgagacc acgctcttgc taatctcatc ctccccaccc acccagcaca   1920
gccacgttgg tcagttggcc acttgactcc caagcaacgt ggcgcaaaca cacctcccta   1980
ggaaccccac tgccatatcc ctaggcttgg tcttccccat gttgcagcca ccgagcaccc   2040
cagatgcccc tttccagaca gcatctcatt cagatggctt cctcttaccc tgtggaagct   2100
ccatgatgtt aaagccaagc ttgtgcctct ccccgacccc cgccagtatc caccagagag   2160
gctggcctct ggcctcaatt catcccacag ccctgtgcag tgagcgtgac atccatcccc   2220
acggtccctg tgacagatgc tggcagtatg gcggccagcc tgaggtcccg tgtgggtggg   2280
caaggaatag catttgagaa gcagaggcag gagggtcaca agttcaaggt tatcctctgc   2340
tatatatgag gatgcatgcg attctttctc aaattttaga aaatgtgcat caaggaagag   2400
gcacaggtcg ggtgtgaggg cagagggggc aagctagtca cctctagaag atcagcaggg   2460
cagagttccc ttgctgagga aagtcagaca tgaacatgtg aggcagatct agagggcagg   2520
ggccacaccc tcggtttcta tcttcatgcc actgaggcac atggggtccc tggtctgaat   2580
```

tttatctctg gtccatgaat taattttcct ctcctccttg gagcagatgc ctccagtcag 2640
ccccatcctc aagccttgcc cagataccttt caattttctc atccaggttc cagtctctcc 2700
ctcctgccca caccatccct ccccgccctc acctctgctc agcccactcc cctggctctg 2760
accgtttcta tgcgtaggtg gcagcgtgta ccctcttcac aggagatttg ttgatttcat 2820
aaccataaat agataaaatg ttctgagtgc ttccatgagc gagtagaatt gagggagtga 2880
tcacacaatg aaaaggctgt aggagaagga aaacagcctg tggagacaca gctgaaaccg 2940
gcttggtgct gcacaaacca gcactacctg agggcgagct tgccgttgca taagaggtat 3000
accataaaca caggacttgg gactgccaga gaaccttctg gaaaacactt atgagactgc 3060
aagtctgtca attcaaagga acaatatatt aagaaaatct agatttaaaa tgaaaacaga 3120
acgtggaagc caacaaacat ggatttttaa ttctgagttc atctcatttt ggctgtgtga 3180
ctatgagcaa gtttctcatc ctctctggga aggtgtatat tcatctcttg ggtcagacta 3240
gagggaccaa tcatataata tgctcatttt ttcctctaag aaaaagtggt cttctcgatt 3300
acaacttaac ctccaatatg gaacaatttg tcttcccaaa acgcagtccc aagacactaa 3360
ggatggatag ggtaacctgc tttttcattg tcaagtagaa ctcatgttga catggaaatg 3420
ggttatgcac aatcattctt ggatggggag accatatatt catagttaca aagaaagcta 3480
gacattagga aggacttccc aagttcttct ccctaagatg ggtaaagaga gtagagagag 3540
gatgtgacag ctcagtttgt tctgagactg gagagctttc cagagagagg gaaagctatt 3600
tctttacctt ctgctctaag ggtggcagga ttttctgtca agggttaggt agtaggtgtt 3660
tgggcttggt gggagctcta gtcccttctg cacttaactc tgtgactgtg tgaagaaggg 3720
caccagccat aagtatgtga atggtctgaa tgtgtcccag taaaacttca ttaatgaaaa 3780
gaaacagcgg accagatttt attcggtgcc atagtgtata ggccccaatc tcgttctaca 3840
cggcaagaga acaagtttga atggggagga atgaaccatg cacagggcac tgccagagcc 3900
ctgctgtctg actttaagtc attgctcact tctgatctta acctcatcga ctatagaatg 3960
aacgtaataa tctcaatcat cagtcctgag acaatagcta agaggaaggt tagggtggct 4020
aggaaggctg tgtgtcaaag tgaaagaact gacgcctgca agttaacctc tgacctccac 4080
acacagacac catgacacat gtgtgttctc ttcatgtgac aaattaaaaa ttgcaaaata 4140
aaaagtgcct aagagatcag agtaagtctc tctctccctt tactccaccc ctttgagtgg 4200

cactgagtct agcagcacac gaggccacat ccttgtctgc tgcaggtgac ggtggccttc 4260
ttggatggag acaaatattt cattatagtt ggattcttgg tctgtctttc taacatgcgg 4320
tcctcagtga ccccatttct ggagcaagcc cagcacagga ggaaacgagg aatctctctt 4380
cctctccact gtccgggcat ttggcagggt gctagagttc atgtcaggga gcaacatggc 4440
cgcagtggct ggtgccagac cttgggagag gccttcaaga ctcaggctgg gatcagagtc 4500
aggaacagaa agctctgagt tctcccagaa cattcagctc tggtcccagc ttccctgggg 4560
tctccacgaa gcagccacag ctgtggtcca ctgggaacct gcagccccac ccacggcatc 4620
ataaagtgaa agttgtcctg ctcatctgct cagatgatct cggagtgctg catccttcag 4680
cactgattta tctcagaagc cctagcaagg gattccttta ttttctcatt ctgtccctct 4740
tcctcttccc ctccctctcc tttgcttcat ccttccttct cttcctcata ggcatacttg 4800
tgcagacaaa taccacatgt atgccgacag tcccccgtca catccttgct ccagtatttg 4860
agaaaaggag ccaggagtct ccatgatatt cttaagaatc aaaccctcca ttccaattcc 4920
tcaggaggtc ttcctcctgg acaatctctg aaaaagatgc accatttctc taatagggat 4980
tgaggggtga tgaccctcta gagccccaat aaagccatga agagaggagc agaggacttc 5040
atggtctgct cttgctataa aaaggccttt ttcgggaaaa aaataaagaa aggaatcagc 5100
caatcccttc acgatgccat cacctcttct tggtggtttt tcggggaagg agtgggtggg 5160
tttccatggc aacagatgcg agctctgctc agtaaagaag ggaccttgat attttttctc 5220
tctcattctc tcagttgtgt gtgtgcctgt gtgtatgtgc gtgctacaca tgcctatgcc 5280
cacaccagca atttttttag aactaaagaa agcccttcta tcagctcccc aaatatggag 5340
tgatagaaaa ccatgcactc ctgcaggcca gagaaggttc tggatggttc cagagaaggg 5400
tgctcctgtg aacttgtttt cctccattgc agagattgtg tgcagcagag aggcctttgc 5460
aaactgttag aggctaagag ttagaaaaaa ggatgtttgg tggagagagg ggaacaaagg 5520
atagatggtg caaaaaccaa cgaatggcgt cctagtgggc aaccaaaggt gcacggagtc 5580
tcaggaagca cagtcagcac aaaccaccta acgctgaaga aaaggctcaa ggcagactca 5640
tatatggaca caaacacaca gagaggtata aaagcaaata tattaggcaa aaccgcaaaa 5700
ctgcatacaa cacagaaagg cagagactta gagaaataaa acagacaaat aaaaacacag 5760
atgcaggtac tggcagatat gtagacacac aaggatgcag agcctatcat caaaacacag 5820

gcaaatagat acatggatgc agatagataa gtgtatccag acagataggt ttggatgcag 5880
acatagaaca tgcaacacag cctcattcaa gtgcacacac tcgtgtgcgc tcacacacac 5940
actccccttt ccccttcag ttgctcaagc ttcctatagc aggaaggcag atctgcaaat 6000
gctgcatgtt cacccagtaa gtttggctgt gaatatcttg taaccccac ctattgcttc 6060
tacacacaca cacacacaca cacacacaca cacacacaca cacacacacc ccaaagcccc 6120
tctcccaact ttgtccactt tcccataacc aaaggctgtg atacctcccc catctcaggc 6180
ttccaattct gttttgctgc tgctgctgcc gccgcctgcc gcttgggggg ggagagagtg 6240
gggtgactca gccaggccag gatgactcac actgacagta tttttagcag cggccaggag 6300
ctctctagcg tgccagccgc ccccctcctc ctgcttgcta tttcggaacc gtcactggtg 6360
atataaatag ctcttctccc acggcctgaa gctgctgcca ggctattttt ggttctgcac 6420
agttaaaaat agtttcatgg aggtgggagg caagaggagt gggagctggc ctagggagag 6480
gagacattgg gggcatacag agcttctcaa cttgaatcag agtagtcgaa ctaagatgat 6540
cccttcccta ccccctccct tgcccctttc tagaaccttc tccccttcca acgttcctta 6600
tccctagtcc atcctcctgg aaaaatccaa ggattcctcc cttgagccca attttctttc 6660
caagcttaac taattcctag aaccgaggag tcttgacagc cacacctgta aatagcccat 6720
atgtattctc aatgaggagg atgacagcat cgggatgcca ttctcatcta tcccgaggcc 6780
cagctcggct ttgatgtcac aggcaaacca cgaccattct gagtgggaag gcaacattca 6840
gcaccacgga cagcgacaac atcccccccc cccctccccc ttccaggtct gcttaaattg 6900
cttggagacc agctgtggac ccagcagaga gatgcaactt attgtggagg agatatcaag 6960
aacgtctcct ggccagggct taaagcacct gtctgtgagg aagacagggc agagatgaac 7020
cccagagata gaatggttgt ctagcataca cagagccctg gtttcatcct cagctttggg 7080
aaactaatct agaaactcca tcttggattt gcatatggaa agagaatcca aaaaccaagg 7140
gaagagaatg gaacagggag tggtggtgtt gagtggggat atcagagtta ataaggatga 7200
aacatgccag agagaaatac atcctgaaga aaccatttct gtcacccata aggttggaaa 7260
cagtgtctta cagacacaca ccattttctc catctcagct ataccactgg ctggctacat 7320
ggttgtatat gtagatgctt tctatctgaa ctaaaattgt acaaaatatt aggatagggg 7380
ctctacaacc atgaacctct ccccgcccct ccccggcatt actagggagt gcactcaagt 7440

cttgagcatg atagaagtgt gaactcctac taagccatgg ccctggtcac caaagtaccc 7500
tcttcccata ccccctgctt ttcactccac gttgcctctc ttgctatcac ccctttccat 7560
gaagaacagg ggtttcttga ccacaaactt ttctccttgg tgtcaaagtt catctctaac 7620
tttctgcagc cagttctgtc cctctctccc aattttttt tgttttttgt tctgtttgtt 7680
tgtgtgtttt tgttttttga gacagggttt ctctgtgtag ccttggctgt cctggaactc 7740
actttgtaga ccaggctggc ctcgaactca gaaatctgct tgcctctgcc tcccaagtgc 7800
tgggattaaa ggcgtgtgcc accacgcccg gcttccctca actttttaaa tggtcttgtt 7860
tttcaggctc taaaagtgct tttatatgtt cctactctaa atgaaatttt gggcaaaaag 7920
tttctctagt cctttgtgaa atggttgtgg gataaaaaaa gggctcccat accctgtgta 7980
gacagcaatc gcatgtaagt gacctgaaga aaggtgtgtg tgggggtgtg tgtctggagg 8040
ggtggggtga tgcaaaggcc acactacaaa gacaagcctg acatgacagg tagttaaacc 8100
aaaggtgcaa attagagggg tgggggtggg gggcgcccac aaagccgaga tagactgtcc 8160
aacgctcaat gaacgaagga aaaaaaaaaa aagaaaggtg tatgttgtgc gctcactcag 8220
tgacaagtgc acaggcagaa cgaggagccc tggagctaaa gatggagcaa gaattgaagg 8280
gagatgggga ggggactctg gcagaattaa agggtcttgg gtaggtgcag cagccactga 8340
gggcacagca gaccctggct acttggcagc ctccccctct tcccggctga agcagtgggg 8400
agagctttct agagctgtgc ggaggccggt aggccccgcc cgccgctgcc gccgccgcag 8460
ccgccggagg attcctgtcc taatatggag ctgggattcc cccggccccg cccccgcccc 8520
ccagcccgcc ggagagactg gggctcgcaa gagggcgggg gaacagctcg tcttggggggc 8580
tgacaagcgg gaggggatcg tgggagaggt tcaaacacac atccagatcc tcaccaggcc 8640
ctgggtcttt gcctcagttt ccccgacagg tggctaagat gaactaatag aggaaaaagg 8700
aatccctgca gatcacagtg gagatgtttg tgttcctatg gtgctaagga aatatgatca 8760
agaccgaatt caagtggtct cttctccaca ggaccaccat tccaccctat cctggagatt 8820
tagaccctca ggtcagggca tggaggcgaa gaaggaatta tttatttgaa actggctaag 8880
ggactttcag attgaataga ccccagaaaa gacccccagt tgtgacctag cccctcccca 8940
aaagccaagg aaagtccctc atgtaatttc gacccctgcc tggcagatgg cggcaaattg 9000
gcagaggacc aagccccttc tcatcctttg cctccttaga aaaatctatg ttgatagcag 9060

cctcgctttg ccttctacaa actctcttgt taagggcttc agaccaccct aatccatgta 9120

ctgttcctcc tcctaataga atgtaatgga aaacctgggt ccctgcaccc cattcctggc 9180

tctgcacagc tcttgccagc cggccccctc tgcaccctcc cttctccccc ttcccccgcc 9240

ccctccctct cccgttcgac gtcacgggat gacgtcggaa gtctgggagg gaggaggagc 9300

acccccccctc cccagccagt ggctccctct gcagcttgct ttagcccagc ctcccgcctc 9360

ccgctgcccc ccccgtctct aaaaacgagc cccccacgcc tgtcaggagc tatataaggc 9420

ggatcgaggc aggcgagggg ggcagcgctg ccgagcggag cccaggagtg gagcgagagc 9480

gagcaagagc ctgagcgaaa agaccgggaa gcaaggaaga ggaagcctcc ggtgcatcgg 9540

gaaaggatcg caggtgctcg ggagccggag ctggagctcc acagccggca gtc atg 9596

Met

1

tac cga tcc acc aag ggc gcc tcc aag gcg cgc cgc gac cag atc aac 9644

Tyr Arg Ser Thr Lys Gly Ala Ser Lys Ala Arg Arg Asp Gln Ile Asn

5 10 15

gcc gag att cgg aac ctc aag gag ctg ctg ccg ttg gct gaa gcg gac 9692

Ala Glu Ile Arg Asn Leu Lys Glu Leu Leu Pro Leu Ala Glu Ala Asp

20 25 30

aag gtc cgg ctg tcc tac ctg cac atc atg agt ctt gcc tgc atc tac 9740

Lys Val Arg Leu Ser Tyr Leu His Ile Met Ser Leu Ala Cys Ile Tyr

35 40 45

act cgc aag ggt gtc ttc ttt gct gga g gtgagcagct tgggctaccg gagac 9793

Thr Arg Lys Gly Val Phe Phe Ala Gly G

50 55

cagagctgac ggggaccagg gatggaggag ctgagggaat gtgctaaaac tgccgcttgt 9853

ctagcacagc gtgctggaag cctgtggaga gaagggactt gaggggaccc tggacttcct 9913

actttttctt ctgagctcca tctagactag cctaaacgat agtcctagca ctggatttgt 9973

gtgagataga gcgctaaaac agaatggtcc aggctcccat tgcctcagag gcactccagg 10033

aatccgggga gggtacggaa ggaagcctgg caagctacag ggaaagcctg caaaggcaaa 10093
gtatgaggaa gagtagcttg tgctagaaaa tgctgagagg gtctttctat gcgctctgga 10153
gctgttcgac gtcctgaagc catcaccctt tctggcgctg cccgcggtgc tgaaatggcc 10213
atagcccctt ttcgcaggag ctgtccgcgg tgctgaatcc cagtcctttc gggagagctc 10273
tgtccacagt gctgacagca gagggctgct gaggttccgg ccaggcttgg aagtccaggg 10333
gctccctggc tagatagttt tagcaacagg tctcctggcc aagatccaca agcatagggt 10393
caacaggtgt tggcagaaat aggtctatgg gaatttcctg tgtcttctcc aagactcaaa 10453
agatgttctc tttatttctg tgttgtccct gattcttatc ctgactcacc acatcttctc 10513
accctacag gc act cct ttg gct ggc ccc acc ggg ctt ctc tct gct caa 10563
ly Thr Pro Leu Ala Gly Pro Thr Gly Leu Leu Ser Ala Gln
60 65 70

gag ctt gaa gac att gtg gca gca cta cct gga ttt ctc ctt gta ttc 10611
Glu Leu Glu Asp Ile Val Ala Ala Leu Pro Gly Phe Leu Leu Val Phe
75 80 85

aca gct gag ggg aag ttg cta tac ctg tcg gag agt gtg agc gag cat 10659

Thr Ala Glu Gly Lys Leu Leu Tyr Leu Ser Glu Ser Val Ser Glu His
90 95 100

ctg ggc cac tct atg gtgagtacta aaagtccttg catctcaagt tggggtatat g 10715
Leu Gly His Ser Met
105

tgagataaaa tgagcctctc actactgaaa acagagttat tagaggcgag tgtgggggag 10775
tcttccctaa gaaaaatcat tggttgcaga taggctcttg ctgccttcac taatgatcac 10835
ttctcctttc tag gtg gac ctg gtt gcc cag ggc gac agt atc tac gat 10884
Val Asp Leu Val Ala Gln Gly Asp Ser Ile Tyr Asp
110 115 120

atc att gac cct gct gac cat ctc act gtg cgc cag cag ctc acc atg 10932

Ile Ile Asp Pro Ala Asp His Leu Thr Val Arg Gln Gln Leu Thr Met
125 130 135

ccc tct gct ctg gat gct g gtaagaacct cctctcggtt cttcagttta ctcctc 10987
Pro Ser Ala Leu Asp Ala A
140

tgctgccctg ccctaactat ctactctcct ccaatgccca ccctcttagt cagtttttcc 11047
ttttgctcac ctag at cgc ctt ttc cgt tgt cga ttc aac acc tcc aag 11096
sp Arg Leu Phe Arg Cys Arg Phe Asn Thr Ser Lys
145 150 155

tcc ctc cgg cgc cag agt tca gga aac aaa ctg gtg ctt att cga ggt 11144
Ser Leu Arg Arg Gln Ser Ser Gly Asn Lys Leu Val Leu Ile Arg Gly
160 165 170

cga ttc cat gct cac cca cct ggg gcc tac tgg gca gga aac cct gtg 11192
Arg Phe His Ala His Pro Pro Gly Ala Tyr Trp Ala Gly Asn Pro Val
175 180 185

ttc acc gct ttc tgc gcc cca ctg gag cca aga ccc cgc cct ggc ccc 11240
Phe Thr Ala Phe Cys Ala Pro Leu Glu Pro Arg Pro Arg Pro Gly Pro
190 195 200

ggc cct ggc cct ggc cct ggt cct gct tct ctc ttc ctg gcc atg ttc 11288
Gly Pro Gly Pro Gly Pro Gly Pro Ala Ser Leu Phe Leu Ala Met Phe
205 210 215

cag agc cgg cat gct aag gac cta gcc cta ctg gac gtt tct gaa ag gt 11337
Gln Ser Arg His Ala Lys Asp Leu Ala Leu Leu Asp Val Ser Glu Se
220 225 230

aagcccaaag tgttcaaact ccagtaagaa gggaggccag aaagaaggga actttagatt 11397
cgtgatctta gattcagggc agggaggatg gggcttaagt gggcagagag catgggaggg 11457
agtgaagtgc atgcattttg agtaaggtaa acagaaagct gacctcatca tttccacctt 11517

```
cccag t gtc cta atc tac ctg ggc ttt gag cgc agc gaa ctg ctc tgt     11565
      r Val Leu Ile Tyr Leu Gly Phe Glu Arg Ser Glu Leu Leu Cys
    235                 240                 245
aaa tca tgg tat gga ctg cta cac ccc gag gac ctg gcc caa gct tct     11613
Lys Ser Trp Tyr Gly Leu Leu His Pro Glu Asp Leu Ala Gln Ala Ser
250                 255                 260                 265
tct caa cac tac cgc ctg t gtgagtgtcc tgagaggccg tgcataacac aggaag  11668
Ser Gln His Tyr Arg Leu L
                270
ctgggagaaa gcatgggaga caggccaggg actggctgtg gtccaaactg atgttaagga  11728
gtttcggagg ctacagagtg agcttgagga tgagaagtca aggcaagaat aggacagagt  11788
tagaaaacac tgtgtgataa ggtcaagtgg ggagcctaga ggtacaggtt agggtagtta  11848
gaagagaata tgtcatggct ccctcaattc agtgtagagg taagaaaggt gggtgtgtag  11908
gtggtgttga ttgatggacc ttctaatccg gtattccttt tttctcccca g tg gct     11964
                                                          eu Ala
gaa agt gga gat att cag gct gaa atg gtg gtg aga ctt caa gcc aag     12012
Glu Ser Gly Asp Ile Gln Ala Glu Met Val Val Arg Leu Gln Ala Lys
    275                 280                 285
cat gga ggc tgg aca tgg att tac tgc atg cta tac tca gaa ggt cca     12060
His Gly Gly Trp Thr Trp Ile Tyr Cys Met Leu Tyr Ser Glu Gly Pro
290                 295                 300                 305
gaa ggc cct ttt act gcc aat aac tac cct atc ag gtaagctgta agataca  12112
Glu Gly Pro Phe Thr Ala Asn Asn Tyr Pro Ile Se
                310                 315
agatggcgga gaggggaggg gagctgaggt cagcatagaa gaaatgcaac gaagaaaact  12172
actctggtaa tggacagcag acccttacaa gctgccacct cttccctttc cag t gac    12229
                                                            r Asp
```

```
acg gaa gcc tgg agc ctc cgc cag cag cta aac tct gaa gac acc cag     12277
Thr Glu Ala Trp Ser Leu Arg Gln Gln Leu Asn Ser Glu Asp Thr Gln
    320                 325                 330
gca gcc tat gtc cta gga acc cca gct gtg cta ccc tca ttc tct gag     12325
Ala Ala Tyr Val Leu Gly Thr Pro Ala Val Leu Pro Ser Phe Ser Glu
335                 340                 345                 350
aat gtc ttc tcc cag gag caa tgc tct aat cca ctc ttt aca cca tcc     12373
Asn Val Phe Ser Gln Glu Gln Cys Ser Asn Pro Leu Phe Thr Pro Ser
                355                 360                 365
ctg ggg act cct aga agt gcc agc ttc ccc agg gct cct gaa cta ggt     12421
Leu Gly Thr Pro Arg Ser Ala Ser Phe Pro Arg Ala Pro Glu Leu Gly
            370                 375                 380
gtg atc tca aca cca gaa gag ctt ccc caa ccc tcc aaa gag ctg gac     12469
Val Ile Ser Thr Pro Glu Glu Leu Pro Gln Pro Ser Lys Glu Leu Asp
        385                 390                 395
ttc agt tac ctg cca ttc cct gct agg cct gag cct tcc ctc caa gca     12517
Phe Ser Tyr Leu Pro Phe Pro Ala Arg Pro Glu Pro Ser Leu Gln Ala
    400                 405                 410
gac ctg agc aag gat ttg gtg tgt act cca cct tac aca ccc cac cag     12565
Asp Leu Ser Lys Asp Leu Val Cys Thr Pro Pro Tyr Thr Pro His Gln
415                 420                 425                 430
cca gga ggc tgt gcc ttc ctc ttc agc ctc cat gaa ccc ttc cag act     12613
Pro Gly Gly Cys Ala Phe Leu Phe Ser Leu His Glu Pro Phe Gln Thr
                435                 440                 445
cac ttg ccc cct ccg tcc agc tct ctc caa gaa cag ctg aca cca agt     12661
His Leu Pro Pro Pro Ser Ser Ser Leu Gln Glu Gln Leu Thr Pro Ser
            450                 455                 460
```

aca gtg act ttc tct gaa cag ttg aca ccc agc agt gct acc ttc cca 12709
Thr Val Thr Phe Ser Glu Gln Leu Thr Pro Ser Ser Ala Thr Phe Pro
465 470 475

gac cca cta acc agt tca cta caa gga cag ttg aca gaa agc tca gcc 12757
Asp Pro Leu Thr Ser Ser Leu Gln Gly Gln Leu Thr Glu Ser Ser Ala
480 485 490

aga agc ttt gaa gac cag ttg act cca tgc acc tct tcc ttc cct gac 12805
Arg Ser Phe Glu Asp Gln Leu Thr Pro Cys Thr Ser Ser Phe Pro Asp
495 500 505 510

cag cta ctt ccc agc act gcc aca ttc cca gag cct ctg ggc agc ccc 12853
Gln Leu Leu Pro Ser Thr Ala Thr Phe Pro Glu Pro Leu Gly Ser Pro
515 520 525

gcc cat gag cag ctg act cct ccc agc aca gca ttc cag gct cat ctg 12901
Ala His Glu Gln Leu Thr Pro Pro Ser Thr Ala Phe Gln Ala His Leu
530 535 540

aac agc ccc agc caa acc ttc cca gag caa ctg agc ccc aat cct acc 12949
Asn Ser Pro Ser Gln Thr Phe Pro Glu Gln Leu Ser Pro Asn Pro Thr
545 550 555

aag act tac ttc gcc cag gag gga tgc agt ttt ctc tat gag aag ttg 12997
Lys Thr Tyr Phe Ala Gln Glu Gly Cys Ser Phe Leu Tyr Glu Lys Leu
560 565 570

ccc cca agt cct agc agc cct ggt aat ggg gac tgt aca ctc ctg gcc 13045
Pro Pro Ser Pro Ser Ser Pro Gly Asn Gly Asp Cys Thr Leu Leu Ala
575 580 585 590

cta gct cag ctc cgg ggc ccc ctc tct gtg gat gtc ccc ctg gtg ccc 13093
Leu Ala Gln Leu Arg Gly Pro Leu Ser Val Asp Val Pro Leu Val Pro
595 600 605

gaa ggc ctg ctc aca cct gag gcc tct cca gtc aag caa agt ttc ttc 13141
Glu Gly Leu Leu Thr Pro Glu Ala Ser Pro Val Lys Gln Ser Phe Phe
610 615 620

cac tac aca gag aaa gag caa aat gag ata gat cgt ctc att cag cag 13189
His Tyr Thr Glu Lys Glu Gln Asn Glu Ile Asp Arg Leu Ile Gln Gln
625 630 635

atc agc cag ttg gct cag ggc gtg gac agg ccc ttc tca gct gag gct 13237
Ile Ser Gln Leu Ala Gln Gly Val Asp Arg Pro Phe Ser Ala Glu Ala
640 645 650

ggc act ggg ggg ctg gag cca ctt gga ggg ctg gag ccc ctg aac cct 13285
Gly Thr Gly Gly Leu Glu Pro Leu Gly Gly Leu Glu Pro Leu Asn Pro
655 660 665 670

aac ctg tcc ctg tca ggg gct gga ccc cct gtg ctt agc ctg gat ctt 13333
Asn Leu Ser Leu Ser Gly Ala Gly Pro Pro Val Leu Ser Leu Asp Leu
675 680 685

aaa ccc tgg aaa tgc cag gag ctg gac ttc ctg gtt gac cct gat aat 13381
Lys Pro Trp Lys Cys Gln Glu Leu Asp Phe Leu Val Asp Pro Asp Asn
690 695 700

tta ttc ctg gaa gag acg cca gtg gaa gac atc ttc atg gat ctt tct 13429
Leu Phe Leu Glu Glu Thr Pro Val Glu Asp Ile Phe Met Asp Leu Ser
705 710 715

act cca gac ccc aat ggg gaa tgg ggt tca ggg gat cct gag gca gag 13477
Thr Pro Asp Pro Asn Gly Glu Trp Gly Ser Gly Asp Pro Glu Ala Glu
720 725 730

gtc cca gga ggg acc ctg tca cct tgc aac aac ctg tcc cca gaa gat 13525
Val Pro Gly Gly Thr Leu Ser Pro Cys Asn Asn Leu Ser Pro Glu Asp
735 740 745 750

cac agc ttc ctg gag gac ttg gcc acc tat gaa acc gcc ttt gag aca 13573
His Ser Phe Leu Glu Asp Leu Ala Thr Tyr Glu Thr Ala Phe Glu Thr
755 760 765

ggt gtc tca aca ttc ccc tac gaa ggg ttt gct gat gag ttg cat caa 13621
Gly Val Ser Thr Phe Pro Tyr Glu Gly Phe Ala Asp Glu Leu His Gln
770 775 780

ctc cag agc caa gtt caa gac agc ttc cat gaa g gtaagtctag cctgaatg 13673
Leu Gln Ser Gln Val Gln Asp Ser Phe His Glu A
785 790

tccaagagcc ctgcccttct aatcagacat tgcatagatt gggtgaatca gtccccaact 13733
ctgaaactct gttttattaa gagaacaata ttacctccta ctaagaagag tagtgaggta 13793
ggaataatac aaagctttgt gtgaaagatg agtagacctg gtgggcgggg gaggtgagct 13853
agaaaaacgc gatagacaat ccctaggcaa aagcttgaaa gcttctgaga gacctagacc 13913
agacaacacc gtcattttat agacaaaaat aatcaaggcc ccagagttaa agaaacttta 13973
agtggcacaa aaattgatag aagttgatgc ttccccctga aggggaccca gagcaacaac 14033
tggttaaaat taggagacag aaagaacaat gccaagcccc tagctccaat ctggcggcct 14093
tgtgctgttt gtccaaagct gtggccacag tttccctcca tatttgcata ttgcctctta 14153
tctgctgaca ccctggggat cagttcattt ggctaacaca tttgacgtcc atagactata 14213
gcaatattgt accactgcct gagcccaatg acgcttttac tgaataagct tgactaacat 14273
acgcactttc tctcttctct ctctctctct ttccccacag at gga agt gga ggg 14327
sp Gly Ser Gly Gly
795

gaa cca acg ttt tga ataagtctgt gacttaacgt cttcaagtat ggcatattgt c 14383
Glu Pro Thr Phe Stop
800

atcaagacgt ggagccgctc tccacccccc cgggactgtt ggggggattc tgggggccag 14443
agggggatat atctgattct ccaggccctg aaggatttag gggggaggtg ggagggtaag 14503

ggaggggagc aactttttaa aatcaagaga cttcgagcga tcccagtttc catttcaatc 14563
tgtattcact cgtagtgagt ttccttgaat ggatttcaag cggagaatgg gggagtctca 14623
cttcctcacc gcgctgcccc atgggcctgg gccagttctc cactcctagg ggcaaagcca 14683
cccctgggct ttggtggggg aaaggcatgg cccacctggg gctagcctgt gccccgaggg 14743
gctcttgaca cccacgtaga attctctaca aaccagtaac gggatttcaa ttccgacgga 14803
ctctgccgcc ctggcggctc ttcctgtgac ttttgcgccc cgcgcctggg gtgggggcg 14863
cgaagagacg ctacattcct ttccgatgga ggaaggcaga tctgccgtca cacgtgtgct 14923
tgcacgagtg cgtgtacctg gtgcgggact cacccggccg ccagaccgcc taggcttgcc 14983
caggtggcca cctcgtggtg ctgcggtgac tttgtagcca actttataat aaagtccagt 15043
ttgccttttt ggtatctctg gtgtcatgcg ctattgtgaa aagggaaggg aggggaaggg 15103
agagattgag gagcccagat aggaggctgg ggcaggagtc acaggttaga cctcctctca 15163
gccctggtat ctctaagtga gtttgttcat atctccattt gactctgctt ggtccacact 15223
gtgctagaag actaagtact tgtcagaagc agacattgca ccaaagacac tggagtcttc 15283
tctctgccct gggtttatgg tgtgatgggg aggaaagagc ctggggctga gcaagtttgt 15343
cactggtctt ggatatgggt ttaaagtttc tggtcatttc ctgcctggtc tttcaggata 15403
ttgatttcct catggaggct tagattttaa aaatcagaag ctgaaacctg ttacgcttgc 15463
gtagggctgt tcagttagca aatacccaat ccactgcaat aaatttccac ttcattggga 15523
aagcaacccg ataacgggtg ttcctccagt tacaggtgag aaacacatca acccctcccc 15583
aaatctgggg agctcccaga tctcaatgcc agcgaataac catcatagac catctcacca 15643
cagagctgag gaccagtcac tggggaggaa atttcagaaa atggtgtttg actctaaact 15703
cgtaggctca accccacagg gtgtggttag tggaggacaa atgaaagtta ggtggtagaa 15763
ggacctgaca gatccaatca cgatcccacc ttttgtattt ggagtgcacc taaagccccc 15823
acttcctcac aggtcaaagg agggcagcaa tcaagaggca gtgtcagaac aggacaagtc 15883
tcttccagct cacgaagtgc agtgaaggct tggtcggtgc gacctccatt tcagtggtga 15943
cccgcagact tagagaaagc cttgtcctca aggagaggac aacaactcca ggctccagtc 16003
tttccacaga agcacagggg cacagccttg aaaaccctgt agcctccact catcctgaag 16063
cccagctgtg gagacagaca ggccctttgg agggtccttc cttcactgtg gagacagaca 16123

ggccctttgg agggtccttc cttcactgtg gagacagaca ggccctttgg agggtccttc 16183

cttcactgtg gagacaggcc ctttggatcc ttccttcaca gaaaggaagg atccacaggg 16243

acctttccct tctttgatgg gtatttgggt ggagccaaga acttccctgt cactcccaag 16303

aggaacctgt cttagctcag ttccctcctc agcacaggga cacggagatg gggagatgga 16363

taaaggtgct gggccaagca tgatgctctg atttgatcct tgatgggaag agataactga 16423

cagttgtcct ctgacgtgta actgcactcc aggacatgtt acactcacat gtgcacacac 16483

acacactaca cacactacat acacatacca tacacatact atacacaata taccacacac 16543

acacatacta tacacacata ccacatacac tacacacagt acacatgcta cacatacata 16603

cacacaccac acacatatac cacacacaaa cactctacac acacacacta cacacactac 16663

atacatatac cacacacact taccacacat acagtatata cagtacatac atatgccaca 16723

cacacataac acacactcac acacaccata tatactacta atagaaaata ataaaaattt 16783

ttaaatgggg tggatttagg aaatgaaatt tctgtgagaa taaaggaaag gcttccttga 16843

tgtttggtgg tggctggcaa tagtgtatgc tttctttgtc tttgtttgtt gtagtttttt 16903

tgtttatttt gcttttgatt tttttttgt tgttgttgtt acttgtttgt tgaaaacctg 16963

cctctgcctc ctaagcactg aattgtcttg ggtggttttt aaaaattaat taatgttgaa 17023

atattttttt catttttgag acaagatttc tttgtgtagc cttagctgtc ttagaactag 17083

ctctgtagac taagctggcc ttaaacttac agagatctgc ttgcttctgc ctcctgagtg 17143

ctgggattaa agtttttagt ttttaaaaaa atataattac agatatgcac tgtctttgca 17203

tcatgtcctc ttgttttggg cttatttttg ttgttgtggt ggtgataagt gatttttttt 17263

gtttgttttt gtttttagtt ttgtttttct tcagctcagg tcaatctgga gttcactatg 17323

tagtccaagg tggccacaga cttttgcaaa tccccctgcc tcagcctccc aggtgctagg 17383

attacagaag aaccagacca actggtcctg tgtgaggaaa ataaagtaga agaggcaata 17443

ctgccacctg ctggaaggaa aagaagctgc ttccttgctg gctgctgagg cccttgcagc 17503

tcagaatatc ttcaccttag aatggagaga taaactgagt ccctgggaga gaaaaggact 17563

tcaggatctg agagtgagtg atgttctgga agcagagtgc atgagagaag gtgtcttaat 17623

cattgtagta ctgctgtgag aagacaccat gaccaaggta acataaaata aagcatttag 17683

ttggggactt gcttagagtt taaaagggtt gctccatgac cagcagagca gggagcatgg 17743

gagtatgcag gtagacacgg cactggagaa gtacctgaga gcttccatct gatccccaag 17803
atagaggcag agagaaccct caaagcccac accccctcca acaacaaaca cctcctgatc 17863
cttcctaaac agtccaccaa atggagacta agcattcaga tatggggacc attatcatcc 17923
aaaccactat ggaaggctcg agtctgggga ccagacagac tgaacccagg agaccaaggg 17983
gatagcttag tgggtaaagg cgctagctgc cgagcttgga gacgcaagtc caatccctag 18043
gttctgtatg gtggaaagaa acgggattcc agtaagtcac cccctggcct tcgcgcacac 18103
catgatgctc atgcccacac acatacaaat ccaaaagaaa gaccgaacct aaggatggtt 18163
ctgctgttgt acatttttcc tgtaatagat catccatgac acttgcctga gttctgggaa 18223
aactgaacaa acaagatggg tggggccaga cagctgtgct ctaactggga acatcacaag 18283
aggtaagaca gagcctgagt gctgaaggca agagctaggg tatcgtgaca gagtaaccgg 18343
ggactgattt atagtgccac tttctgagaa ggtgacactg agcttgttag caacaggtga 18403
caacaaagaa gagtccaacc taaaggaagc atctgtaatg acattaaaac gggagagtgt 18463
ctgagctgct taagaagtac acaggaagtg ggctgagaca agcaggagag gggctggaga 18523
gaaggtcgcc cagtacttct agaccacaat aaaagatgta ggttgcattc tggctgagcg 18583
tggtagtgca cacctgcaat tgcagcctca aaaggccgag ggtggaaaat cttgagctcc 18643
tggacagcct gggctccata gaaagaaaag tctgcaaaca acagcaacaa aaaacccaaa 18703
ccaaaaacca aagtgctggt gtcctagtga gggttcctat tgctatgagg aaaaacaatg 18763
atcaaaaaca aactgcggac gaaagggttt gtttgcctgg cacttccaca tcacagtcca 18823
tcattgaagg aatccagaac aggaacgcaa gcaaggcagg aacctggagg caggagccaa 18883
tgaagaggtc atgaagggtt gctgcttatg gcttgctcca catggcttta cagcctgcta 18943
gatctcagca ccaacagcct accatgagcg tggccctcct ccatcaatca ctgattaaga 19003
aaatgtccta cacaggaagg gaggaaggaa gagagagtta ggagcatatt ggatggggat 19063
agtgacagga taagatgtag ctactagagt cttctggttt agatggtgaa tctgccagaa 19123
tttgccactg aaggatttag atttagattt aacataactt acaagattag cattctagtt 19183
gttgcaccca gagactgagt taccattgtt tctgaactaa gtttgtgtgc tgtttttctt 19243
cacgcggtgg ctcgactggg ttcaagagag aaaggtacag cggcaaagcc tgggtttgcc 19303
agatgcgcac cacaaaggca gtgggggttt gaacgatggg gctagcacgg cagtgggaac 19363

tcattgagcc gggtggaggg attttggagc tccaggtcag agagtttgct gagatgagaa 19423
caccaggctg gagccatgtg gcctgccggt accttggcat aatgagggaa cttgctgttc 19483
tttttaatat ttcccacaac aggtggtgaa ccagcatgtt ggggaagaat ccactagaaa 19543
tgtaagatta tgccgggcgt ggtggtgctc gcctttaatc ccagcactcg ggaggcagag 19603
gcaggcagat ttctgagttc gaggccagcc tggtctacaa agtgagttcc aggacagcca 19663
gggctacaca gagaaaccct gtctcgaaag acaaaacaaa acaaaacaaa caaaacaaaa 19723
caaaacgtat gatcattagc ctgagagtta gagttttatt tgtttgtttg tttgtttgtt 19783
atttaaaatg agtagctggg tagtgctgac acaagtcatg tggacccaag cgtggaattg 19843
aaacaaagac tgtaactctg aggtcccctg ctgtgggggc tgcaggctgt tctgagtcag 19903
gagaagaagg atgaagttgc ctacttctta gggcagagat ggattgaact gtgaatttat 19963
aaaattggta ttatttgctt ttaggaaaga tttatatctg ggttttgcct gaatcacatg 20023
gggattttcg cccactgttc agaattagga taggaaaaaa atcagtccct gactccaggt 20083
agaaaagaca gtgattatcg tctgctacaa acaggtatca attaactatg tctgtggctc 20143
cctgtagaga gctcaaaaga tggatattat aacaggtatt aataaaatta atgtcaccca 20203
ggcagtggtg gcacacgcct ttaatcccag cacttgggag gcagaggcag gcggatttct 20263
gagttcgagg ccagcctggt ctacagagtg agttccagca cagccagggc tacacagaga 20323
aaccctatct tgaaaaaaaa attaaataaa attaatgtct gtggccccag tgctgagcag 20383
atagacagtg taacaagatg gctgctctag gcagagagct gaacaggaag atggtatgaa 20443
gatagtttgc tctaacacac ctcacaggat gctcaaatcc tgtctatgtg ggctccatgg 20503
gaatcttttt tttaattagg tattttcctc atttacattt ccaatgctat cccaaaagtc 20563
ccccataccc tcctcccaac cccccaacca cccactccca ctttttggcc ctggcgttcc 20623
cctgtactgg ggcatataaa gtttgcgtgt ccaatgggcc tctctttcca gtgatggctg 20683
actaggccac cttttgatac atatgcagct agagtcaaga gctccggggt actggttagt 20743
tcataatgtt gttccaccta tagggttgca ga 20775